# EUROPEAN PATENT APPLICATION

(11) **EP 1 709 976 A1**
(43) Date of publication of application: **11.10.2006**
(21) Application number: 04807603.8
(22) Date of filing: 22.12.2004
(51) Int. Cl.: A61K 48/00, A61K 38/17, A61P 43/00, A61P 35/00, A61P 35/04, A61P 17/02, A61P 29/00, C12N 15/12, C12Q 1/66, C12Q 1/68, C07K 14/435

(54) **EMT- INDUCING AGENTS**

(30) Priority: 26.12.2003 JP 2003435122
(71) Applicant: HIRANO, Toshio, Osaka-shi, Osaka-fu 559-0003 (JP)
(72) Inventor: HIRANO, Toshio, 5590003 (JP); YAMASHITA, Susumu, 5650872 (JP)
(74) Representative: Bassil, Nicholas Charles
(86) International application number: PCT/JP2004/019246
(87) International publication number: WO 2005/063301

(57) **Abstract**

The mechanism of STAT3, which is considered to play a crucial role in EMT, was elucidated using zebrafish embryos. Unexpectedly, a STAT3 target gene turned out to be zinc transporter LIV1. The present inventors studied the relationship between STAT3 and LIV1 in EMT, and further studied their relationship with zinc finger protein Snail, known for its association with EMT. The results showed that LIV1, whose expression is regulated by STAT3, activated Snail, thereby ultimately inducing EMT. LIV1 can be used as an EMT regulatory agent. Further, because EMT is involved in cancer progression, LIV1 antisense nucleotides and the like may be used as pharmaceuticals for treating cancer.

## Description

### Technical Field

This invention relates to the uses of LIV1 for regulating epithelial-mesenchymal transition (EMT).

### Background Art

Epithelial-mesenchymal transition (EMT) is a central event in embryonic development, organ and tissue regeneration, and tumor metastasis and progression. Epithelial-mesenchymal transition (EMT) is a phenotypic cell transition where, in the processes of gastrulation, wound healing, tumor progression, and the like, epithelial cells disperse by weakening the cell-cell adhesion system, and show invasive cell behavior as mesenchymal cells (Non-Patent Document 1). To understand development and tumor metastasis and progression, the mechanism of EMT must be elucidated; however, much regarding EMT remains unknown.

There are a few reports of evidence that indicates a relationship between EMT and the changes in the cell-cell adhesion system caused by Snail and Slug. Snail and Slug are zinc-finger proteins that change the cell-cell adhesion system. Snail family proteins were first reported as substances playing an important role in *Drosophila* gastrulation. Snail has recently come to be considered important as a direct repressor of the transcription of cell-cell adhesion molecules such as E-cadherin, which is involved in adhesive binding, and claudins and occludins, which are involved in tight junctions (Non-Patent Documents 1-4). Snail and Slug show similar localization patterns in the embryos of mice, chickens, *Xenopus,* zebrafish, and *Drosophila.* Loss of Snail or Slug function in these embryos results in defective gastrulation and/or neural crest migration (Non-Patent Documents 1, 5-7). Further, enhanced Snail expression is thought to correlate with dedifferentiation and acquirement of metastatic potential in many human cancers. Thus, the potential role of Snail and Slug in the physiological or pathological *in vivo* processes of EMT can be considered to be evolutionarily conserved (Non-Patent Document 1). However, mechanisms for regulating the activity of expressed Snail remain unknown.

On the other hand, signal transducers and activators of transcription (STAT) are transcription factors that respond to a variety of cytokines and growth factors, and mediate their biological functions (Non-Patent Documents 8-10). For example, they mediate biological functions such as cell proliferation, differentiation, and survival. Furthermore, STATs are involved in vertebrate gastrulation and wound healing, as well as in cancer metastasis in vertebrate animals and cell movement in similar processes in *Drosophila* and *Dictyostelium discoideum* (Non-Patent Document 11). Previously, the present inventors revealed that STAT3 is activated in the organizer during the gastrulation process in zebrafish, and that STAT3 activity is essential for gastrulation movements, but is not required for initial cell developmental fate specificity. This requirement for STAT3 is cell autonomous for the anterior migration of gastrula organizer cells, and non-cell-autonomous for convergence of neighboring cells (Non-Patent Document 12). In addition, STAT is required for cell migration but is not required for cell proliferation, with respect to the border cell migration that occurs during oogenesis in *Drosophila* (Non-Patent Document 13), chemotaxis of *Dictyostelium discoideum* (Non-Patent Document 14), and dermal wound healing processes in mice (Non-Patent Document 15). Furthermore, constitutive activation of STAT family members, and of STAT3 in particular, is observed in many human cancers. This fact means that the above STAT family members are involved in cell growth and survival (Non-Patent Document 16). However, it is thought that STAT3 may also influence cell-cell adhesion and cancer cell movement. Based on the above observations, it is highly likely that the role of STAT signal transduction in EMT is evolutionarily conserved throughout those processes. However, the entire molecular mechanism of STAT function in EMT is unknown.
[Non-Patent Document 1] Thiery, J. P. Epithelial-mesenchymal transitions in tumour progression. Nat Rev Cancer 2, 442-54 (2002).
[Non-Patent Document 2] Batlle, E. et al. The transcription factor snail is a repressor of E-cadherin gene expression in epithelial tumor cells. Nat Cell Biol 2, 84-9 (2000).
[Non-Patent Document 3] Cano, A. et al. The transcription factor snail controls epithelial-mesenchymal transitions by repressing E-cadherin expression. Nat Cell Biol 2, 76-83 (2000).
[Non-Patent Document 4] Ikenouchi, J., Matsuda, M., Furuse, M. & Tsukita, S. Regulation of tight junctions during the epithelium-mesenchyme transition: direct repression of the gene expression of claudins/occludin by Snail. J Cell Sci 116, 1959-67 (2003).
[Non-Patent Document 5] Leptin, M. twist and snail as positive and negative regulators during Drosophila mesoderm development. Genes Dev 5, 1568-76 (1991).
[Non-Patent Document 6] Nieto, M. A., Sargent, M. G., Wilkinson, D. G. & Cooke, J. Control of cell behavior during vertebrate development by Slug, a zinc finger gene. Science 264, 835-9 (1994).
[Non-Patent Document 7] Carver, E. A., Jiang, R., Lan, Y., Oram, K. F. & Gridley, T. The mouse snail gene encodes a key regulator of the epithelial-mesenchymal transition. Mol Cell Biol 21, 8184-8 (2001). [Non-Patent Document 8] Bromberg, J. & Darnell, J. E., Jr. The role of STATs in transcriptional control and their impact on cellular function. Oncogene 19, 2468-73 (2000).
[Non-Patent Document 9] Darnell, J. E., Jr. STATs and gene regulation. Science 277, 1630-5 (1997).
[Non-Patent Document 10] Hirano, T., Ishihara, K. & Hibi, M. Roles of STAT3 in mediating the cell growth, differentiation and survival signals relayed through the IL-6 family of cytokine receptors. Oncogene 19, 2548-56 (2000).
[Non-Patent Document 11] Yamashita, S. & Hirano, T. in Signal Transducers and Activators of Transcription (STATs): Activation and Biology (eds. Sehgal, P. B., Hirano, T. & Levy, D. E.) (Kluwer Academic Publishers, Dordrecht, The Netherlands, in press).
[Non-Patent Document 12] Yamashita, S. et al. Stat3 Controls Cell Movements during Zebrafish Gastrulation. Dev Cell 2, 363-75 (2002).
[Non-Patent Document 13] Silver, D. L. & Montell, D. J. Paracrine signaling through the JAK/STAT pathway activates invasive behavior of ovarian epithelial cells in Drosophila. Cell 107, 831-41 (2001).
[Non-Patent Document 14] Mohanty, S. et al. Evidence that the Dictyostelium Dd-STATa protein is a repressor that regulates commitment to stalk cell differentiation and is also required for efficient chemotaxis. Development 126, 3391-405 (1999).
[Non-Patent Document 15] Sano, S. et al. Keratinocyte-specific ablation of Stat3 exhibits impaired skin remodeling, but does not affect skin morphogenesis. Embo J 18, 4657-68 (1999). [Non-Patent Document 16] Bowman, T., Garcia, R., Turkson, J. & Jove, R. STATs in oncogenesis. Oncogene 19, 2474-88 (2000).
[Non-Patent Document 17] Manning, D. L., Daly, R. J., Lord, P. G., Kelly, K. F. & Green, C. D. Effects of oestrogen on the expression of a 4.4 kb mRNA in the ZR-75-1 human breast cancer cell line. Mol Cell Endocrinol 59, 205-12 (1988).
[Non-Patent Document 18] Manning, D. L. et al. Oestrogen-regulated genes in breast cancer: association of pLIV1 with lymph node involvement. Eur J Cancer 30A, 675-8 (1994).
[Non-Patent Document 19] Taylor, K. M. & Nicholson, R. I. The LZT proteins; the LIV-1 subfamily of zinc transporters. Biochim Biophys Acta 1611, 16-30 (2003).
[Non-Patent Document 20] Taylor, K. M., Morgan, H. E., Johnson, A., Hadley, L. J. & Nicholson, R. I. Structure-function analysis of LIV-1, the breast cancer-associated protein that belongs to a new subfamily of zinc transporters. Biochem J 375, 51-9 (2003).
[Non-Patent Document 21] Nasevicius, A. & Ekker, S. C. Effective targeted gene 'knockdown' in zebrafish. Nat enet 26, 216-20 (2000).
[Non-Patent Document 22] Kozlowski, D. J. & Weinberg, E. S. Photoactivatable (caged) fluorescein as a cell tracer for fate mapping in the zebrafish embryo. Methods Mol Biol 135, 349-55 (2000).
[Non-Patent Document 23] Thisse, C., Thisse, B., Schilling, T. F. & Postlethwait, J. H. Structure of the zebrafish snail 1 gene and its expression in wild-type, spadetail and no tail mutant embryos. Development 119, 1203-15 (1993).
[Non-Patent Document 24] Thisse, C., Thisse, B. & Postlethwait, J. H. Expression of snail2, a second member of the zebrafish snail family, in cephalic mesendoderm and presumptive neural crest of wild-type and spadetail mutant embryos. Dev Biol 172, 86-99 (1995).
[Non-Patent Document 25] Solnica-Krezel, L., Stemple, D. L. & Driever, W. Transparent things: cell fates and cell movements during early embryogenesis of zebrafish. Bioessays 17, 931-9 (1995).
[Non-Patent Document 26] Blanco, M. J. et al. Correlation of Snail expression with histological grade and lymph node status in breast carcinomas. Oncogene 21, 3241-6 (2002). [Non-Patent Document 27] Fujita, N. et al. MTA3, a Mi-2/NuRD complex subunit, regulates an invasive growth pathway in breast cancer. Cell 113, 207-19 (2003).
[Non-Patent Document 28] Peinado, H., Quintanilla, M. & Cano, A. Transforming growth factor beta-1 induces snail transcription factor in epithelial cell lines: mechanisms for epithelial mesenchymal transitions. J Biol Chem 278, 21113-23 (2003).

### Disclosure of the Invention

### Problems to be Solved by the Invention

An objective of the present invention is to provide EMT regulatory agents and novel pharmaceuticals for treating cancer.

### Means to Solve the Problems

To solve the above problems, the present inventors used zebrafish embryos to elucidate the molecular mechanism of STAT3 function, considered to be critical to EMT. The present inventors isolated a STAT3 target gene, which was unexpectedly found to be LIV 1.

Thus, the present inventors continued to study LIV1. First, they revealed that the LIV1 gene was expressed in gastrula organizer cells in zebrafish. Next, they examined the effect of suppressing LIV1 expression on embryos, by specifically suppressing LIV1 gene expression in several kinds of zebrafish embryos. The results indicated that defective LIV1 activity in zebrafish embryos interferes with the extent of organizer cell migration. By expressing the LIV 1 gene in the organizer cells of zebrafish embryos with inhibited STAT3 expression, the present inventors also revealed that, of the defective STAT3 functions, cell autonomous functions could be rescued by LIV 1 gene expression, but non-cell-autonomous functions could not. Further, they showed that LIV 1 enhanced the repressor activity of the zinc finger protein Snail, which is expressed independently of STAT3 and LIV 1 activities, and also showed that Snail was essential for LIV 1 function in EMT. These results indicated that LIV 1 is an essential and sufficient STAT3 target gene for STAT3's cell autonomous role in anterior migration of organizer cells. The three molecules, STAT3, LIV1, and Snail, were also confirmed to have some relationship regarding the EMT process of gastrula organizer cells in zebrafish embryos.

LIV 1 was initially identified as a breast cancer protein whose expression was regulated by estrogen. Although LIV 1 was known to be involved in the expansion of cancer metastasis (Non-Patent Documents 17 and 18), it was recently revealed that LIV1 belongs to the ZIP zinc transporter subfamily (Zrt-, Irt-like proteins), termed LZT (an LIV-1 subfamily of ZIP zinc transporters) (Non-Patent Document 19), and that LIV1 functions as a zinc transporter protein (Non-Patent Document 20).

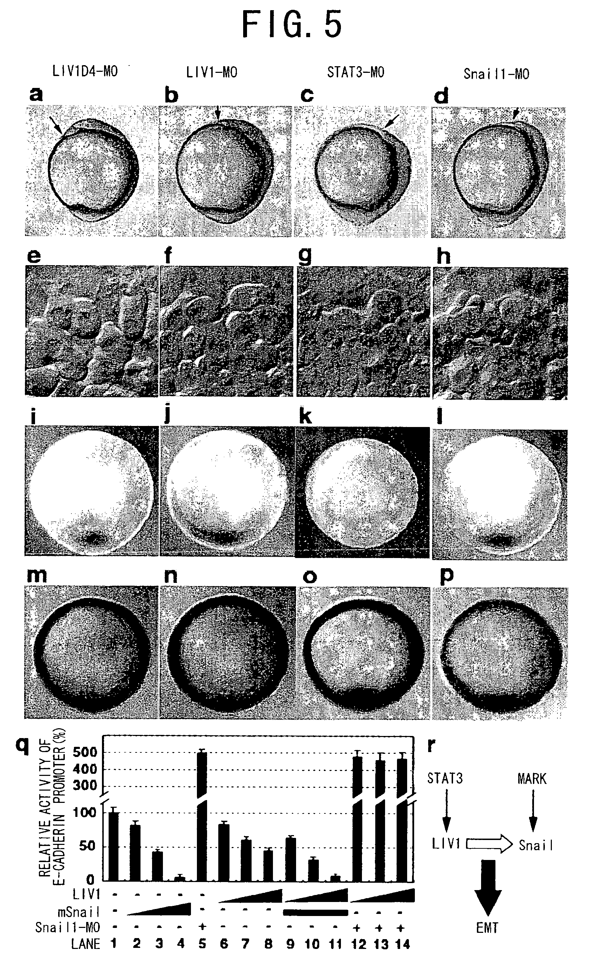

Based on the above findings, the present inventors established the following model EMT mechanism. The expression of zinc finger protein Snail is regulated by MAPK through TGF-β or FGF (Non-Patent Document 28). The zinc transporter LIV1, whose expression is regulated by STAT3, activates the zinc finger protein Snail, which leads to down-regulation of the cell-cell adhesion system, and ultimately induces EMT. The above model EMT mechanism is shown in Fig. 5r.

LIV1 and Snail are reported to be involved in the expansion of breast cancer metastasis (Non-Patent Documents 1, 18, 26, and 27). In addition, STAT3 is constitutively activated in many tumors including breast cancer. Thus, similar mechanisms may contribute to cancer progression (Non-Patent Document 16). However, nothing was known regarding the role of LIV 1 *in vivo* and whether or not it was critical for cancer cell metastasis. Herein, the present inventors demonstrated for the first time that LIV 1 contributes to EMT, and is further involved in cancer metastasis. Specifically, the present inventors elucidated the mechanism of Epithelial-mesenchymal transition (EMT) related to cancer metastasis and progression, and provided the EMT regulatory agents, pharmaceuticals for treating cancer, and the like, of this invention. More specifically, the present invention provides the following:
[1] A regulatory agent of Snail activity, which is an isolated DNA of any one of the following (a) to (d):
   (a) a DNA encoding a protein comprising the amino acid sequence of SEQ ID NO: 1, 2, 26, 28, 30, 32, 34, 36, 38, 40, or 42;
   (b) a DNA comprising the sequence of SEQ ID NO: 3, 4, 25, 27, 29, 31, 33, 35, 37, 39, or 41;
   (c) a DNA encoding a protein comprising an amino acid sequence with one or more amino acid substitutions, deletions, insertions, and/or additions in the amino acid sequence of SEQ ID NO: 1, 2, 26, 28, 30, 32, 34, 36, 38, 40, or 42; and
   (d) a DNA hybridizing under stringent conditions with the sequence of SEQ ID NO: 3, 4, 25, 27, 29, 31, 33, 35, 37, 39, or 41.
[2] A regulatory agent of Snail activity, which is a vector into which a DNA of any one of the following (a) to (d) is inserted:
   (a) a DNA encoding a protein comprising the amino acid sequence of SEQ ID NO: 1, 2, 26, 28, 30, 32, 34, 36, 38, 40, or 42;
   (b) a DNA comprising the sequence of SEQ ID NO: 3, 4, 25, 27, 29, 31, 33, 35, 37, 39, or 41;
   (c) a DNA encoding a protein comprising an amino acid sequence with one or more amino acid substitutions, deletions, insertions, and/or additions in the amino acid sequence of SEQ ID NO: 1, 2, 26, 28, 30, 32, 34, 36, 38, 40, or 42; and
   (d) a DNA hybridizing under stringent conditions with the sequence of SEQ ID NO: 3, 4, 25, 27, 29, 31, 33, 35, 37, 39, or 41.
[3] A regulatory agent of Snail activity, which is an isolated protein encoded by a DNA of any one of the following (a) to (d):
   (a) a DNA encoding a protein comprising the amino acid sequence of SEQ ID NO: 1, 2, 26, 28, 30, 32, 34, 36, 38, 40, or 42;
   (b) a DNA comprising the sequence of SEQ ID NO: 3, 4, 25, 27, 29, 31, 33, 35, 37, 39, or 41;
   (c) a DNA encoding a protein comprising an amino acid sequence with one or more amino acid substitutions, deletions, insertions, and/or additions in the amino acid sequence of SEQ ID NO: 1, 2, 26, 28, 30, 32, 34, 36, 38, 40, or 42; and
   (d) a DNA hybridizing under stringent conditions with the sequence of SEQ ID NO: 3, 4, 25, 27, 29, 31, 33, 35, 37, 39, or 41.
[4] An agent for suppressing Snail activity, which is an antisense oligonucleotide targeting a DNA sequence comprising the sequence of SEQ ID NO: 3, 4, 25, 27, 29, 31, 33, 35, 37, 39, or 41.
[5] An agent for suppressing Snail activity, which is a double-stranded RNA comprising a sequence identical or similar to a portion of a DNA comprising the sequence of SEQ ID NO: 3, 4, 25, 27, 29, 31, 33, 35, 37, 39, or 41.
[6] A pharmaceutical for treating cancer, which comprises a nucleotide or vector of any one of the following (a) to (c), as an active ingredient:
   (a) an antisense oligonucleotide of a DNA comprising the sequence of SEQ ID NO: 3;
   (b) a vector into which an antisense oligonucleotide of a DNA comprising the sequence of SEQ ID NO: 3 is inserted; and
   (c) an oligonucleotide that is a double-stranded RNA comprising a sequence identical or similar to a portion of a DNA comprising the sequence of SEQ ID NO: 3.
[7] A pharmaceutical for treating cancer, which comprises the agent for suppressing Snail activity of [4 or 5 as an active ingredient.
[8] An EMT-inducing agent, which is an isolated DNA of any one of the following (a) to (d):
   (a) a DNA encoding a protein comprising the amino acid sequence of SEQ ID NO: 1, 2, 26, 28, 30, 32, 34, 36, 38, 40, or 42;
   (b) a DNA comprising the sequence of SEQ ID NO: 3, 4, 25, 27, 29, 31, 33, 35, 37, 39, or 41;
   (c) a DNA encoding a protein comprising an amino acid sequence with one or more amino acid substitutions, deletions, insertions, and/or additions in the amino acid sequence of SEQ ID NO: 1, 2, 26, 28, 30, 32, 34, 36, 38, 40, or 42; and
   (d) a DNA hybridizing under stringent conditions with the sequence of SEQ ID NO: 3, 4, 25, 27, 29, 31, 33, 35, 37, 39, or 41.
[9] An EMT-inducing agent, which is a vector into which a DNA of any one of the following (a) to (d) is inserted:
   (a) a DNA encoding a protein comprising the amino acid sequence of SEQ ID NO: 1, 2, 26, 28, 30, 32, 34, 36, 38, 40, or 42;
   (b) a DNA comprising the sequence of SEQ ID NO: 3, 4, 25, 27, 29, 31, 33, 35, 37, 39, or 41;
   (c) a DNA encoding a protein comprising an amino acid sequence with one or more amino acid substitutions, deletions, insertions, and/or additions in the amino acid sequence of SEQ ID NO: 1, 2, 26, 28, 30, 32, 34, 36, 38, 40, or 42; and
   (d) a DNA hybridizing under stringent conditions with the sequence of SEQ ID NO: 3, 4, 25, 27, 29, 31, 33, 35, 37, 39, or 41.
[10] An EMT-inducing agent, which is an isolated protein encoded by a DNA of any one of the following (a) to (d):
   (a) a DNA encoding a protein comprising the amino acid sequence of SEQ ID NO: 1, 2, 26, 28, 30, 32, 34, 36, 38, 40, or 42;
   (b) a DNA comprising the sequence of SEQ ID NO: 3, 4, 25, 27, 29, 31, 33, 35, 37, 39, or 41;
   (c) a DNA encoding a protein comprising an amino acid sequence with one or more amino acid substitutions, deletions, insertions, and/or additions in the amino acid sequence of SEQ ID NO: 1, 2, 26, 28, 30, 32, 34, 36, 38, 40, or 42; and
   (d) a DNA hybridizing under stringent conditions with the sequence of SEQ ID NO: 3, 4, 25, 27, 29, 31, 33, 35, 37, 39, or 41.
[11] An EMT-suppressing agent, which is an antisense oligonucleotide targeting a DNA sequence comprising the sequence of SEQ ID NO: 3, 4, 25, 27, 29, 31, 33, 35, 37, 39, or 41.
[12] An EMT-suppressing agent, which is a double-stranded RNA comprising a sequence identical or similar to a portion of a DNA comprising the sequence of SEQ ID NO: 3, 4, 25, 27, 29, 31, 33, 35, 37, 39, or 41.
[13] A pharmaceutical for treating cancer, which comprises the EMT-suppressing agent of [11] or [12] as an active ingredient.
[14] A method of screening for a candidate substance for an agent for suppressing Snail activity, wherein the method comprises the following steps of (a) to (c):
   (a) injecting a vector which comprises a reporter gene operably linked under the control of the E-cadherin promoter, and a test substance into a one-cell-stage embryo;
   (b) measuring the expression level of the reporter gene; and
   (c) selecting a compound that reduces or increases the measured expression level of the reporter gene, compared with the measured expression level in the absence of the test substance.
[15] A wound healing agent, which comprises the regulatory agent of Snail activity of any one of [1] to [3], or the EMT-inducing agent of any one of [8] to [10], as an active ingredient.
[16] An anti-inflammatory agent, which comprises the agent for suppressing Snail activity of [4] or [5], or the EMT-suppressing agent of [11] or [12], as an active ingredient.
[17] A regulatory agent of a protein activity that requires zinc, which comprises a protein comprising the amino acid sequence of SEQ ID NO: 1, 2, 26, 28, 30, 32, 34, 36, 38, 40, or 42 as an active ingredient.

### Brief Description of the Drawings

Fig. 1 a shows a diagram of a zebrafish LIV 1 protein and its putative amino acid sequence. The locations of eight predicted transmembrane domains (marked (3) in the figure), the CPALLY motif (marked (2) in the figure), and the histidine rich repeats (marked (1) in the figure) are shown. Roman numerals indicate the transmembrane domains. The underlined regions in the amino acid sequence shown by a number in parentheses correspond to the regions in the protein diagram indicated by the same number in parentheses.
Fig. 1b shows a comparison between zebrafish LIV1 (LZT-Zf3) and the human LZT subfamily of ZIP transporter proteins (LZT-H). The sequences are across the transmembrane domains IV and V, containing the conserved HNF motif and HEXPHE motif. Amino acids that correspond to those in the zebrafish LIV1 protein are emphasized (enclosed within a border).
Figs. 1c-j show the expression of zebrafish LIV1 mRNA in normal embryos (Figs. 1c-h), a STAT3D4 control morpholino-injected embryo (Fig. 1i), and a STAT3-morpholino-injected embryo (Fig. 1j) at the gastrula stage. Figs. 1c-e, i and j show lateral views; Figs. 1f and h show animal-pole views; and Fig. 1g shows a dorsal view. Dorsal is to the right in Figs. 1c-f, i and j .

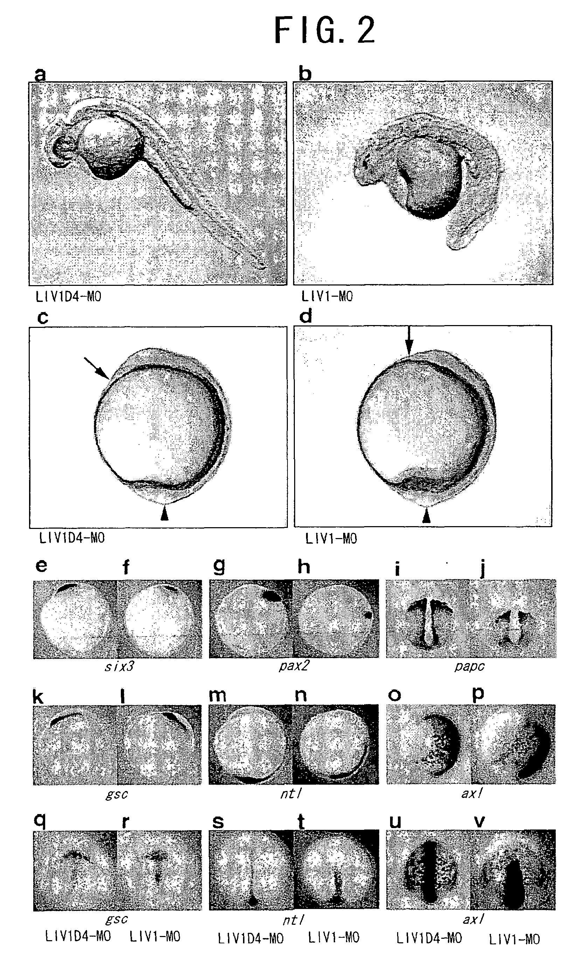

Figs. 2a-d show the phenotypes one day after fertilization (Figs. 2a and b; side views, with anterior to the left) and at the end of gastrulation (Figs. 2c and d; lateral views, with dorsal to the right). The arrows in Fig. 2c and Fig. 2d indicate the anterior limit of the hypoblast (Polster). The black triangles indicate the posterior limit of the hypoblast (tail bud). Figs. 2e-v show the expression of the various markers. Figs. 2e-h and k-p show lateral views, with dorsal to the right, at the one-somite stage. Figs. 2i-j and s-v show dorsal views at the one-somite stage. Figs. 2q and r show animal-pole views at the one-somite stage.

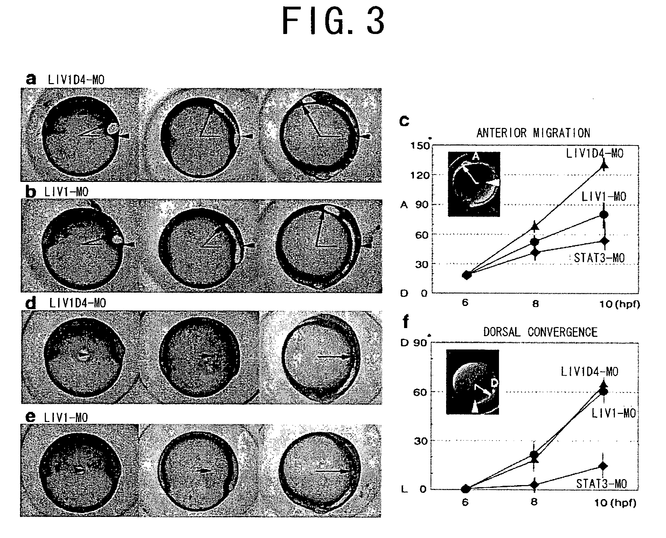

Fig. 3 shows the results of cell-tracing experiments. Fig. 3a: axial mesendodermal cells in a normal control embryo; Fig. 3b: axial mesendodermal cells in a LIV1-depleted embryo; Fig. 3d: lateral mesendodermal cells in a normal control embryo; and Fig. 3e: lateral mesendodermal cells in a LIV1-depleted embryo. The animal pole is facing up, and dorsal is to the right in Figs. 3a, b, d, and e. Figs. 3c and f show graphs comparing anterior migration (Fig. 3c), and dorsal convergence (Fig. 3f) of labeled cell groups in the control (LIV1D4-MO), LIV1-depleted (LIV1-MO), and Stat3-depleted (Stat3-MO) embryos. The arrows indicate the leading edge of anterior migrating axial mesendodermal cells (Fig. 3c) and dorsal converging lateral mesendodermal cells (Fig. 3f); and the black triangles indicate the initial position at the shield stage.
Fig. 4a shows a scheme illustrating cell transplantation experiments. Figs. 4b-m show the migration of prechordal mesendodermal cells obtained from predetermined morpholino- and mRNA-injected donors after being transplanted into the shield region of a predetermined morpholino-injected host at the early gastrula stage. All panels show the transplanted host embryos at the end of gastrulation. The donors (donor 1 and donor 2) and hosts in each panel were as follows:
   Fig. 4b: donor 1: LIV1D4-MO, donor 2: LIV1-MO, host: LIV1D4-MO Host;
   Fig. 4c: donor 1: LIV1D4-MO, donor 2: LIV1-MO + LiV1, host: LIV1D4-MO Host;
   Fig. 4d: donor 1: LIV1D4-MO, donor 2: LIV1-MO, host: LIV1-MO Host;
   Fig. 4e: donor 1: LIV1D4-MO, donor 2: LIV1-MO + LiV1, host: LIV1-MO Host;
   Fig. 4f: donor 1: STAT3D4-MO, donor 2: STAT3-MO, host: STAT3D4-MO Host;
   Fig. 4g: donor 1: STAT3D4-MO, donor 2: STAT3-MO + STAT3, host: STAT3D4-MO Host;
   Fig. 4h: donor 1: STAT3D4-MO, donor 2: STAT3-MO + STAT5, host: STAT3D4-MO Host;
   Fig. 4i: donor 1: STAT3D4-MO, donor 2: STAT3-MO + LiV1, host: STAT3D4-MO Host;
   Fig. 4j: donor 1: STAT3D4-MO, donor 2: Snail1-MO, host: STAT3D4-MO Host;
   Fig. 4k: donor 1: STAT3D4-MO, donor 2: Snaill-MO + Snail1, host: STAT3D4-MO Host;
   Fig. 41: donor 1: STAT3D4-MO, donor 2: Snail1-MO + LiV1, host: STAT3D4-MO Host; and

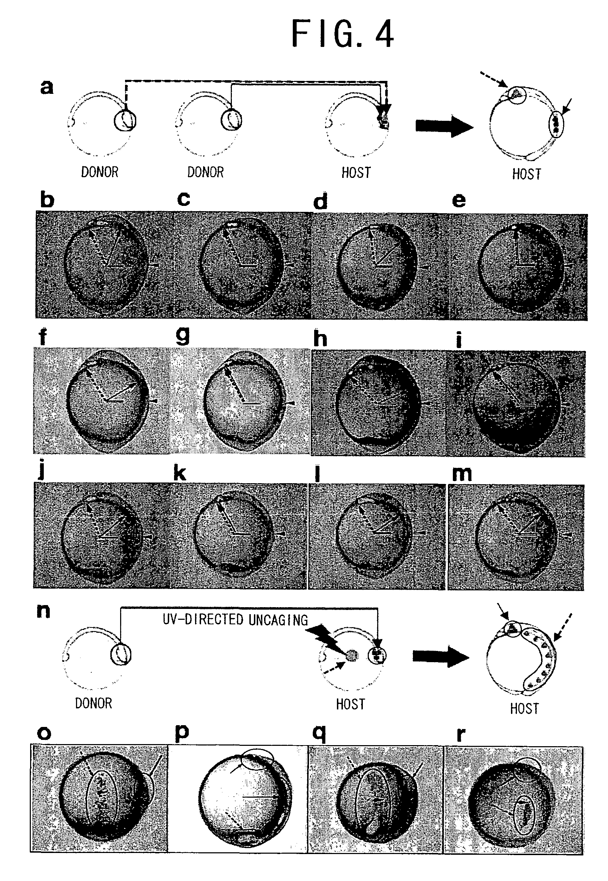

   Fig. 4m: donor 1: STAT3D4-MO, donor 2: STAT3-MO + LiV1 + Snail1-MO, host: STAT3D4-MO Host.

   Black triangles indicate the initial transplant position, and arrows indicate the leading edge of the anterior migrating transplanted cells. Dotted arrows indicate the migration of donor 1 cells, and solid arrows indicate the migration of donor 2 cells. Fig. 4n shows a scheme illustrating cell tracing experiments. Figs. 4o-r show lateral mesendodermal cells in hosts (Host; circled area indicated by the dotted arrow) transplanted with prechordal mesendodermal cells (Donor; circled area indicated by the solid arrow) at the end of gastrulation. The donor and host in the each panel are as follows:
   Fig. 4o: donor: STAT3-MO, host: STAT3-MO Host;
   Fig. 4p: donor: STAT3-MO + STAT3, host: STAT3-MO Host;
   Fig. 4q: donor: STAT3-MO + STAT5, host: STAT3-MO Host; and
   Fig. 4r: donor: STAT3-MO + LiV1, host: STAT3-MO Host.

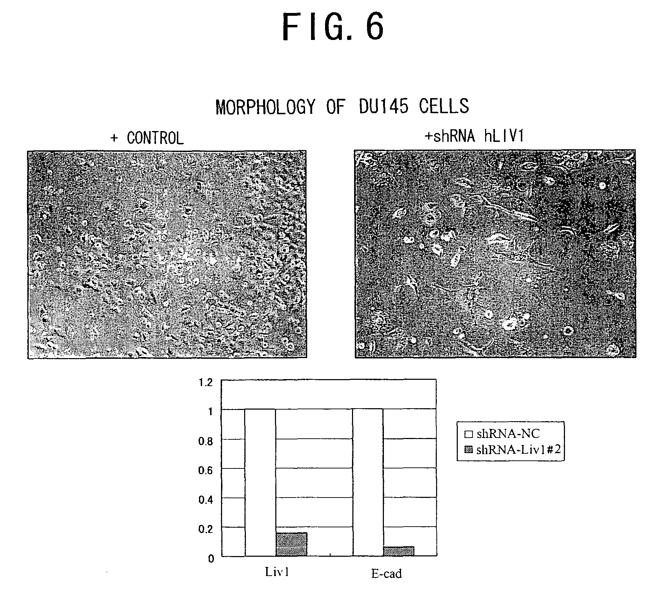

   The arrows with black triangles indicate the leading edge of labeled cells dorsally converging from the initial position.
Figs. 5a-d show the phenotypes of various morpholino-oligonucleotide-injected embryos at the end of the gastrulation stage (lateral views, with dorsal to the right). Fig. 5a: LIV1D4 control-morpholino-injected embryo; Fig. 5b: LIV1-morpholino-injected embryo; Fig. 5c: STAT3-morpholino-injected embryo; and Fig. 5d: Snaill-morpholino-injected embryo. The arrows indicate the anterior limit of the hypoblast. Figs. 5e-h show the morphology of organizer cells in the anterior-most limit of the hypoblast in each embryo at the mid-gastrula stage. Fig. 5e: Control; Fig. 5f: LIV1-morphant; Fig. 5g: STAT3-morphant; and Fig. 5h: Snail1-morphant. Figs. 5i-p show the expression of LIV1 (Figs. 5i-1) and Snail1 (Figs. 5m-p) in each embryo at the early gastrula stage (6 hpf.). Figs. 5i and m: Control; Figs. 5j and n: LIV1-morphant; Figs. 5k and o: STAT3-morphant; and Figs. 51 and p: Snaill-morphant. All panels in Figs. 5i-p show animal-pole views.
Fig. 5q shows Snail-dependent LIV1 activity using the E-cadherin promoter activity as an indicator. Zebrafish LIV1 RNA (1, 10, or 100 pg/embryo; lanes 6-14); mouse Snail RNA (1, 10, or 100 pg/embryo; lanes 2-4 and 9-11); and/or zebrafish Snail1 morpholino (10 ng/embryo; lanes 5 and 12-14). Fig. 5r shows a model for the interaction between LIV1 and Snail in EMT.
   The upper half of Fig. 6 shows photographs of the morphological changes in DU145 cells after using RNAi to silence the endogenous human LIV1 in DU145 cells. The lower half of Fig. 6 is the results of using RT-PCR to show the expression levels of LIV1 and E-cadherin in the cultured cells. shRNA-NC indicates cells transfected with control shRNA, and shRNA-Liv1#2 indicates cells where LIV1 is silenced by shRNA.

### Best Mode for Carrying Out the Invention

The present invention relates to LIV1, a downstream target of STAT3. As described above, the present inventors revealed that LIV 1 gene expression regulates EMT by influencing the activity of Snail. Therefore, the LIV1 protein can be used to regulate Snail activity and EMT.

Herein, regulatory agents of Snail activity are defined as substances capable of positively regulating the activity of the zinc finger protein Snail, directly or indirectly. By regulating Snail activity, the regulatory agents of Snail activity may be capable of changing cell adhesion and inducing epithelial-mesenchymal transition (EMT), or of affecting cancer metastasis and progression.

Preferable examples of substances that can be used as such regulatory agents of Snail activity may be isolated LIV1 proteins comprising the amino acid sequence of SEQ ID NO: 1 or 2. SEQ ID NOs: 3 and 4 show examples of DNA sequences encoding the LIV1 protein. In addition, proteins similar to LIV1 may be used as the regulatory agents of Snail activity of this invention. The proteins similar to LIV1 may preferably be isolated proteins of the human LZT subfamily, comprising the amino acid sequence of SEQ ID NO: 26, 28, 30, 32, 34, 36, 38, 40, or 42. DNA sequences encoding the human LZT subfamily are shown in SEQ ID NO: 25, 27, 29, 31, 33, 35, 37, 39, and 41. Herein, the term "LIV 1 protein" includes proteins similar to LIV1 unless otherwise specifically stated.

Herein, the term "isolated" means that a substance is separated from its original environment (for example, from the natural environment, for a naturally occurring substance), and that the environment of the substance has been artificially changed. In addition, the term "isolation" means comprising a compound present in a sample where the compound of interest is substantially rich, and/or a compound present in a sample where the compound of interest is partially or substantially purified. Herein, the term "substantially purified" means that a compound is separated from its natural environment, and comprises less than 60% of other naturally associated components, preferably less than 75%, and most preferably less than 90%.

The above LIV 1 proteins may be prepared by a variety of methods well known to those skilled in the art. For example, such a protein may be prepared by producing it in a transformant carrying a vector to which a DNA comprising the nucleotide sequence of SEQ ID NO: 3, 4, 25, 27, 29, 31, 33, 35, 37, 39, or 41 has been inserted, and then purifying it. Vectors for such use may be appropriately selected depending on the translation system used for protein production. In addition, LIV 1 is known to be expressed in hormonal tissues such as breast, prostate, hypophysis, and brain (Non-Patent Document 19). Thus, LIV1 protein may be purified from cell extracts expressing LIV1, for example, from human and zebrafish cell extracts of the above hormonal tissues, by preparing an anti-LIV1 protein antibody according to known methods and creating an affinity column using the antibody.

The above-described proteins that are similar to LIV 1 are capable of regulating the activity of Snail and may include, for example, proteins comprising an amino acid sequence with one or more amino acid substitutions, deletions, insertions, and/or additions in the amino acid sequence of SEQ ID NO: 1, 2, 26, 28, 30, 32, 34, 36, 38, 40, or 42, and proteins encoded by DNAs that hybridize under stringent conditions with a DNA comprising the nucleotide sequence of SEQ ID NO: 1.

Examples of methods that can be used to prepare the above proteins, which are similar to LIV 1, include methods that carry out hybridization with a nucleotide sequence encoding LIV1 to obtain highly homologous DNAs, prepare transformants using these DNAs, and produce the desired proteins in those transformants. For example, a highly homologous DNA may be obtained from human or non-human vertebrate cells or such by performing hybridization under stringent conditions, using part of the nucleotide sequence of SEQ ID NO: 3, 4, 25, 27, 29, 31, 33, 35, 37, 39, or 41 as a probe.

Those skilled in the art can appropriately select the above stringent hybridization conditions. For example, pre-hybridization is carried out in a hybridization solution containing 25% formamide, or 50% formamide under more stringent conditions, and 4x SSC, 50 mM Hepes (pH7.0), 10x Denhardt's solution, and 20 µg/ml denatured salmon sperm DNA at 42°C overnight. A labeled probe is then added to the solution and hybridization is carried out by incubation at 42°C overnight. Post-hybridization washes are carried out at different levels of stringency, including the moderately stringent "1x SSC, 0.1% SDS, 37°C", highly stringent "0.5x SSC, 0.1% SDS, 42°C", and more highly stringent "0.2x SSC, 0.1% SDS, 65°C" conditions. As the stringency of the post-hybridization washes increases, DNAs with greater homology to the probe sequence are expected to be isolated. The above-described combinations of SSC, SDS, and temperature are merely examples of washing conditions. Those skilled in the art can achieve the same stringencies as those described above by appropriately combining the above factors or others (such as probe concentration, probe length, or hybridization period) that affect hybridization stringency.

Polypeptides encoded by DNAs isolated using such hybridization techniques will usually comprise amino acid sequences highly homologous to the polypeptides identified by the present inventors. "High homology" refers to sequence homology of at least 40% or more, preferably 60% or more, further preferably 80% or more, further preferably 90% or more, further preferably at least 95% or more, and further preferably at least 97% or more (for example, 98% to 99%). Amino acid sequence identity can be determined, for example, using the BLAST algorithm of Karlin and Altschul (Proc. Natl. Acad. Sci. USA. 87:2264-2268, 1990; Proc. Natl. Acad. Sci. USA 90: 5873-5877, 1993). A program called BLASTX has been developed based on this algorithm (Altschul et al., J. Mol. Biol. 215: 403-410, 1990). When using BLASTX to analyze amino acid sequence identity, the parameters are, for example, a score of 50 and a word length of 3. When using the BLAST and Gapped BLAST programs, the default parameters for each program are used. Specific methodology for these analysis methods is well known (http://www.ncbi.nlm.nih.gov).

The above LIV1-like proteins may be prepared by other known methods. For example, LIV1 DNA may be artificially altered using site-directed mutagenesis, such as methods for constructing deletion mutants using exonuclease, or cassette mutagenesis; the altered LIV1 DNA may then be used to prepare a desired protein.

As described in the Examples, the activity of proteins prepared as LIV1-like proteins as regulatory agents of Snail activity may be determined by using their activity in suppressing the expression of the cell adhesion molecule E-cadherin as an indicator, since Snail protein suppresses the expression of E-cadherin.

Another preferable example of substances available as regulatory agents of Snail activity is isolated DNAs comprising the sequence of SEQ ID NO: 3, 4, 25, 27, 29, 31, 33, 35, 37, 39, or 41. The above DNAs encode LIV1 protein, and once introduced into a cell, they can indirectly regulate Snail activity through LIV1 protein expression.

The above DNAs may be prepared from cDNAs from LIV1-expressing cells by hybridization techniques well known to those skilled in the art, using a part of the sequence of SEQ ID NO: 3, 4, 25, 27, 29, 31, 33, 35, 37, 39, or 41 as a probe. In addition, the DNAs may be obtained from mRNAs by performing RT-PCR using a part of the sequence of SEQ ID NO: 3, 4, 25, 27, 29, 31, 33, 35, 37, 39, or 41 as a primer. Alternatively, such DNAs may be artificially synthesized using a commercially available DNA synthesizer.

Furthermore, DNAs similar to the above DNAs are another example of regulatory agents of Snail activity. Such DNAs include DNAs that hybridize under stringent conditions with the sequence of SEQ ID NO: 3, 4, 25, 27, 29, 31, 33, 35, 37, 39, or 41, and that encode a protein capable of regulating Snail activity. Methods for preparing such DNAs are as described above.

Each of the above-described DNAs may be inserted into appropriate vectors for use. Such DNAs inserted into vectors is another embodiment of the regulatory agents of Snail activity. The vectors for use may be expression vectors appropriately selected depending on the purpose. Specifically, mammalian-derived vectors (such as pcDNA3 (Invitrogen Inc.), pEGF-BOS (Nucleic Acids Res., 18(17), p.5322, 1990), pEF, pCDM8, and pCXN), insect cell-derived vectors (such as the "Bac-to-Bac baculovirus expression system" (Invitrogen Inc.) and pBacPAK8), plant-derived expression vectors (such as pMH1 and pMH2), animal virus vectors (such as pHSV, pMV, and pAdexLcw), retroviral vectors (such as pZIPneo), yeast-derived vectors (such as "Pichia Expression Kit" (Invitrogen Inc.), pNV11, and SP-Q01), *Bacillus Subtilis*-derived vectors (such as pPL608 and pKTH50), *E. coli* vectors (such as the M13 vector series, pUC vector series, pBR322, pBluescript, and pCR-Script) and such may be used. Herein, the use of vectors that can be expressed in mammalian cells is preferable, and the use of expression vectors is preferable. Vectors maybe introduced into cells by methods selected from, for example, calcium phosphate methods (Virology, Vol.52, p.456, 1973), DEAE-dextran method, methods using cationic liposome DOTAP (Roche Diagnostics), electroporation methods (Nucleic Acids Res., Vol15, p.1311, 1987), lipofection methods (J. Clin. Biochem. Nutr., Vol.7, p.175 1989), transfection mediated by viral infection (Sci. Am., p.34, 1994), and particle guns.

As described above, a variety of regulatory agents of Snail activity directly or indirectly induce activation of the zinc finger protein Snail. Furthermore, Snail activation contributes to EMT induction, and EMT relates to gastrulation in embryos, regeneration of organs and tissues, and cancer metastasis and progression. Thus, the regulatory agents of Snail activity may be used to elucidate the developmental processes and the mechanisms of cancer metastasis and progression. Furthermore, such regulatory agents may be effectively used as agents for promoting organ- and tissue-regeneration in regenerative medicine. In addition, because the regulatory agents promote cell regeneration, they may be applied as wound-healing agents.

The present invention relates to agents for suppressing Snail activity. Suppression of Snail activity inhibits Snail's suppression of adhesion molecule expression, which leads to inhibition of EMT induction and prevention of cancer metastasis and progression.

Examples of the agents for suppressing Snail activity may be antisense oligonucleotides targeting DNAs or mRNAs that encode LIV 1. Because LIV1 can regulate Snail activity, as demonstrated by the present inventors, antisense oligonucleotides against DNAs encoding LIV1 are expected to inhibit expression of the endogenous LIV1 gene, and thereby negatively regulate Snail activity. Examples of such antisense oligonucleotides are antisense oligonucleotides that target DNAs comprising the sequence of SEQ ID NO: 3, 4, 25, 27, 29, 31, 33, 35, 37, 39, or 41, or mRNAs generated from these DNAs. As an example, oligonucleotides of SEQ ID NOs: 5 and 6 may be used. In addition, any oligonucleotides may be included in the antisense oligonucleotides of this invention, as long as they can hybridize with a part of a DNA comprising the sequence of SEQ ID NO: 3, 4, 25, 27, 29, 31, 33, 35, 37, 39, or 41, and effectively inhibit LIV1 expression. Such oligonucleotides need not be completely complementary to the DNAs or corresponding mRNAs comprising the nucleotide sequence of SEQ ID NO: 3, 4, 25, 27, 29, 31, 33, 35, 37, 39, or 41.

The above-described antisense oligonucleotides may be inserted in an appropriate vector for use, depending on the purpose. For example, vectors used in gene therapy applications may be appropriately selected from viral vectors such as retrovirus vectors, adenovirus vectors, and vaccinia virus vectors, non-viral vectors such as cationic liposomes and ligand-DNA complexes, and such. Further, antisense oligonucleotides can be administered as naked plasmid DNAs (naked pDNAs) along with a large volume of aqueous solution, without using a carrier.

Another example of the agents for suppressing Snail activity may be double-stranded RNAs comprising a sequence identical or similar to a part of a DNA encoding LIV1. Such double-stranded RNAs comprising a sequence identical or similar to a target gene sequence may trigger RNA interference (RNAi), which inhibits expression of the target gene. RNAi is a phenomenon caused when a double-stranded RNA (dsRNA) is introduced into cells, specifically degrading the cellular mRNA that corresponds to the RNA sequence and preventing its expression as a protein. The regions forming double strands may form double strands over an entire region, or parts (both or either of the termini, for example) may form single strands. Thus, the double-stranded RNAs of this invention may also comprise regions that are not double stranded. The oligo RNAs used for RNAi are often 10-100 bp, and normally 19-23 bp. RNAi may be performed according to the methods described in Nature, Vol.391, p.806, 1998; Proc.Natl.Acsd.Sci.USA, Vol.95, p.15502, 1998; Nature, Vol.395, p.854, 1998; Proc.Natl.Acsd.Sci.USA, Vol.96, p.5049, 1999; Cell, Vol.95, p.1017, 1998; Proc.Natl.Acsd.Sci.USA, Vol.96, p.1451, 1999; Proc.Natl.Acsd.Sci.USA, Vol.95, p.13959, 1998; Nature Cell Biol., Vol.2, p.70, 2000, etc.

The above-described agents for suppressing Snail activity may be used for studying Snail-related embryology and oncology, and in addition, they can conceivably be used as therapeutic pharmaceuticals for suppressing cancer metastasis and progression by the negative regulation of Snail activity. They are expected to be effective for cancers in tissues where LIV 1 is expressed, and especially effective for breast cancers. In addition, since they can suppress cell adhesion and cell movement, they are expected to suppress the invasion and spread of inflammatory cells, and can therefore be applied as anti-inflammatory agents.

Gene therapy is an example of the methods for using the above agents for suppressing Snail activity for cancer therapy. Gene therapy is a method for treating a disease by introducing an exogenous normal gene into patient cells and changing the cellular phenotype to correct a mutated gene. Gene therapy is thought to be effective for treating not only genetic diseases but also other diseases, such as AIDS and cancers. Gene therapy is classified into: methods where a gene is directly introduced *in vivo* to be incorporated into cells *(in vivo* methods); and methods where patient cells are sampled, a gene is introduced thereto *ex vivo,* and then the cells are transplanted back into the patient *(ex vivo* methods). In *in vivo* gene therapy methods, the above agents for suppressing Snail activity may be introduced *in vivo* by an administration method such as intramuscular injection, local injection, rubbing, and inhalation, either directly or through insertion into a vector.

The proteins, DNAs and oligonucleotides of this invention may be used as pharmaceuticals by directly administrating these substances, or preparing formulations using known formulation techniques. For example, they may be formulated in combination with a pharmacologically acceptable vehicle or stabilizer. The administration route, dose, and methods may be appropriately selected according to the purpose and subject of treatment.

The present invention also provides EMT-inducing agents. As described above, LIV1 activates Snail, which induces EMT. Thus, the above-described regulatory agents of Snail activity may also be used as EMT regulatory agents.

Herein, the term "EMT-inducing agents" refers to substances capable of directly or indirectly inducing epithelial-mesenchymal transition (EMT).

Examples of the EMT-inducing agents may be isolated LIV1 proteins comprising the amino acid sequence of SEQ ID NO: 1, 2, 26, 28, 30, 32, 34, 36, 38, 40, or 42, and proteins similar to LIV 1.

Methods for preparing LIV1 proteins are as described above.

The above-described proteins similar to LIV1 are those capable of inducing EMT. Examples of the proteins may be proteins comprising an amino acid sequence with one or more amino acid substitutions, deletions, insertions, and/or additions in the amino acid sequence of SEQ ID NO: 1, 2, 26, 28, 30, 32, 34, 36, 38, 40, or 42, and proteins encoded by DNAs that hybridize under stringent conditions with a DNA comprising the nucleotide sequence of SEQ ID NO: 3, 4, 25, 27, 29, 31, 33, 35, 37, 39, or 41.

Preparation methods are the same as those described in the section on the regulatory agents of Snail activity. To confirm the activity of an EMT-inducing agent, as described in the Examples, a test substance may be injected into an embryo in which LIV1 is specifically suppressed, and cell migratory movements and morphological changes can be observed through the test substance's effects. If the observed results of cell migratory movements and morphological changes are the same as those in an embryo where LIV 1 is not specifically suppressed, the test substance is considered to have an effect as an EMT-inducing agent.

Another preferable example of a substance useful as an EMT-inducing agent may be an isolated DNA comprising the sequence of SEQ ID NO: 3, 4, 25, 27, 29, 31, 33, 35, 37, 39, or 41. The above-described DNAs encode a LIV1 protein, and once introduced into cells they can indirectly induce EMT through LIV1 protein expression. Furthermore, DNAs similar to the above DNAs and vectors into which the DNAs are inserted can be used as EMT-inducing agents. Methods for preparing them are described above.

Since EMT is involved in the gastrulation of embryos, the regeneration of organs and tissues, and cancer metastasis and progression, these EMT-inducing agents may be used for elucidating the developmental processes and mechanisms of cancer metastasis and progression. Moreover, by promoting cellular regeneration, these agents may be effectively used as agents for promoting organ- and tissue-regeneration in regenerative medicine, and as wound-healing agents.

The present invention relates to EMT-suppressing agents. Suppression of EMT leads to prevention of cancer metastasis and progression by inhibiting EMT induction. For example, the EMT-suppressing agents of this invention are useful for suppressing estrogen receptor-negative (ER(-)) breast cancers and their metastasis.

Since the present inventors revealed that LIV 1 can induce EMT, antisense oligonucleotides targeting DNA or mRNA sequences encoding LIV1 are thought to interfere with the expression of the endogenous LIV1 gene, thereby suppressing EMT. Such antisense oligonucleotides may include those targeting DNAs comprising the sequence of SEQ ID NO: 3, 4, 25, 27, 29, 31, 33, 35, 37, 39, or 41, or mRNAs purified from these DNAs. For example, oligonucleotides of SEQ ID NOs: 5 and 6 may be used. In addition, the antisense oligonucleotides of this invention include any oligonucleotides as long as they can hybridize with a part of a DNA comprising the nucleotide sequence of SEQ ID NO: 3, 4, 25, 27, 29, 31, 33, 35, 37, 39, or 41, or the like, and effectively inhibit the expression of LIV1. Such antisense oligonucleotides need not be completely complementary to the above DNAs comprising the sequence of SEQ ID NO: 3, 4, 25, 27, 29, 31, 33, 35, 37, 39, or 41, and the like.

Moreover, double-stranded RNAs comprising a sequence identical or similar to a part of a DNA encoding LIV 1 are also examples of EMT-suppressing agents.

The embodiments for the use of LIV 1-suppressing agents are the same as for the agents for suppressing Snail activity.

The present invention relates to methods for screening candidate substances for agents for suppressing Snail activity. The screening methods of this invention comprise the steps of: injecting one-cell stage embryos with a test substance and a vector which comprises a reporter gene operably linked under the control of the E-cadherin promoter; measuring the expression level of the reporter gene; and selecting a compound that reduces or increases the measured expression level of the reporter gene, compared with that in the absence of the test substance.

The reporter genes used in this invention are not limited as long as their expression is detectable. For example, luciferase, CAT gene, and β-galactosidase may be used.

Examples of test substances for the screening methods of this invention include expression products of gene libraries, synthetic low-molecular-weight compounds, synthetic peptides, natural compounds, and such.

Further, the present invention relates to regulatory agents of protein activities that require zinc (Zn). As described above, LIV1 protein is a zinc transporter, and is thought to regulate not only the activity of Snail, but also that of other proteins that require Zn. Thus, LIV 1 is expected to be useful as a regulatory agent of protein activities that require Zn The regulatory agents of protein activities that require zinc of the present invention may be useful in studies for elucidating the molecular mechanisms to which proteins that requires Zn are related, and they may be applied as immune regulatory agents.

All prior art literatures cited in this description are incorporated herein by reference.

### Examples

### [Example 1] Identification of STAT3 target genes

To elucidate the molecular mechanism of STAT3, which is involved in gastrulation, downstream target genes of STAT3 were identified. First, to isolate STAT3-induced genes induced in the gastrula organizer, subtraction screening was performed using normal embryos and STAT3-morphant embryos.

The term "morphant embryo" refers to an embryo in which the expression of a particular gene is fully inhibited by morpholino. In this Example, morphant embryos with inhibited expression of STAT3 gene were generated. To generate the morphant embryos, two kinds of STAT3-morpholino nucleotides (STAT3-MO) were prepared. STAT3D4-MO were also prepared as controls, by adding a mutation to the STAT3-MO. These morpholino oligonucleotides were generated by GENE TOOLS. The sequences of the above morpholino oligonucleotides are shown below:
STAT3-MO:

   5'-CTCAAGGTTTCAGATAAATCGTCCT-3' (SEQ ID NO: 9)

   5'-GCCATGTTGACCCCTTAATGTGTCG-3' (SEQ ID NO: 10)
STAT3D4-MO:

   5'-CTCtAGGaTTCAGATAAAaCGTgCT-S' (SEQ ID NO: 11)

   5'-GCCtTGTaGACCCCTTAAaGTGaCG-3' (SEQ ID NO: 12)

The two kinds of STAT3-MO were injected into the yolk of one-cell stage embryos in equal amounts to prepare the morpholino embryos (STAT3-MO-injected). The control embryos (STAT3D4-MO-injected) were similarly generated.

Poly (A)⁺ RNAs were isolated from the STAT3-MO-injected mid-gastrula-stage embryos and STAT3D4-MO-injected mid-gastrula-stage embryos. Subtraction screening was performed using PCR-Select™ cDNA subtraction kits (Clontech), as per to the manufacturer's protocol.

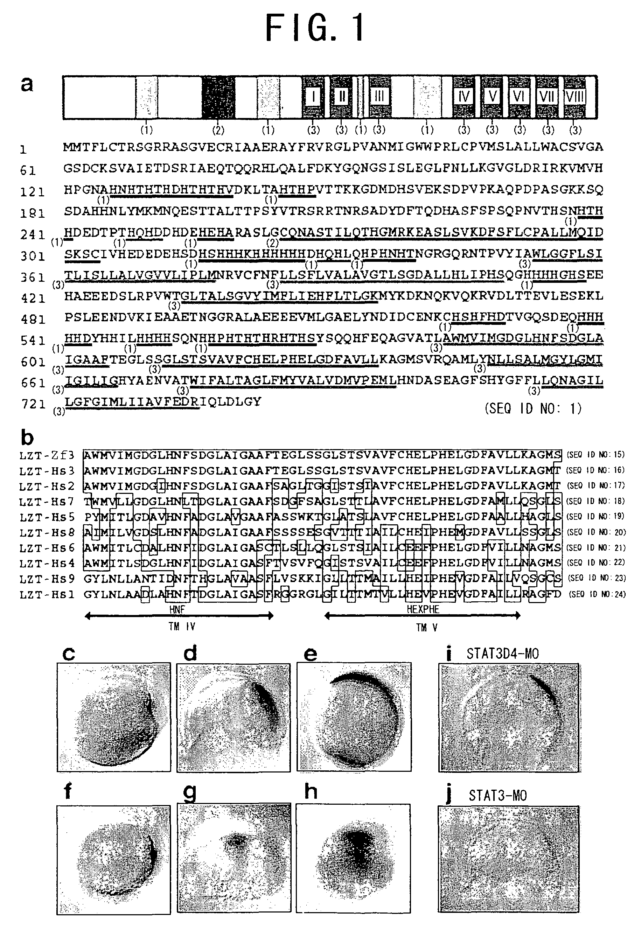

The sequences of the cDNAs isolated by the above subtraction screening were determined using standard methods, and subjected to database searches. Unexpectedly, one of the cDNAs isolated by the present inventors turned out to be zebrafish LIV1 (LZT-Zf3; SEQ ID NO:3). The deduced amino acid sequence of LIV1 and the LIV1 protein domains are shown in SEQ ID NO: 1 and Fig. 1a. Since the sequence comprises a long extracellular N-terminus, a short extracellular C-terminus, a long variable region in the cytoplasmic loop between the TMIII and TMIV domains, an HNF motif in the TMIV domain, an HEXPHE motif in the TMV domain, and eight transmembrane (TM) domains comprising extensive histidine-rich repeats, it was characterized as the ZIP family of zinc transporters (Taylor K.M., and Nicholson R.I. The LZT proteins; the LIV-1 subfamily of zinc transporters. Biochim. Biophys. Acta 1611: 16-30 (2003)). The above cDNAs were identified as zebrafish homologues of human LIV1 by phylogenic tree analysis and alignment of the regions spanning the transmembrane domains IV and V of the LZT subfamily of ZIP transporters (LZT-Hs3; Fig. 1b). The present inventors had previously demonstrated that STAT3 is activated in prechordal mesendodermal cells (Yamashita S. et al. Stat3 Controls Cell Movements during Zebrafish Gastrulation. Dev Cell 2, 363-75 (2002)).

Based on the above findings, the expression of LIV1 mRNA in zebrafish embryos at the gastrula stage was examined using the WISH method.

The whole-mount *in situ* hybridization (WISH) method was basically performed using the zebrafish LIV1 cDNA sequence (SEQ ID NO: 1) as a probe for LIV1 mRNA, according to previously described methods (Yamashita S. et al. Stat3 Controls Cell Movements during Zebrafish Gastrulation. Dev Cell 2, 363-75 (2002)).

The results showed that zebrafish LIV1 mRNA was maternally expressed, and that embryonic transcripts increased at the shield stage and accumulated in the prechordal mesendodermal cells in which STAT3 was activated (Figs. 1c-h). Expression of LIV 1 mRNA in the gastrula organizer was completely absent from STAT3-morphant embryos, indicating that LIV1 is a downstream target of STAT3 (Figs. 1i and j).

### [Example 2] Examination of LIV1 function in early stage embryos

To examine the role of LIV1 in zebrafish embryos in the early stages of gastrulation, LIV1 antisense morpholino (LIV1-MO), a specific translation inhibitor (Non-Patent Document 21: Nasevicius A. and Ekker, S.C. Effective targeted gene 'knockdown' in zebrafish. Nat Genet 26, 216-20 (2000)), was used to generate embryos lacking in LIV1 activity, and the effect of losing LIV1 activity was observed. The LIV1-MO and control oligonucleotide sequences are shown below:
LIV1-MO:

   5'-CGGAAACAGCGCGAGTGTCTTTTGT-3' (SEQ ID NO: 5)

   5'-ACCGTGTGCAAAGAAACGTCATCAT-3' (SEQ ID NO: 6)
LIV1D4-MO (a control in which the sequence of LIV1-antisense morpholin was mutated):

   5'-CGcAAAaAGCGaGAGTGTaTTaTGT-3' (SEQ ID NO: 7)

   5'-AcgGTcTGCAAAcAAACGTgATgAT-3' (SEQ ID NO: 8)

Morpholino embryos were generated by using these morpholino oligonucleotides as described for the above STAT3-morpholino embryos.

Embryos injected with LIV1-MO were confirmed to have mislocated heads and shortened anterior-posterior axis in the later and final stages of gastrulation. On the other hand, embryos injected with the control LIV1D4-MO did not show any obvious phenotypic changes (Figs. 2a-d). In the LIV1-MO-injected zebrafish embryos, the axial anterior-most mesendodermal (Polster) and anterior ectodermal structures could be observed with the naked eye, and were transposed toward the vegetal pole, up to 45 degrees from the normal position at the animal pole. Moreover, although the axial hypoblast (notochord and somite) was formed in the LIV1-MO-injected zebrafish embryos, it was shorter and thicker than normal. This suggests that the anterior movement of the axial mesendoderm was severely impaired, whereas the involution, epiboly, and dorsal convergence movements were normal. Consistent with this, at the shield stage the LIV1-MO-injected embryos showed a germ ring (a hypoblast of mesendoderm formed by the involution movement of surrounding cells) and embryonic shield (a thickening of the dorsal surroundings; gastrula organizer) (data not shown). Taken together, these results indicate that during the gastrulation process the LIV 1-depleted-zebrafish-embryos retained the ability to form mesendoderm, organizer, and dorsal-ventral and anterior-posterior axes, and the ability to initiate involution, epiboly, and dorsal convergence movements. However, axial mesendodermal cells in the LIV1-MO-injected embryos did not move anteriorly, and head positioning and elongation of the anterior-posterior axis were impaired.

Next, marker gene expression was confirmed using the WISH method. Six3, pax2, goosecoid, no tail, axial, and papc were used as the markers.

The results also provided evidence for morphological abnormalities associated with the loss of LIV1 function (Figs. 2e-v). The markers for the forebrain (six3), mid-hindbrain bundle (pax2), anterior axial mesoderm (goosecoid), posterior axial mesoderm (no tail and axial), paraxial mesoderm (papc), and endoderm (axial) were expressed in the LIV1-MO-injected embryos at the late gastrula stage, but the expression domains of these marker genes were mislocated on the vegetal pole. In addition, shortening of the expression domain in the anterior-posterior axis, and slight mediolateral expansion were confirmed in the LIV1-MO-injected embryos. Consistent with normal fate specification and regionalization in LIV1-deficient embryos at the late gastrula stages, normal expression of the above mesendodermal genes was observed at the earlier stages of development (data not shown). These results indicate that LIV 1 plays an essential role in the gastrulation process by affecting cell movement, without significantly altering the early cell-fate specification of mesendodermal cells.

### [Example 3] Examination of LIV 1 function during gastrulation

To further examine the effect of LIV 1 on cell movement during gastrulation, cell-tracing experiments were performed in zebrafish embryos using 4,5-dimethoxy-2-nitrobenzyl (DMNB)-caged fluorescein dextran (Kozlowski, D.J. and Weinberg, E.S. Photoactivatable (caged) fluorescein as a cell tracer for fate mapping in the zebrafish embryo. Methods Mol Biol 135, 349-55 (2000)) (Figs. 3a-f).

Cell-tracing experiments were performed essentially according to previously described methods (Yamashita, S. et al. Stat3 Controls Cell Movements during Zebrafish Gastrulation. Dev Cell 2, 363-75 (2002)). Specifically, 100 pl of 0.5% DMNB-caged fluorescein-dextran (molecular weight 10,000; Molecular Probes) was injected into the yolk cell of one-cell-stage embryos previously injected with 10 ng of LIV1D4-MO or LIV1-MO. To uncage at the shield stage, a beam of ultraviolet light (λ < 360 nm), generated using a DAPI filter set, was directed for one second at the dorsal or lateral blastoderm margin. Then, the cells in the embryonic shield were labeled, and the position of the labeled cells during gastrulation was traced.

At the end of gastrulation the labeled cells in the control LIV1D4-MO-injected embryos (Fig. 3 a) were distributed in the dorsal axial hypoblast along the entire length of the AP axis structure, as reported previously (Yamashita, S. et al. Stat3 Controls Cell Movements during Zebrafish Gastrulation. Dev Cell 2, 363-75 (2002)). In contrast, the anterior movement of the labeled cells in the LIV1-MO-injected embryos (Fig. 3b) was disturbed, resulting in a shortened axial mesendoderm. To monitor the dorsal convergence movements, cells in the lateral blastoderm margin, 90 degrees from the dorsal embryonic shield, were labeled at the shield stage. During gastrulation, the labeled cells in the LIV1-MO-injected embryos moved dorsally and anteriorly, and just as for the control LIV1D4-MO-injected embryos, at the end of gastrulation they extended along the anterior-posterior-axis structure (Figs. 3d and e). These results clearly show that LIV 1 is essential for the active migration of organizer cells, but not for the dorsal convergence of non-axial mesodermal cells.

The above observations suggest that LIV1 is one of the essential STAT3 target genes, and that LIV1 is required for STAT3's cell-autonomous roles, but not for its non-cell-autonomous roles. If this is so, LIV1 is predicted to act cell-autonomously in the active migration of organizer cells.

Transplantation experiments were performed to confirm the above prediction. The transplantation experiments were performed according to previously described methods (Yamashita, S. et al. Stat3 Controls Cell Movements during Zebrafish Gastrulation. Dev Cell 2, 363-75 (2002)). Specifically, donor embryos were prepared by injecting the yolk cell of one-cell-stage embryos with 100 pl of 0.5% rhodamine-dextran (molecular weight 10,000; Molecular Probes) or 100 pl of 0.5% fluorescein-dextran (molecular weight 10,000; Molecular Probes), with 10 ng of predetermined morpholino. A small population of deep cells (10 to 30 cells) obtained from the embryonic shield of donor embryos was transplanted into the embryonic shield of host embryos at the same developmental stage. In addition, the plasmid pCS2 + zebrafish LIV1 (NotI, SP6) was linearized to prepare sense-strand capped mRNA, which was used for injection into cells.

First, prechordal mesendodermal cells, obtained from the embryonic shield of LIV1-MO-injected donor embryos or LIV1D4-MO-injected control donor embryos, were co-transplanted into the embryonic shield of normal host embryos or LIV1-depleted embryos, and the cells were traced (Figs. 4a-e). When the transplanted prechordal mesendodermal cells were derived from control donors, and not from LIV1-depleted donors, the cells migrated anteriorly to the animal pole in both the normal host embryos (Fig. 4b) and the LIV1-depleted host embryos (Fig. 4d). The anterior migration of the LIV1-MO-injected cells was reduced, however, in both the normal control hosts (Fig. 4c) and the LIV 1-depleted hosts (Fig. 4e), it was rescued by co-injecting LIV1 mRNA into the LIV1-depleted donor cells. These results indicate that with regards to the anterior migration of the prechordal mesendoderm, LIV1 acts on cells autonomously.

### [Example 4] Examination of the involvement of LIV1 in the role of STAT3

Next, to examine whether LIV1 can sufficiently restore those functions impaired by STAT3 depletion in the anterior migration of STAT3-depleted prechordal mesendodermal cells, transplantation and cell-tracing experiments were performed. STAT3 mRNA for injection was prepared by linearizing pCS2 + zebrafish STAT3 (NotI, SP6) (Yamashita, S. et al. Stat3 Controls Cell Movements during Zebrafish Gastrulation. Dev Cell 2, 363-75 (2002)).

As shown in Fig. 4f, when STAT3-depleted cells were transplanted into the embryonic shield, the cells did not migrate anteriorly beyond the animal pole. This dysfunction was rescued by the co-injection of STAT3 mRNA (Fig. 4g), but not of STAT5 mRNA (Fig. 4h). The most significant of the results obtained in this Example is that LIV1 mRNA rescued the defects of STAT3-depleted cells (Fig. 4i). These results clearly show that LIV1 sufficiently rescues the anterior migration function of prechordal mesendodermal cells in STAT3-depleted embryos.

The constraints of LIV1, predicted from the above observations, were further clarified from the results of the cell-tracing experiments below, showing that LIV1 mRNA could not rescue dysfunctions in the non-cell-autonomous role of STAT3 in shield cells, that is, dysfunctions in the induction of convergence.

Prechordal mesendodermal cells obtained from donors injected with STAT3-morpholino and predetermined mRNAs were transplanted into the shield region, and the lateral mesendodermal cells in hosts co-injected with STAT3-morpholino and caged FITC were then labeled by UV-directed uncaging at the early gastrula stage. As shown in Figs. 4n-r, reduced convergence observed in the STAT3-depleted host embryos was rescued by the transplantation of prechordal mesendodermal cells from STAT3-depleted donors injected with STAT3 mRNA (Fig. 4p), but not by those cells from STAT3-depleted donors injected with LIV1 mRNA (Fig. 4r).

All these data established that LIV1 is an essential and sufficient target gene for the cell-autonomous roles of STAT3, but not for its non-cell-autonomous roles. LIV1 is suggested to play a key role in the EMT of organizer cells regulated by STAT3 activity.

### [Example 5] Examination of the relationship between the LIV1-induced EMT of organizer cells and Snail

As described above, LIV1 has been shown to induce the EMT of gastrula organizer cells. Accordingly, the present inventors examined whether LIV1 affects the expression and/or activity of Snail during induction of organizer cell EMT by LIV 1.

First, to examine the effect on Snail activity, the phenotypes at the end of gastrulation of LIV1-depleted, STAT3-depleted, and Snail-depleted embryos, and the migratory behavior of organizer cells in the embryos, were analyzed (Figs. 5a-h). LIV1-depleted embryos and STAT3-depleted embryos were prepared as described above. The Snail-MO sequence used for preparing Snail-depleted embryos is shown below:

5'-GTCCACTCCAGTTACTTTCAGGGAT-3' (SEQ ID NO: 13)

5'-CATGCTGAACTCTGAAGTTGATC-3' (SEQ ID NO: 14)

During the gastrulation process, the organizer cells in normal embryos actively weakened the cell-cell adhesion system, and left their marginal region to migrate individually, resulting in full anterior extension along the body axis at the end of gastrulation (Figs. 5a and e). However, in LIV1-depleted embryos, the organizer cells could not weaken their association, resulting in severe disturbance of organizer cell migration, mislocation of the head, and the formation of a shortened anterior-posterior axis structure (Figs. 5b and f). Similar defects in the migratory behavior of organizer cells and body axis extension were also observed in STAT3-depleted embryos (Figs. 5c and g) and Snail-depleted embryos (Figs. 5d and h). These abnormal organizer cell behaviors clearly indicated a severe disturbance of organizer cell EMT in LIV1-depleted embryos, as in Snail-depleted embryos.

Next, Snail expression was examined. Snail1 mRNA used for injection was prepared by linearizing pCS2 + mouse Snail (NotI, SP6) (Yamashita, S. et al. Stat3 Controls Cell Movements during Zebrafish Gastrulation. Dev Cell 2, 363-75 (2002)). LIV1 mRNA and Snail1 mRNA were normally expressed in Snaill-MO-injected embryos and LIV1-MO-injected embryos respectively (Figs. 5i-p).

These results indicate that LIV 1 and Snail 1 are essential for EMT during the gastrulation process, and that the expression of LIV 1 and Snail1 are independently regulated in gastrula organizer cells. On the other hand, cell-autonomous defects in the anterior migration of Snaill-depleted organizer cells were not rescued by co-injection of LIV 1 mRNA (Fig. 41), and vice versa (data not shown). These results suggested that LIV1 may affect Snail activity.

To address the above issue, the effect of LIV1 on the repressor activity of Snail was examined by reporter assay using a Snail-responsive reporter plasmid (Batlle, E. et al. The transcription factor snail is a repressor of E-cadherin gene expression in epithelial tumor cells. Nat Cell Biol 2, 84-9 (2000)) (Fig. 5q).

The reporter assay was essentially performed according to previously described methods (Batlle, E. et al. The transcription factor snail is a repressor of E-cadherin gene expression in epithelial tumor cells. Nat Cell Biol 2, 84-9 (2000)). Specifically, a reporter construct pGL3-E-cadh promoter (2.5 pg/embryo), the pGL3 vector comprising the Luc gene under the control of the human E-cadherin promoter (-178 to +92), and a control reporter vector (pRLtk) (0.5 g/embryo) were injected into one-cell-stage embryos with or without zebrafish LIV1 RNA (1, 10, or 100 pg/embryo), mouse Snail (1, 10, or 100 pg/embryo), or zebrafish Snail morpholino (10 pg/embryo). Firefly luciferase (Luc) and *Renilla reniformis* luciferase (RLluc) activities were measured using the Dual Luciferase Reporter Assay System (Promega) at the shield stage (6 hpf.), according to the manufacturer's protocol. Luciferase activity was always normalized using Rluc activity.

Snail RNA co-injected with the reporter plasmid into zebrafish embryos suppressed transcription from the reporter plasmid in a manner dependent on the dose of Snail RNA (Fig. 5q; lanes 1-4). In contrast, injection of Snail-MO into zebrafish embryos enhanced transcription from the reporter plasmid (Fig. 5q; lane 5). Similarly, LIV1 RNA also suppressed transcription from the reporter plasmid in a dose-dependent manner (Fig. 5q; lanes 6-8). Furthermore, co-injection of various amounts of LIV1 RNA with a constant small amount of Snail RNA enhanced the repressor activity of Snail in a manner dependent on the LIV1 RNA dose (Fig. 5q; lanes 9-11).

As LIV1 is a zinc transporter protein, LIV1 is very likely to regulate the activity of the zinc finger protein Snail. If this is so, reporter suppression by LIV1 is thought to be sensitive to Snail-specific translation inhibitors. As shown in Fig. 5q, LIV1 activity was completely absent in Snaill-MO-injected zebrafish embryos (Fig. 5q; lanes 12-14). In the cell-tracing experiments, the cell-autonomous rescue by LIV1 of the anterior migration defects of STAT3-depleted organizer cells was also sensitive to Snail-MO (Figs. 4i and m). These results, along with the results of phenotypic and gene expression analyses (Figs. 5a-p), provide evidence that LIV1 activates Snail and induces the EMT of organizer cells during gastrulation in zebrafish.

### [Example 6] Analysis of the LIV1 function in DU145 (human prostate cancer cells)

To clarify the function of LIV1 in human cells, LIV1 function was analyzed in DU145 (human prostate cancer cells). The endogenous human LIV1 in DU145 was silenced by RNAi, and changes in cell morphology were observed.

First, DU145 cells were transfected with an hLIV1-targeting shRNA (AGGAGAAAGTAGATACAGA; SEQ ID NO: 43). Transfection was performed using the Lipofectamine method (Invitrogen) in the presence of 10% fetal calf serum (FCS) for ten hours. The piGENE PUR hU6 (iGENE), which carries puromycin resistant marker, was used as a vector for the transfection. As a control experiment, a control shRNA was transfected into other DU145 cells.

Next, DU145 cells into which LIV1 shRNA or control shRNA were respectively introduced, were cultured in the presence of 2 µg/ml of puromycin dihydrochloride (Puromycin Dihydrochloride from Streptomyces alboniger; Nakarai standard, Special Grade) in 10% FCS-DMEM medium for about a week.

In DU145 cells in which human LIV1 was silenced, morphological observation of each of the cells cultured for about a week confirmed morphological changes in mesenchymal-like-cells with numerous projections (Fig. 6).

The expression level of LIV 1 and E-cadherin in the cultured cells was confirmed by RT-PCR. The results showed that the expression of both LIV1 and E-cadherin was suppressed in DU145 cells in which human LIV1 was silenced by RNAi (Fig. 6).

Furthermore, forced expression of zebrafish LIV1 in DU145 cells was found to cause similar morphological changes in mesenchymal-like-cells.

### Industrial Applicability

The present invention provides LIV1, which is a regulatory agent of Snail activity and an EMT-inducing agent. The present inventors demonstrated for the first time that LIV1, a downstream target of STAT3, plays a key role in the EMT of gastrula organizer cells by affecting Snail activity. Thus, the regulatory agents of Snail activity and EMT-inducing agents of the present invention may not only contribute to the advancement of developmental studies as substances which regulate development, but may also be applied in regenerative medicine as agents for promoting the regeneration of organs for transplantation. The present invention also provides agents for suppressing Snail activity and EMT-suppressing agents. Since EMT is deeply involved in cancer metastasis and progression, agents for suppressing Snail activity and EMT-suppressing agents, both of which suppress EMT, are expected to block cancer metastasis and progression, and may be applied as novel pharmaceuticals for treating cancer. The EMT-suppressing agents of the present invention are useful for suppressing estrogen receptor-negative (ER(-)) breast cancer and its metastasis.

## Claims

1. A regulatory agent of Snail activity, which is an isolated DNA of any one of the following (a) to (d):
(a) a DNA encoding a protein comprising the amino acid sequence of SEQ ID NO: 1, 2, 26, 28, 30, 32, 34, 36, 38, 40, or 42;
(b) a DNA comprising the sequence of SEQ ID NO: 3, 4, 25, 27, 29, 31, 33, 35, 37, 39, or 41;
(c) a DNA encoding a protein comprising an amino acid sequence with one or more amino acid substitutions, deletions, insertions, and/or additions in the amino acid sequence of SEQ ID NO: 1, 2, 26, 28, 30, 32, 34, 36, 38, 40, or 42; and
(d) a DNA hybridizing under stringent conditions with the sequence of SEQ ID NO: 3, 4, 25, 27, 29, 31, 33, 35, 37, 39, or 41.

2. A regulatory agent of Snail activity, which is a vector into which a DNA of any one of the following (a) to (d) is inserted:
(a) a DNA encoding a protein comprising the amino acid sequence of SEQ ID NO: 1, 2, 26, 28, 30, 32, 34, 36, 38, 40, or 42;
(b) a DNA comprising the sequence of SEQ ID NO: 3, 4, 25, 27, 29, 31, 33, 35, 37, 39, or 41;
(c) a DNA encoding a protein comprising an amino acid sequence with one or more amino acid substitutions, deletions, insertions, and/or additions in the amino acid sequence of SEQ ID NO: 1, 2, 26, 28, 30, 32, 34, 36, 38, 40, or 42; and
(d) a DNA hybridizing under stringent conditions with the sequence of SEQ ID NO: 3, 4, 25, 27, 29, 31, 33, 35, 37, 39, or 41.

3. A regulatory agent of Snail activity, which is an isolated protein encoded by a DNA of any one of the following (a) to (d):
(a) a DNA encoding a protein comprising the amino acid sequence of SEQ ID NO: 1, 2, 26, 28, 30, 32, 34, 36, 38, 40, or 42;
(b) a DNA comprising the sequence of SEQ ID NO: 3, 4, 25, 27, 29, 31, 33, 35, 37, 39, or 41;
(c) a DNA encoding a protein comprising an amino acid sequence with one or more amino acid substitutions, deletions, insertions, and/or additions in the amino acid sequence of SEQ ID NO: 1, 2, 26, 28, 30, 32, 34, 36, 38, 40, or 42; and
(d) a DNA hybridizing under stringent conditions with the sequence of SEQ ID NO: 3, 4, 25, 27, 29,31, 33, 35, 37, 39, or 41.

4. An agent for suppressing Snail activity, which is an antisense oligonucleotide targeting a DNA sequence comprising the sequence of SEQ ID NO: 3, 4, 25, 27, 29, 31, 33, 35, 37, 39, or 41.

5. An agent for suppressing Snail activity, which is a double-stranded RNA comprising a sequence identical or similar to a portion of a DNA comprising the sequence of SEQ ID NO: 3, 4, 25, 27, 29, 31, 33, 35, 37, 39, or 41.

6. A pharmaceutical for treating cancer, which comprises a nucleotide or vector of any one of the following (a) to (c), as an active ingredient:
(a) an antisense oligonucleotide of a DNA comprising the sequence of SEQ ID NO: 3;
(b) a vector into which an antisense oligonucleotide of a DNA comprising the sequence of SEQ ID NO: 3 is inserted; and
(c) an oligonucleotide that is a double-stranded RNA comprising a sequence identical or similar to a portion of a DNA comprising the sequence of SEQ ID NO: 3.

7. A pharmaceutical for treating cancer, which comprises the agent for suppressing Snail activity of claim 4 or 5 as an active ingredient.

8. An EMT-inducing agent, which is an isolated DNA of any one of the following (a) to (d):
(a) a DNA encoding a protein comprising the amino acid sequence of SEQ ID NO: 1, 2, 26, 28, 30, 32, 34, 36, 38, 40, or 42;
(b) a DNA comprising the sequence of SEQ ID NO: 3, 4, 25, 27, 29, 31, 33, 35, 37, 39, or 41;
(c) a DNA encoding a protein comprising an amino acid sequence with one or more amino acid substitutions, deletions, insertions, and/or additions in the amino acid sequence of SEQ ID NO: 1, 2, 26, 28, 30, 32, 34, 36, 38, 40, or 42; and
(d) a DNA hybridizing under stringent conditions with the sequence of SEQ ID NO: 3, 4, 25, 27, 29, 31, 33, 35, 37, 39, or 41.

9. An EMT-inducing agent, which is a vector into which a DNA of any one of the following (a) to (d) is inserted:
(a) a DNA encoding a protein comprising the amino acid sequence of SEQ ID NO: 1, 2, 26, 28, 30, 32, 34, 36, 38, 40, or 42;
(b) a DNA comprising the sequence of SEQ ID NO: 3, 4, 25, 27, 29, 31, 33, 35, 37, 39, or 41;
(c) a DNA encoding a protein comprising an amino acid sequence with one or more amino acid substitutions, deletions, insertions, and/or additions in the amino acid sequence of SEQ ID NO: 1, 2, 26, 28, 30, 32, 34, 36, 38, 40, or 42; and
(d) a DNA hybridizing under stringent conditions with the sequence of SEQ ID NO: 3, 4, 25, 27, 29, 31, 33, 35, 37, 39, or 41.

10. An EMT-inducing agent, which is an isolated protein encoded by a DNA of any one of the following (a) to (d):
(a) a DNA encoding a protein comprising the amino acid sequence of SEQ ID NO: 1, 2, 26, 28, 30, 32, 34, 36, 38, 40, or 42;
(b) a DNA comprising the sequence of SEQ ID NO: 3, 4, 25, 27, 29, 31, 33, 35, 37, 39, or 41;
(c) a DNA encoding a protein comprising an amino acid sequence with one or more amino acid substitutions, deletions, insertions, and/or additions in the amino acid sequence of SEQ ID NO: 1, 2, 26, 28, 30, 32, 34, 36, 38, 40, or 42; and
(d) a DNA hybridizing under stringent conditions with the sequence of SEQ ID NO: 3, 4, 25, 27, 29, 31, 33, 35, 37, 39, or 41.

11. An EMT-suppressing agent, which is an antisense oligonucleotide targeting a DNA sequence comprising the sequence of SEQ ID NO: 3, 4, 25, 27, 29, 31, 33, 35, 37, 39, or 41.

12. An EMT-suppressing agent, which is a double-stranded RNA comprising a sequence identical or similar to a portion of a DNA comprising the sequence of SEQ ID NO: 3, 4, 25, 27, 29, 31, 33, 35, 37, 39, or 41.

13. A pharmaceutical for treating cancer, which comprises the EMT-suppressing agent of claim 11 or 12 as an active ingredient.

14. A method of screening for a candidate substance for an agent for suppressing Snail activity, wherein the method comprises the following steps of (a) to (c):
(a) injecting a vector which comprises a reporter gene operably linked under the control of the E-cadherin promoter, and a test substance into a one-cell-stage embryo;
(b) measuring the expression level of the reporter gene; and
(c) selecting a compound that reduces or increases the measured expression level of the reporter gene, compared with the measured expression level in the absence of the test substance.

15. A wound healing agent, which comprises the regulatory agent of Snail activity of any one of claims 1 to 3, or the EMT-inducing agent of any one of claims 8 to 10, as an active ingredient.

16. An anti-inflammatory agent, which comprises the agent for suppressing Snail activity of claim 4 or 5, or the EMT-suppressing agent of claim 11 or 12, as an active ingredient.

17. A regulatory agent of a protein activity that requires Zn, which comprises a protein comprising the amino acid sequence of SEQ ID NO: 1, 2, 26, 28, 30, 32, 34, 36, 38, 40, or 42 as an active ingredient.


SEQUENCE LISTING

<110>  HIRANO, Toshio

<120>  EMT INDUCER

<130>  C1-A0325P

<150>  JP 2003-435122
<151>  2003-12-26

<160>  43

<170>  PatentIn version 3.3

<210>  1
<211>  742
<212>  PRT
<213>  Danio rerio

<400>  1

Met Met Thr Phe Leu Cys Thr Arg Ser Gly Arg Arg Ala Ser Gly Val
1               5                   10                  15

Glu Cys Arg Ile Ala Ala Glu Arg Ala Tyr Phe Arg Val Arg Gly Leu
            20                  25                  30

Pro Val Ala Asn Met Ile Gly Trp Trp Pro Arg Leu Cys Pro Val Met
        35                  40                  45

Ser Leu Ala Leu Leu Trp Ala Cys Ser Val Gly Ala Gly Ser Asp Cys
    50                  55                  60

Lys Ser Val Ala Ile Glu Thr Asp Ser Arg Ile Ala Glu Gln Thr Gln
65                  70                  75                  80

Gln Arg His Leu Gln Ala Leu Phe Asp Lys Tyr Gly Gln Asn Gly Ser
                85                  90                  95

Ile Ser Leu Glu Gly Leu Phe Asn Leu Leu Lys Gly Val Gly Leu Asp
            100                 105                 110

Arg Ile Arg Lys Val Met Val His His Pro Gly Asn Ala His Asn His
        115                 120                 125

Thr His Thr His Asp His Thr His Thr His Val Asp Lys Leu Thr Ala
    130                 135                 140

His Thr His Pro Val Thr Thr Lys Lys Gly Asp Met Asp His Ser Val
145                 150                 155                 160

Glu Lys Ser Asp Pro Val Pro Lys Ala Gln Pro Asp Pro Ala Ser Gly
                165                 170                 175

Lys Lys Ser Gln Ser Asp Ala His His Asn Leu Tyr Met Lys Met Asn
            180                 185                 190

Gln Glu Ser Thr Thr Ala Leu Thr Thr Pro Ser Tyr Val Thr Arg Ser
        195                 200                 205

Arg Arg Thr Asn Arg Ser Ala Asp Tyr Asp Phe Thr Gln Asp His Ala
    210                 215                 220

Ser Phe Ser Pro Ser Gln Pro Asn Val Thr His Ser Asn His Thr His
225                 230                 235                 240

His Asp Glu Asp Thr Pro Thr His Gln His Asp Asp His Asp Glu His
                245                 250                 255

Glu His Ala Arg Ala Ser Leu Gly Cys Gln Asn Ala Ser Thr Ile Leu
            260                 265                 270

Gln Thr His Gly Met Arg Lys Glu Ala Ser Leu Ser Val Lys Asp Phe
        275                 280                 285

Ser Phe Leu Cys Pro Ala Leu Leu Met Gln Ile Asp Ser Lys Ser Cys
    290                 295                 300

Ile Val His Glu Asp Glu Asp Glu His Ser Asp His Ser His His His
305                 310                 315                 320

Lys His His His His His His Asp His Gln His Leu Gln His Pro His
                325                 330                 335

Asn His Thr Asn Gly Arg Gly Gln Arg Asn Thr Pro Val Tyr Ile Ala
            340                 345                 350

Trp Leu Gly Gly Phe Leu Ser Ile Thr Leu Ile Ser Leu Leu Ala Leu
        355                 360                 365

Val Gly Val Val Leu Ile Pro Leu Met Asn Arg Val Cys Phe Asn Phe
    370                 375                 380

Leu Leu Ser Phe Leu Val Ala Leu Ala Val Gly Thr Leu Ser Gly Asp
385                 390                 395                 400

Ala Leu Leu His Leu Ile Pro His Ser Gln Gly His His His His Gly
                405                 410                 415

His Ser Glu Glu His Ala Glu Glu Glu Asp Ser Leu Arg Pro Val Trp
            420                 425                 430

Thr Gly Leu Thr Ala Leu Ser Gly Val Tyr Ile Met Phe Leu Ile Glu
        435                 440                 445

His Phe Leu Thr Leu Gly Lys Met Tyr Lys Asp Lys Asn Gln Lys Val
    450                 455                 460

Gln Lys Arg Val Asp Leu Thr Thr Glu Val Leu Glu Ser Glu Lys Leu
465                 470                 475                 480

Pro Ser Leu Glu Glu Asn Asp Val Lys Ile Glu Ala Ala Glu Thr Asn
                485                 490                 495

Gly Gly Arg Ala Leu Ala Glu Glu Glu Glu Val Met Leu Gly Ala Glu
            500                 505                 510

Leu Tyr Asn Asp Ile Asp Cys Glu Asn Lys Cys His Ser His Phe His
        515                 520                 525

Asp Thr Val Gly Gln Ser Asp Glu Gln His His His His His Asp Tyr
    530                 535                 540

His His Ile Leu His His His His Ser Gln Asn His His Pro His Thr
545                 550                 555                 560

His Thr His Arg His Thr His Ser Tyr Ser Gln Gln His Phe Glu Gln
                565                 570                 575

Ala Gly Val Ala Thr Leu Ala Trp Met Val Ile Met Gly Asp Gly Leu
            580                 585                 590

His Asn Phe Ser Asp Gly Leu Ala Ile Gly Ala Ala Phe Thr Glu Gly
        595                 600                 605

Leu Ser Ser Gly Leu Ser Thr Ser Val Ala Val Phe Cys His Glu Leu
    610                 615                 620

Pro His Glu Leu Gly Asp Phe Ala Val Leu Leu Lys Ala Gly Met Ser
625                 630                 635                 640

Val Arg Gln Ala Met Leu Tyr Asn Leu Leu Ser Ala Leu Met Gly Tyr
                645                 650                 655

Leu Gly Met Ile Ile Gly Ile Leu Ile Gly His Tyr Ala Glu Asn Val
            660                 665                 670

Ala Thr Trp Ile Phe Ala Leu Thr Ala Gly Leu Phe Met Tyr Val Ala
        675                 680                 685

Leu Val Asp Met Val Pro Glu Met Leu His Asn Asp Ala Ser Glu Ala
    690                 695                 700

Gly Phe Ser His Tyr Gly Phe Phe Leu Leu Gln Asn Ala Gly Ile Leu
705                 710                 715                 720

Leu Gly Phe Gly Ile Met Leu Ile Ile Ala Val Phe Glu Asp Arg Ile
                725                 730                 735

Gln Leu Asp Leu Gly Tyr

            740

<210> 2
<211> 749
<212> PRT
<213> Homo sapiens

<400> 2

Met Ala Arg Lys Leu Ser Val Ile Leu Ile Leu Thr Phe Ala Leu Ser
1               5                   10                  15

Val Thr Asn Pro Leu His Glu Leu Lys Ala Ala Ala Phe Pro Gln Thr
            20                  25                  30

Thr Glu Lys Ile Ser Pro Asn Trp Glu Ser Gly Ile Asn Val Asp Leu
        35                  40                  45

Ala Ile Ser Thr Arg Gln Tyr His Leu Gln Gln Leu Phe Tyr Arg Tyr
    50                  55                  60

Gly Glu Asn Asn Ser Leu Ser Val Glu Gly Phe Arg Lys Leu Leu Gln
65                  70                  75                  80

Asn Ile Gly Ile Asp Lys Ile Lys Arg Ile His Ile His His Asp His
                85                  90                  95

Asp His His Ser Asp His Glu His His Ser Asp His Glu Arg His Ser
            100                 105                 110

Asp His Glu His His Ser Asp His Glu His His Ser Asp His Asn His
        115                 120                 125

Ala Ala Ser Gly Lys Asn Lys Arg Lys Ala Leu Cys Pro Asp His Asp
    130                 135                 140

Ser Asp Ser Ser Gly Lys Asp Pro Arg Asn Ser Gln Gly Lys Gly Ala
145                 150                 155                 160

His Arg Pro Glu His Ala Ser Gly Arg Arg Asn Val Lys Asp Ser Val
                165                 170                 175

Ser Ala Ser Glu Val Thr Ser Thr Val Tyr Asn Thr Val Ser Glu Gly
            180                 185                 190

Thr His Phe Leu Glu Thr Ile Glu Thr Pro Arg Pro Gly Lys Leu Phe
        195                 200                 205

Pro Lys Asp Val Ser Ser Ser Thr Pro Pro Ser Val Thr Ser Lys Ser
    210                 215                 220

Arg Val Ser Arg Leu Ala Gly Arg Lys Thr Asn Glu Ser Val Ser Glu
225                 230                 235                 240

Pro Arg Lys Gly Phe Met Tyr Ser Arg Asn Thr Asn Glu Asn Pro Gln
                245                 250                 255

Glu Cys Phe Asn Ala Ser Lys Leu Leu Thr Ser His Gly Met Gly Ile
            260                 265                 270

Gln Val Pro Leu Asn Ala Thr Glu Phe Asn Tyr Leu Cys Pro Ala Ile
        275                 280                 285

Ile Asn Gln Ile Asp Ala Arg Ser Cys Leu Ile His Thr Ser Glu Lys
    290                 295                 300

Lys Ala Glu Ile Pro Pro Lys Thr Tyr Ser Leu Gln Ile Ala Trp Val
305                 310                 315                 320

Gly Gly Phe Ile Ala Ile Ser Ile Ile Ser Phe Leu Ser Leu Leu Gly
                325                 330                 335

Val Ile Leu Val Pro Leu Met Asn Arg Val Phe Phe Lys Phe Leu Leu
            340                 345                 350

Ser Phe Leu Val Ala Leu Ala Val Gly Thr Leu Ser Gly Asp Ala Phe
        355                 360                 365

Leu His Leu Leu Pro His Ser His Ala Ser His His His Ser His Ser
    370                 375                 380

His Glu Glu Pro Ala Met Glu Met Lys Arg Gly Pro Leu Phe Ser His
385                 390                 395                 400

Leu Ser Ser Gln Asn Ile Glu Glu Ser Ala Tyr Phe Asp Ser Thr Trp
                405                 410                 415

Lys Gly Leu Thr Ala Leu Gly Gly Leu Tyr Phe Met Phe Leu Val Glu
            420                 425                 430

His Val Leu Thr Leu Ile Lys Gln Phe Lys Asp Lys Lys Lys Lys Asn
        435                 440                 445

Gln Lys Lys Pro Glu Asn Asp Asp Asp Val Glu Ile Lys Lys Gln Leu
    450                 455                 460

Ser Lys Tyr Glu Ser Gln Leu Ser Thr Asn Glu Glu Lys Val Asp Thr
465                 470                 475                 480

Asp Asp Arg Thr Glu Gly Tyr Leu Arg Ala Asp Ser Gln Glu Pro Ser
                485                 490                 495

His Phe Asp Ser Gln Gln Pro Ala Val Leu Glu Glu Glu Glu Val Met
            500                 505                 510

Ile Ala His Ala His Pro Gln Glu Val Tyr Asn Glu Tyr Val Pro Arg
        515                 520                 525

Gly Cys Lys Asn Lys Cys His Ser His Phe His Asp Thr Leu Gly Gln
    530                 535                 540

Ser Asp Asp Leu Ile His His His His Asp Tyr His His Ile Leu His
545                 550                 555                 560

His His His His Gln Asn His His Pro His Ser His Ser Gln Arg Tyr
                565                 570                 575

Ser Arg Glu Glu Leu Lys Asp Ala Gly Val Ala Thr Leu Ala Trp Met
            580                 585                 590

Val Ile Met Gly Asp Gly Leu His Asn Phe Ser Asp Gly Leu Ala Ile
        595                 600                 605

Gly Ala Ala Phe Thr Glu Gly Leu Ser Ser Gly Leu Ser Thr Ser Val
    610                 615                 620

Ala Val Phe Cys His Glu Leu Pro His Glu Leu Gly Asp Phe Ala Val
625                 630                 635                 640

Leu Leu Lys Ala Gly Met Thr Val Lys Gln Ala Val Leu Tyr Asn Ala
                645                 650                 655

Leu Ser Ala Met Leu Ala Tyr Leu Gly Met Ala Thr Gly Ile Phe Ile
            660                 665                 670

Gly His Tyr Ala Glu Asn Val Ser Met Trp Ile Phe Ala Leu Thr Ala
        675                 680                 685

Gly Leu Phe Met Tyr Val Ala Leu Val Asp Met Val Pro Glu Met Leu
    690                 695                 700

His Asn Asp Ala Ser Asp His Gly Cys Ser Arg Trp Gly Tyr Phe Phe
705                 710                 715                 720

Leu Gln Asn Ala Gly Met Leu Leu Gly Phe Gly Ile Met Leu Leu Ile
                725                 730                 735

Ser Ile Phe Glu His Lys Ile Val Phe Arg Ile Asn Phe
            740                 745

<210>  3
<211>  2229
<212>  DNA
<213>  Danio rerio

<400>  3
atgatgacgt ttctttgcac acggtctggt cgccgtgcta gtggtgtgga gtgcagaatc      60

gccgctgaac gcgcttactt tcgagtgcgt ggactcccgg ttgccaatat gattggctgg     120

tggccacgcc tctgcccagt gatgtcactg gcactgctgt gggcgtgttc agtgggggcg 180
ggttcagact gcaaatctgt ggccattgag actgacagcc gcatagcaga acaaacacag 240
cagcgtcacc tacaggctct gttcgacaag tatggccaga acggcagcat ctccctagaa 300
ggcctcttca acctacttaa aggggtcggg cttgaccgca tccggaaagt gatggtgcat 360
catcctggaa atgcccataa tcacacacac acgcatgatc acacacacac tcatgtggac 420
aaactcacgg cgcacacaca tccggtcacc accaagaagg gagacatgga tcacagcgtg 480
gagaagagtg accctgtccc aaaagcacag ccagatcctg cctctgggaa gaaaagccag 540
tcagatgcgc atcacaacct gtacatgaag atgaaccagg aatccaccac agctttgact 600
acgccgtcat atgttaccag atcacggcgg accaatcgca gcgccgatta tgattttaca 660
caggaccacg cctcctttag ccccagtcag cccaatgtga cacactcaaa ccacacccat 720
catgatgagg acacgcccac acaccagcat gatgaccatg atgagcacga acatgcccgt 780
gctagtttag ggtgtcaaaa tgcctccacg atcctgcaga cgcatggcat gagaaaggaa 840
gcaagtctct cagttaagga cttcagtttc ctctgccctg ctcttctcat gcagattgat 900
tccaagtctt gcatcgtgca tgaagacgag gacgagcatt cagatcattc ccatcatcac 960
aaacaccacc accatcatca tgatcaccaa cacctgcagc atccacataa ccacaccaat 1020
ggaagaggcc agaggaacac tccagtctac atcgcatggc ttggagggtt tctctccatc 1080
actctgatca gtttgctggc gttggttggt gtggttttga tcccactcat gaacagagtt 1140
tgcttcaact tcctgctgag cttcctggtg gcccttgcag tgggcactct gagcggagac 1200
gctctcctcc acctcatacc acattctcag ggtcatcacc atcacggcca ctctgaagag 1260
cacgctgaag aggaggactc ccttcgccct gtgtggaccg gactcacagc tctaagtgga 1320
gtttacatca tgttcctcat cgaacacttc ctgacccttg gcaaaatgta caaagacaaa 1380
aaccagaagg tgcagaagag ggttgatctc accacagaag ttttggagtc tgagaaactg 1440
ccatcattag aagaaaatga tgtcaaaatt gaagctgctg aaacgaatgg tgggcgtgca 1500
ctggcagagg aggaggaggt gatgttgggg gcggagctct acaacgacat agactgcgag 1560
aacaaatgcc actcccactt ccatgacacg gtcggccaat cggatgagca gcatcatcat 1620
catcacgact accaccacat actgcatcat caccactccc agaaccacca cccgcacaca 1680
cacacgcaca gacacacaca ctcctactcg cagcagcact ttgagcaggc tggtgtggcc 1740
acactcgcct ggatggtcat catgggagac ggactgcaca acttcagtga tggacttgcc 1800
ataggggcgg ctttcacaga aggtttgtcc agtggtctta gtacctcagt cgctgtgttc 1860
tgccatgagc ttcctcatga actcggtgat tttgccgtcc tactgaaagc cggtatgtca 1920
gttcgacagg ccatgctgta taatctgctg tcagcactga tgggatatct gggcatgatc 1980
atcgggattc tcatcggaca ttatgctgaa aatgttgcca catggatctt tgctctcaca 2040
gctgggttat tcatgtacgt cgcgctcgtg gacatggtac ctgagatgct gcacaatgac 2100
gcgagcgaag caggtttcag tcactacggc ttcttcctcc tgcagaacgc tgggatactc 2160

ctaggcttcg gcatcatgct tatcattgct gtctttgagg acaggatcca actggactta 2220

ggttactga 2229

<210> 4
<211> 2744
<212> DNA
<213> Homo sapiens

<400> 4
ctcgtgccga attcggcacg agaccgcgtg ttcgcgcctg gtagagattt ctcgaagaca 60

ccagtgggcc cgtgtggaac caaacctgcg cgcgtggccg ggccgtggga caacgaggcc 120

gcggagacga aggcgcaatg gcgaggaagt tatctgtaat cttgatcctg acctttgccc 180

tctctgtcac aaatcccctt catgaactaa aagcagctgc tttcccccag accactgaga 240

aaattagtcc gaattgggaa tctggcatta atgttgactt ggcaatttcc acacggcaat 300

atcatctaca acagcttttc taccgctatg gagaaaataa ttctttgtca gttgaagggt 360

tcagaaaatt acttcaaaat ataggcatag ataagattaa aagaatccat atacaccatg 420

accacgacca tcactcagac cacgagcatc actcagacca tgagcgtcac tcagaccatg 480

agcatcactc agaccacgag catcactctg accataatca tgctgcttct ggtaaaaata 540

agcgaaaagc tctttgccca gaccatgact cagatagttc aggtaaagat cctagaaaca 600

gccaggggaa aggagctcac cgaccagaac atgccagtgg tagaaggaat gtcaaggaca 660

gtgttagtgc tagtgaagtg acctcaactg tgtacaacac tgtctctgaa ggaactcact 720

ttctagagac aatagagact ccaagacctg gaaaactctt ccccaaagat gtaagcagct 780

ccactccacc cagtgtcaca tcaaagagcc gggtgagccg gctggctggt aggaaaacaa 840

atgaatctgt gagtgagccc cgaaaaggct ttatgtattc cagaaacaca aatgaaaatc 900

ctcaggagtg tttcaatgca tcaaagctac tgacatctca tggcatgggc atccaggttc 960

cgctgaatgc aacagagttc aactatctct gtccagccat catcaaccaa attgatgcta 1020

gatcttgtct gattcataca agtgaaaaga aggctgaaat ccctccaaag acctattcat 1080

tacaaatagc ctgggttggt ggttttatag ccatttccat catcagtttc ctgtctctgc 1140

tgggggttat cttagtgcct ctcatgaatc gggtgttttt caaatttctc ctgagtttcc 1200

ttgtggcact ggccgttggg actttgagtg gtgatgcttt tttacacctt cttccacatt 1260

ctcatgcaag tcaccaccat agtcatagcc atgaagaacc agcaatggaa atgaaaagag 1320

gaccactttt cagtcatctg tcttctcaaa acatagaaga aagtgcctat tttgattcca 1380

cgtggaaggg tctaacagct ctaggaggcc tgtatttcat gtttcttgtt gaacatgtcc 1440

tcacattgat caaacaattt aaagataaga agaaaaagaa tcagaagaaa cctgaaaatg 1500

atgatgatgt ggagattaag aagcagttgt ccaagtatga atctcaactt tcaacaaatg 1560

aggagaaagt agatacagat gatcgaactg aaggctattt acgagcagac tcacaagagc 1620

cctcccactt tgattctcag cagcctgcag tcttggaaga agaagaggtc atgatagctc 1680

atgctcatcc acaggaagtc tacaatgaat atgtacccag agggtgcaag aataaatgcc 1740

attcacattt ccacgataca ctcggccagt cagacgatct cattcaccac catcatgact 1800
accatcatat tctccatcat caccaccacc aaaaccacca tcctcacagt cacagccagc 1860
gctactctcg ggaggagctg aaagatgccg gcgtcgccac tttggcctgg atggtgataa 1920
tgggtgatgg cctgcacaat ttcagcgatg gcctagcaat tggtgctgct tttactgaag 1980
gcttatcaag tggtttaagt acttctgttg ctgtgttctg tcatgagttg cctcatgaat 2040
taggtgactt tgctgttcta ctaaaggctg gcatgaccgt taagcaggct gtcctttata 2100
atgcattgtc agccatgctg gcgtatcttg gaatggcaac aggaattttc attggtcatt 2160
atgctgaaaa tgtttctatg tggatatttg cacttactgc tggcttattc atgtatgttg 2220
ctctggttga tatggtacct gaaatgctgc acaatgatgc tagtgaccat ggatgtagcc 2280
gctgggggta tttcttttta cagaatgctg ggatgctttt gggttttgga attatgttac 2340
ttatttccat atttgaacat aaaatcgtgt ttcgtataaa tttctagtta aggtttaaat 2400
gctagagtag cttaaaaagt tgtcatagtt tcagtaggtc atagggagat gagtttgtat 2460
gctgtactat gcagcgttta aagttagtgg gttttgtgat ttttgtattg aatattgctg 2520
tctgttacaa agtcagttaa aggtacgttt taatatttaa gttattctat cttggagata 2580
aaatctgtat gtgcaattca ccggtattac cagtttatta tgtaaacaag agatttggca 2640
tgacatgttc tgtatgtttc agggaaaaat gtctttaatg ctttttcaag aactaacaca 2700
gttattccta tactggattt taggtctctg aagaactgct ggtg 2744

<210> 5
<211> 25
<212> DNA
<213> Artificial

<220>
<223> antisense sequence

<400> 5
cggaaacagc gcgagtgtct tttgt 25

<210> 6
<211> 25
<212> DNA
<213> Artificial

<220>
<223> antisense sequence

<400> 6
accgtgtgca aagaaacgtc atcat 25

<210> 7
<211> 25
<212> DNA
<213> Artificial

<220>
<223> antisense sequence

<400> 7
cgcaaaaagc gagagtgtat tatgt 25

<210> 8
<211> 25
<212> DNA
<213> Artificial

<220>
<223> antisense sequence

<400> 8
acggtctgca aacaaacgtg atgat 25


<210> 9
<211> 25
<212> DNA
<213> Artificial

<220>
<223> antisense sequence

<400> 9
ctcaaggttt cagataaatc gtcct 25


<210> 10
<211> 25
<212> DNA
<213> Artificial

<220>
<223> antisense sequence

<400> 10
gccatgttga cccccttaatg tgtcg 25


<210> 11
<211> 25
<212> DNA
<213> Artificial

<220>
<223> antisense sequence

<400> 11
ctctaggatt cagataaaac gtgct 25


<210> 12
<211> 25
<212> DNA
<213> Artificial

<220>
<223> antisense sequence

<400> 12
gccttgtaga ccccttaaag tgacg 25


<210> 13
<211> 25
<212> DNA
<213> Artificial

<220>
<223> antisense sequence

<400>  13
gtccactcca gttactttca gggat                                              25


<210>  14
<211>  23
<212>  DNA
<213>  Artificial

<220>
<223>  antisense sequence

<400>  14
catgctgaac tctgaagttg atc                                                23


<210>  15
<211>  58
<212>  PRT
<213>  Danio rerio

<400>  15

Ala Trp Met Val Ile Met Gly Asp Gly Leu His Asn Phe Ser Asp Gly
1               5                   10                  15


Leu Ala Ile Gly Ala Ala Phe Thr Glu Gly Leu Ser Ser Gly Leu Ser
            20                  25                  30


Thr Ser Val Ala Val Phe Cys His Glu Leu Pro His Glu Leu Gly Asp
        35                  40                  45


Phe Ala Val Leu Leu Lys Ala Gly Met Ser
    50                  55


<210>  16
<211>  58
<212>  PRT
<213>  Homo sapiens

<400>  16

Ala Trp Met Val Ile Met Gly Asp Gly Leu His Asn Phe Ser Asp Gly
1               5                   10                  15


Leu Ala Ile Gly Ala Ala Phe Thr Glu Gly Leu Ser Ser Gly Leu Ser
            20                  25                  30


Thr Ser Val Ala Val Phe Cys His Glu Leu Pro His Glu Leu Gly Asp
        35                  40                  45


Phe Ala Val Leu Leu Lys Ala Gly Met Thr
    50                  55


<210>  17
<211>  58
<212>  PRT
<213>  Homo sapiens

<400>  17

Ala Trp Met Val Ile Met Gly Asp Gly Ile His Asn Phe Ser Asp Gly
1               5                   10                  15

Leu Ala Ile Gly Ala Ala Phe Ser Ala Gly Leu Thr Gly Gly Ile Ser
            20                  25                  30

Thr Ser Ile Ala Val Phe Cys His Glu Leu Pro His Glu Leu Gly Asp
        35                  40                  45

Phe Ala Val Leu Leu Lys Ala Gly Met Thr
    50                  55

<210>  18
<211>  58
<212>  PRT
<213>  Homo sapiens

<400>  18

Thr Trp Met Val Leu Leu Gly Asp Gly Leu His Asn Leu Thr Asp Gly
1               5                   10                  15

Leu Ala Ile Gly Ala Ala Phe Ser Asp Gly Phe Ser Ala Gly Leu Ser
            20                  25                  30

Thr Thr Leu Ala Val Phe Cys His Glu Leu Pro His Glu Leu Gly Asp
        35                  40                  45

Phe Ala Met Leu Leu Gln Ser Gly Leu Ser
    50                  55

<210>  19
<211>  58
<212>  PRT
<213>  Homo sapiens

<400>  19

Pro Tyr Met Ile Thr Leu Gly Asp Ala Val His Asn Phe Ala Asp Gly
1               5                   10                  15

Leu Ala Val Gly Ala Ala Phe Ala Ser Ser Trp Lys Thr Gly Leu Ala
            20                  25                  30

Thr Ser Leu Ala Val Phe Cys His Glu Leu Pro His Glu Leu Gly Asp
        35                  40                  45

Phe Ala Ala Leu Leu His Ala Gly Leu Ser
    50                  55

<210>  20
<211>  58
<212>  PRT
<213>  Homo sapiens

<400>  20

Ala Ile Met Ile Leu Val Gly Asp Ser Leu His Asn Phe Ala Asp Gly
1               5                   10                  15

Leu Ala Ile Gly Ala Ala Phe Ser Ser Ser Ser Glu Ser Gly Val Thr
            20                  25                  30

Thr Thr Ile Ala Ile Leu Cys His Glu Ile Pro His Glu Met Gly Asp
        35                  40                  45

Phe Ala Val Leu Leu Ser Ser Gly Leu Ser
    50                  55

<210>  21
<211>  58
<212>  PRT
<213>  Homo sapiens

<400>  21

Ala Trp Met Ile Thr Leu Cys Asp Ala Leu His Asn Phe Ile Asp Gly
1               5                   10                  15

Leu Ala Ile Gly Ala Ser Cys Thr Leu Ser Leu Leu Gln Gly Leu Ser
            20                  25                  30

Thr Ser Ile Ala Ile Leu Cys Glu Glu Phe Pro His Glu Leu Gly Asp
        35                  40                  45

Phe Val Ile Leu Leu Asn Ala Gly Met Ser
    50                  55

<210>  22
<211>  58
<212>  PRT
<213>  Homo sapiens

<400>  22

Ala Trp Met Ile Thr Leu Ser Asp Gly Leu His Asn Phe Ile Asp Gly
1               5                   10                  15

Leu Ala Ile Gly Ala Ser Phe Thr Val Ser Val Phe Gln Gly Ile Ser
            20                  25                  30

Thr Ser Val Ala Ile Leu Cys Glu Glu Phe Pro His Glu Leu Gly Asp
        35                  40                  45

Phe Val Ile Leu Leu Asn Ala Gly Met Ser
    50                  55

<210>  23
<211>  58
<212>  PRT
<213>  Homo sapiens

<400>  23

Gly Tyr Leu Asn Leu Leu Ala Asn Thr Ile Asp Asn Phe Thr His Gly
1               5                   10                  15

Leu Ala Val Ala Ala Ser Phe Leu Val Ser Lys Lys Ile Gly Leu Leu
            20                  25                  30

Thr Thr Met Ala Ile Leu Leu His Glu Ile Pro His Glu Val Gly Asp
        35                  40                  45

Phe Ala Ile Leu Val Gln Ser Gly Cys Ser
    50                  55

<210>  24
<211>  58
<212>  PRT
<213>  Homo sapiens

<400>  24

Gly Tyr Leu Asn Leu Ala Ala Asp Leu Ala His Asn Phe Thr Asp Gly
1               5                   10                  15

Leu Ala Ile Gly Ala Ser Phe Arg Gly Gly Arg Gly Leu Gly Ile Leu
            20                  25                  30

Thr Thr Met Thr Val Leu Leu His Glu Val Pro His Glu Val Gly Asp
        35                  40                  45

Phe Ala Ile Leu Leu Arg Ala Gly Phe Asp
    50                  55

<210>  25
<211>  2110
<212>  DNA
<213>  Homo sapiens

<220>
<221>  CDS
<222>  (56)..(1465)

<400>  25
gcgggactgc cacgtccaag caaaccggga aaggagagga tcccggagcc gcgtg atg      58
gcc aga ggc ctg ggg gcc ccc cac tgg gtg gcc gtg gga ctg ctg acc      106
tgg gcg acc ttg ggg ctt ctg gtg gct gga ctc ggg ggt cat gac gac      154
ctg cac gac gat ctg caa gag gac ttc cat ggc cac agc cac agg cac      202
tca cat gaa gat ttc cac cat ggc cac agc cat gcc cat ggc cat ggc      250
cac act cac gag agc atc tgg cat gga cat acc cac gat cac gac cat      298
gga cat tca cat gag gat tta cac cat ggc cat agc cat ggc tac tcc      346
cat gag agc ctc tac cac aga gga cat gga cat gac cat gag cat agc      394
cat gga ggc tat ggg gag tct ggg gct cca ggc atc aag cag gac ctg      442
gat gct gtc act ctc tgg gct tat gca ctg ggg gcc aca gtg ctg atc      490
tca gca gct cca ttt ttt gtc ctc ttc ctt atc ccc gtg gag tcg aac      538
tct ccc cgg cat cgc tct cta ctt cag atc ttg ctc agt ttt gct tcc      586
ggt ggg ctc ctg gga gat gct ttc ctg cac ctc att cct cat gct ctt      634
gaa cct cat tct cac cac act ctg gag caa ccc gga cat gga cac tcc      682
cac agt ggc cag ggc ccc att ctg tct gtg gga ctg tgg gtt ctc agt      730
gga att gtt gcc ttt ctt gtc gtg gag aaa ttt gtg aga cat gtg aaa      778
gga gga cat ggt cac agt cat gga cat gga cac gct cac agt cat aca      826
cgt gga agt cat gga cat gga aga caa gag cgt tct acc aag gag aag      874
cag agc tca gag gaa gaa gaa aag gaa aca aga ggg gtt cag aag agg      922
cga gga ggg agc aca gta ccc aaa gat ggg cca gtg aga cct cag aac      970
gct gaa gaa gaa aaa aga ggc tta gac ctg cgt gtg tcg ggg tac ctg     1018
aat ctg gct gct gac ttg gca cac aac ttc act gat ggt ctg gcc att     1066
ggg gct tcc ttt cga ggg ggc cgg gga cta ggg atc ctg acc aca atg 1114
act gtc ctg cta cat gaa gtg ccc cac gag gtc gga gac ttt gcc atc 1162
ttg gtc cag tct ggc tgc agc aaa aag cag gcg atg cgt ctg caa cta 1210
ctg aca gca gta ggg gca ctg gca ggc aca gcc tgt gcc ctt ctc act 1258
gaa gga gga gca gtg ggc agt gaa att gca ggt ggt gca ggt cct ggc 1306
tgg gtc ctg cca ttt act gca ggt ggc ttt atc tac gta gca aca gtg 1354
tct gtg ttg ccc gag ctg ctg agg gag gca tca cca ttg caa tca ctt 1402
ctg gag gtg ctg ggg ctg ctg ggg gga gtt atc atg atg gtg ctg att 1450
gcc cac ctt gag tga ggggtggata aactacccct gccccaaacc tctaccccta 1505
actccaggtc aggggtgcgt agaggttggg ggccctggcc agggacatct gccaaaggaa 1565
ggaactgtag cctgggagaa tggttacttt ggcattaggg ccttcaaggg ctggcagtct 1625
tacagaggct ggagcggtga gaatgagagg ccagagggac catagtgttg ggcactgtct 1685
gaccatgttg catttggaag gctaaatggg gccatgaaga aggctggaag ggacaggggg 1745
tgatggcagc ctacctggtg tcccctaccc cacctgttct cggagaacca agttgctaca 1805
caggaagttc tccaaggtcc agtttccttt ctcccaccag ttggtggagg cttcagggaa 1865
gaccagagtc ctggacagag agggtaacag gaggagtcgg ggataaacat caaacatcaa 1925
tcgtgtgtcc tgatttggga gtgattgggg ggatggggtg ggagagggtt agttggtatt 1985
ctcatggcct gatttttttt gtttctattc cttttatatc actgtgtttg aatcgagggg 2045
gaggggtggt aaccggaaat aaagacctcc gatcttccgc cccaaaaaaa aaaaaaaaaa 2105
aaaaa 2110

<210> 26
<211> 469
<212> PRT
<213> Homo sapiens

<400> 26

<210>  27
<211>  5231
<212>  DNA
<213>  Homo sapiens

<220>
<221>  CDS
<222>  (79)..(2574)

<400>  27
cacgatttgg tgcagccggg gtttggtacc gagcggagag gagatgcaca cggcactcga      60

gtgtgaggaa aaatagaa atg aag gta cat atg cac aca aaa ttt tgc ctc      111
                    Met Lys Val His Met His Thr Lys Phe Cys Leu
                    1               5                   10

att tgt ttg ctg aca ttt att ttt cat cat tgc aac cat tgc cat gaa      159
Ile Cys Leu Leu Thr Phe Ile Phe His His Cys Asn His Cys His Glu
            15                  20                  25

gaa cat gac cat ggc cct gaa gcg ctt cac aga cag cat cgt gga atg 207
Glu His Asp His Gly Pro Glu Ala Leu His Arg Gln His Arg Gly Met
        30                  35                  40

aca gaa ttg gag cca agc aaa ttt tca aag caa gct gct gaa aat gaa 255
Thr Glu Leu Glu Pro Ser Lys Phe Ser Lys Gln Ala Ala Glu Asn Glu
    45                  50                  55

aaa aaa tac tat att gaa aaa ctt ttt gag cgt tat ggt gaa aat gga 303
Lys Lys Tyr Tyr Ile Glu Lys Leu Phe Glu Arg Tyr Gly Glu Asn Gly
60                  65                  70                  75

aga tta tcc ttt ttt ggt ttg gag aaa ctt tta aca aac ttg ggc ctt 351
Arg Leu Ser Phe Phe Gly Leu Glu Lys Leu Leu Thr Asn Leu Gly Leu
                80                  85                  90

gga gag aga aaa gta gtt gag att aat cat gag gat ctt ggc cac gat 399
Gly Glu Arg Lys Val Val Glu Ile Asn His Glu Asp Leu Gly His Asp
            95                  100                 105

cat gtt tct cat tta gat att ttg gca gtt caa gag gga aag cat ttt 447
His Val Ser His Leu Asp Ile Leu Ala Val Gln Glu Gly Lys His Phe
        110                 115                 120

cac tca cat aac cac cag cat tcc cat aat cat tta aat tca gaa aat 495
His Ser His Asn His Gln His Ser His Asn His Leu Asn Ser Glu Asn
    125                 130                 135

caa act gtg acc agt gta tcc aca aaa aga aac cat aaa tgt gat cca 543
Gln Thr Val Thr Ser Val Ser Thr Lys Arg Asn His Lys Cys Asp Pro
140                 145                 150                 155

gag aaa gag aca gtt gaa gtg tct gta aaa tct gat gat aaa cat atg 591
Glu Lys Glu Thr Val Glu Val Ser Val Lys Ser Asp Asp Lys His Met
                160                 165                 170

cat gac cat aat cac cgc cta cgt cat cac cat cgt ttg cat cat cat 639
His Asp His Asn His Arg Leu Arg His His His Arg Leu His His His
            175                 180                 185

ctt gat cat aac aac act cac cat ttt cat aat gat tcc att act ccc 687
Leu Asp His Asn Asn Thr His His Phe His Asn Asp Ser Ile Thr Pro
        190                 195                 200

agt gag cgt ggg gag cct agc aat gaa cct tca aca gag acc aat aaa 735
Ser Glu Arg Gly Glu Pro Ser Asn Glu Pro Ser Thr Glu Thr Asn Lys
    205                 210                 215

acc cag gaa caa tct gat gtt aaa cta ccg aaa gga aag agg aag aaa 783
Thr Gln Glu Gln Ser Asp Val Lys Leu Pro Lys Gly Lys Arg Lys Lys
220                 225                 230                 235

aaa ggg agg aaa agt aat gaa aat tct gag gtt att aca cca ggt ttt 831
Lys Gly Arg Lys Ser Asn Glu Asn Ser Glu Val Ile Thr Pro Gly Phe
                240                 245                 250

ccc cct aac cat gat cag ggt gaa cag tat gag cat aat cgg gtc cac 879
Pro Pro Asn His Asp Gln Gly Glu Gln Tyr Glu His Asn Arg Val His
            255                 260                 265

aaa cct gat cgt gta cat aac cca ggt cat tct cat gta cat ctt cca 927
Lys Pro Asp Arg Val His Asn Pro Gly His Ser His Val His Leu Pro
        270                 275                 280

gaa cgt aat ggt cat gat cct ggt cgt gga cac caa gat ctt gat cct 975
Glu Arg Asn Gly His Asp Pro Gly Arg Gly His Gln Asp Leu Asp Pro
    285                 290                 295

gat aat gaa ggt gaa ctt cga cat act aga aag aga gaa gca cca cat 1023
Asp Asn Glu Gly Glu Leu Arg His Thr Arg Lys Arg Glu Ala Pro His
300                 305                 310                 315

gtt aaa aat aat gca ata att tct ttg aga aaa gat cta aat gaa gat 1071
Val Lys Asn Asn Ala Ile Ile Ser Leu Arg Lys Asp Leu Asn Glu Asp
                320                 325                 330

gac cat cat cat gaa tgt ttg aac gtc act cag tta tta aaa tac tat 1119
Asp His His His Glu Cys Leu Asn Val Thr Gln Leu Leu Lys Tyr Tyr
            335                 340                 345

ggt cat ggt gcc aac tct ccc atc tca act gat tta ttt aca tac ctt 1167
Gly His Gly Ala Asn Ser Pro Ile Ser Thr Asp Leu Phe Thr Tyr Leu
        350                 355                 360

tgc cct gca ttg tta tat caa atc gac agc aga ctt tgt att gag cat 1215
Cys Pro Ala Leu Leu Tyr Gln Ile Asp Ser Arg Leu Cys Ile Glu His
    365                 370                 375

ttt gac aaa ctt tta gtt gaa gat ata aat aag gat aaa aac ctg gtt 1263
Phe Asp Lys Leu Leu Val Glu Asp Ile Asn Lys Asp Lys Asn Leu Val
380                 385                 390                 395

cct gaa gat gag gca aat ata ggg gca tca gcc tgg att tgt ggt atc 1311
Pro Glu Asp Glu Ala Asn Ile Gly Ala Ser Ala Trp Ile Cys Gly Ile
                400                 405                 410

att tct atc act gtc att agc ctg ctt tcc ttg cta ggc gtg atc ttg 1359
Ile Ser Ile Thr Val Ile Ser Leu Leu Ser Leu Leu Gly Val Ile Leu
            415                 420                 425

gtt cct atc att aac caa gga tgc ttc aaa ttc ctt ctt aca ttc ctt 1407
Val Pro Ile Ile Asn Gln Gly Cys Phe Lys Phe Leu Leu Thr Phe Leu
        430                 435                 440

gtt gca tta gct gta gga aca atg agt gga gac gcc ctt ctt cat cta 1455
Val Ala Leu Ala Val Gly Thr Met Ser Gly Asp Ala Leu Leu His Leu
    445                 450                 455

ctg ccc cat tct cag ggt gga cat gat cac agt cac caa cat gca cat 1503
Leu Pro His Ser Gln Gly Gly His Asp His Ser His Gln His Ala His
460                 465                 470                 475

ggg cat gga cat tct cat gga cat gaa tct aac aag ttt ttg gaa gaa 1551
Gly His Gly His Ser His Gly His Glu Ser Asn Lys Phe Leu Glu Glu
                480                 485                 490

tat gat gct gta ttg aaa gga ctt gtt gct cta gga ggc att tac ttg 1599
Tyr Asp Ala Val Leu Lys Gly Leu Val Ala Leu Gly Gly Ile Tyr Leu
            495                 500                 505

cta ttt atc att gaa cac tgc att aga atg ttt aag cac tac aaa caa 1647
Leu Phe Ile Ile Glu His Cys Ile Arg Met Phe Lys His Tyr Lys Gln
        510                 515                 520

caa aga gga aaa cag aaa tgg ttt atg aaa cag aac aca gaa gaa tca 1695
Gln Arg Gly Lys Gln Lys Trp Phe Met Lys Gln Asn Thr Glu Glu Ser
    525                 530                 535

act att gga aga aag ctt tca gat cac aag tta aac aat aca cca gat 1743
Thr Ile Gly Arg Lys Leu Ser Asp His Lys Leu Asn Asn Thr Pro Asp
540                 545                 550                 555

tct gac tgg ctt caa ctc aag cct ctt gcc gga act gat gac tcg gtt 1791
Ser Asp Trp Leu Gln Leu Lys Pro Leu Ala Gly Thr Asp Asp Ser Val
                560                 565                 570

gtt tct gaa gat cga ctt aat gaa act gaa ctg aca gat tta gaa ggc 1839

Val Ser Glu Asp Arg Leu Asn Glu Thr Glu Leu Thr Asp Leu Glu Gly
            575                 580                 585

caa caa gaa tcc cct cct aaa aat tac ctt tgt ata gaa gag gag aaa 1887
Gln Gln Glu Ser Pro Pro Lys Asn Tyr Leu Cys Ile Glu Glu Glu Lys
        590                 595                 600

atc ata gac cat tct cac agt gat gga tta cat acc att cat gag cat 1935
Ile Ile Asp His Ser His Ser Asp Gly Leu His Thr Ile His Glu His
    605                 610                 615

gat ctc cat gct gct gca cat aac cac cac ggc gag aac aaa act gtg 1983
Asp Leu His Ala Ala Ala His Asn His His Gly Glu Asn Lys Thr Val
620                 625                 630                 635

ctg agg aag cat aat cac cag tgg cac cac aag cat tct cat cat tcc 2031
Leu Arg Lys His Asn His Gln Trp His His Lys His Ser His His Ser
                640                 645                 650

cat ggc ccc tgt cat tct gga tcc gat ctg aaa gaa aca gga ata gct 2079
His Gly Pro Cys His Ser Gly Ser Asp Leu Lys Glu Thr Gly Ile Ala
            655                 660                 665

aat ata gcc tgg atg gtg atc atg ggg gat ggc atc cac aac ttc agt 2127
Asn Ile Ala Trp Met Val Ile Met Gly Asp Gly Ile His Asn Phe Ser
        670                 675                 680

gat ggg ctc gca att ggt gca gct ttc agt gct gga ttg aca gga gga 2175
Asp Gly Leu Ala Ile Gly Ala Ala Phe Ser Ala Gly Leu Thr Gly Gly
    685                 690                 695

atc agt act tct ata gcc gtc ttc tgt cat gaa ctg cca cat gaa tta 2223
Ile Ser Thr Ser Ile Ala Val Phe Cys His Glu Leu Pro His Glu Leu
700                 705                 710                 715

gga gat ttt gca gtt ctt ctt aaa gca ggc atg act gta aag caa gca 2271
Gly Asp Phe Ala Val Leu Leu Lys Ala Gly Met Thr Val Lys Gln Ala
                720                 725                 730

att gta tac aac ctc ctc tct gcc atg atg gct tac ata ggc atg ctc 2319
Ile Val Tyr Asn Leu Leu Ser Ala Met Met Ala Tyr Ile Gly Met Leu
            735                 740                 745

ata ggc aca gct gtt ggt cag tat gcc aat aac atc aca ctt tgg atc 2367
Ile Gly Thr Ala Val Gly Gln Tyr Ala Asn Asn Ile Thr Leu Trp Ile
        750                 755                 760

ttt gca gtc act gca ggc atg ttc ctc tat gta gcc ttg gtg gat atg 2415
Phe Ala Val Thr Ala Gly Met Phe Leu Tyr Val Ala Leu Val Asp Met
    765                 770                 775

ctt cca gaa atg ttg cat ggt gat ggt gac aat gaa gaa cat ggc ttt 2463
Leu Pro Glu Met Leu His Gly Asp Gly Asp Asn Glu Glu His Gly Phe
780                 785                 790                 795

tgt cct gtg ggg caa ttc atc ctt cag aat tta gga ttg ctc ttt gga 2511
Cys Pro Val Gly Gln Phe Ile Leu Gln Asn Leu Gly Leu Leu Phe Gly
                800                 805                 810

ttt gcc att atg ctg gtg att gcc ctc tat gaa gat aaa att gtg ttt 2559
Phe Ala Ile Met Leu Val Ile Ala Leu Tyr Glu Asp Lys Ile Val Phe
            815                 820                 825

gac atc cag ttt tga cctttcccag taatcactgt tgattacgag aatgttacca 2614
Asp Ile Gln Phe
        830

tgcagctttg catctgttcc ttgtactgta tgcacattgc tcaaaggaaa gtcagtggct 2674

tgcactactt acaagtttca tagatttgag cctaaccaca agaggctggt gcttagtact 2734
gttttccctg cacgtagggg tcttttaaaa atataaagct tgtgataaag agaggagaat 2794
atgggactcc atgaaccagt gttgatatgt ttgattaaga cttttcacaa aataatcata 2854
taaaacacta gtctctttat tagtagaaac ttctgtggct atgcagaaat agagatcgaa 2914
ccaaaaaaaa tcatttaaac tttaaaaata ttttaaatgg actttgggga gacatttttt 2974
gtgtgtttta agaatgaatt gtagtgctct ttaattcagc tacatatatt catgtggtga 3034
tagggatcaa cttgacacaa ctttgaaact gcataaagta gacataggaa ctagaggaaa 3094
gctcaggctg cattagagta tgaatttagc attgggaaaa gcccttattc ttgaatctag 3154
agttactatt tttgtatata tttgcatagt gtttaaacct gcagcctaaa ctactgaaat 3214
ttgtgattgt atgtttgtgt gagcttcagt ttaatgaaag attcataatg gttctttgta 3274
ttattataat acttggtgtt ggggtgttct ttctgttttg ttttttactt taattttgtt 3334
ttgatttttt tttttttttt tttggcgggg gtaggtgagg gtttggagca tgtggtcttt 3394
ttaaaaaatt gtaaccctct agaaaatatc aaagaaatga accagacgtg gtttaaatag 3454
ttgattttcc tattttaaca gtaccaacta gttaattggg aaatgtaagt tctgaatgtt 3514
cacattgctt taccagtttg gcactggaac caagagcaca tgtcgtggct ggctacaagg 3574
ttgtaaagca gaaaatcgaa gtttaccatg tctgtaatgt gtacatgaag tgtcaattta 3634
gaacagttac taggataaac tccattattg ccatggctgt catggtaccc aagtgacttg 3694
gaagatgcat ttaaattact cagctgaaat cacttgatca tcttgtgcca agatatgctg 3754
ttggtgcctg atagggatta gtcttttagg tgccctgttc tcctaccata attgtgaatg 3814
atttgtgaga agtgcaagcc atgtttatcc tgaattttta cttaataatt tgtattacta 3874
gtcatatgca tgtagctttc tgtttacatc ctatgccaca tggtcttcat ttatgccagg 3934
taaactgtat ttgaactatg tgcagctagc tttgttttaa tctgcttggc aaccagtgta 3994
gctgctgtaa caatctatct tattgttcaa atatataaga gccaaactct tttccattcc 4054
atctaaaatg ttttcattta gtactcttct ttcctcctac tctatgaact tcaaaacaaa 4114
aacaaaactt tgagagcagc acatgcatcc aggtatttat agattattgc cagtgtcttt 4174
tctgtatgct ataagcaagg gagcttaggt gttatttctt taatttatgc ttgaatctga 4234
aaaattattt ctgacttact ccatggcctc cttataataa gtagaagttt tatatataat 4294
taattttcag cattgggcac tgaattagga cagtcctcat ctcattgctt ggcccttcaa 4354
gcaacctagc taaaaggtgc tgatatttta tttagtactg ccaacttcaa gtgatttaga 4414
tatctatcta tctagatttc tgaaccaaga tatatttata gttcactttt gggtttttat 4474
acccacggta ggattctgca ttccagcatt aaatctgctt cattttagaa cctttataaa 4534
agcaatagct ggaatatact cccagtttta aaataaatgc ctgattgatt taaagcaagt 4594
aggttatgct gaagtatata aagaagtttt atattctctc aaaaatggta ttatctttct 4654
ttatttgcta gattcttaca aatcttttaa gagggctgta acagttgctg ctagtattag 4714
ggttccacat cattctaatg tatagtttca agtcttaata gacaatctga attccactac 4774

atttcttttg gctccaacat tccttttagc ttgaccagtc taatttaaaa tgtgtttgtt 4834

ggaggtcatt aacgttactt gtacaatgct gtcactgtgt gacatccata tgaattttgg 4894

tatatatcaa tcaatcaatc aatcacattg cattcaatca atcagctgtg attgattgat 4954

tatgcttaga aatactatag taactagatg cagtgtgaat tttttccatt aacaaacaaa 5014

caaacaagtc agtggcttaa atgtgattat ggtcctgcaa ggtgattctt gctaaaatat 5074

ctaaactttt gttttgtttt aactgaatca ttttttaact taaaaagctg gaaaatatca 5134

aatgctgttt ttttttttca ttgtcaacag tggtgtgtca ttttatgtat gttcctaatg 5194

cttatggaac tcctccaaaa taaagttact caaagag 5231

<210> 28
<211> 831
<212> PRT
<213> Homo sapiens

<400> 28

Met Lys Val His Met His Thr Lys Phe Cys Leu Ile Cys Leu Leu Thr
1               5                   10                  15

Phe Ile Phe His His Cys Asn His Cys His Glu Glu His Asp His Gly
            20                  25                  30

Pro Glu Ala Leu His Arg Gln His Arg Gly Met Thr Glu Leu Glu Pro
        35                  40                  45

Ser Lys Phe Ser Lys Gln Ala Ala Glu Asn Glu Lys Lys Tyr Tyr Ile
    50                  55                  60

Glu Lys Leu Phe Glu Arg Tyr Gly Glu Asn Gly Arg Leu Ser Phe Phe
65                  70                  75                  80

Gly Leu Glu Lys Leu Leu Thr Asn Leu Gly Leu Gly Glu Arg Lys Val
                85                  90                  95

Val Glu Ile Asn His Glu Asp Leu Gly His Asp His Val Ser His Leu
            100                 105                 110

Asp Ile Leu Ala Val Gln Glu Gly Lys His Phe His Ser His Asn His
        115                 120                 125

Gln His Ser His Asn His Leu Asn Ser Glu Asn Gln Thr Val Thr Ser
    130                 135                 140

Val Ser Thr Lys Arg Asn His Lys Cys Asp Pro Glu Lys Glu Thr Val
145                 150                 155                 160

Glu Val Ser Val Lys Ser Asp Asp Lys His Met His Asp His Asn His
                165                 170                 175

Arg Leu Arg His His His Arg Leu His His His Leu Asp His Asn Asn
            180                 185                 190

Thr His His Phe His Asn Asp Ser Ile Thr Pro Ser Glu Arg Gly Glu
        195                 200                 205

Pro Ser Asn Glu Pro Ser Thr Glu Thr Asn Lys Thr Gln Glu Gln Ser
    210                 215                 220

Asp Val Lys Leu Pro Lys Gly Lys Arg Lys Lys Lys Gly Arg Lys Ser
225                 230                 235                 240

Asn Glu Asn Ser Glu Val Ile Thr Pro Gly Phe Pro Pro Asn His Asp
                245                 250                 255

Gln Gly Glu Gln Tyr Glu His Asn Arg Val His Lys Pro Asp Arg Val
            260                 265                 270

His Asn Pro Gly His Ser His Val His Leu Pro Glu Arg Asn Gly His
        275                 280                 285

Asp Pro Gly Arg Gly His Gln Asp Leu Asp Pro Asp Asn Glu Gly Glu
    290                 295                 300

Leu Arg His Thr Arg Lys Arg Glu Ala Pro His Val Lys Asn Asn Ala
305                 310                 315                 320

Ile Ile Ser Leu Arg Lys Asp Leu Asn Glu Asp Asp His His His Glu
                325                 330                 335

Cys Leu Asn Val Thr Gln Leu Leu Lys Tyr Tyr Gly His Gly Ala Asn
            340                 345                 350

Ser Pro Ile Ser Thr Asp Leu Phe Thr Tyr Leu Cys Pro Ala Leu Leu
        355                 360                 365

Tyr Gln Ile Asp Ser Arg Leu Cys Ile Glu His Phe Asp Lys Leu Leu
    370                 375                 380

Val Glu Asp Ile Asn Lys Asp Lys Asn Leu Val Pro Glu Asp Glu Ala
385                 390                 395                 400

Asn Ile Gly Ala Ser Ala Trp Ile Cys Gly Ile Ile Ser Ile Thr Val
                405                 410                 415

Ile Ser Leu Leu Ser Leu Leu Gly Val Ile Leu Val Pro Ile Ile Asn
            420                 425                 430

Gln Gly Cys Phe Lys Phe Leu Leu Thr Phe Leu Val Ala Leu Ala Val
        435                 440                 445

Gly Thr Met Ser Gly Asp Ala Leu Leu His Leu Leu Pro His Ser Gln

    450                 455                 460

Gly Gly His Asp His Ser His Gln His Ala His Gly His Gly His Ser
465                 470                 475                 480

His Gly His Glu Ser Asn Lys Phe Leu Glu Glu Tyr Asp Ala Val Leu
                485                 490                 495

Lys Gly Leu Val Ala Leu Gly Gly Ile Tyr Leu Leu Phe Ile Ile Glu
            500                 505                 510

His Cys Ile Arg Met Phe Lys His Tyr Lys Gln Gln Arg Gly Lys Gln
        515                 520                 525

Lys Trp Phe Met Lys Gln Asn Thr Glu Glu Ser Thr Ile Gly Arg Lys
    530                 535                 540

Leu Ser Asp His Lys Leu Asn Asn Thr Pro Asp Ser Asp Trp Leu Gln
545                 550                 555                 560

Leu Lys Pro Leu Ala Gly Thr Asp Asp Ser Val Val Ser Glu Asp Arg
                565                 570                 575

Leu Asn Glu Thr Glu Leu Thr Asp Leu Glu Gly Gln Gln Glu Ser Pro
            580                 585                 590

Pro Lys Asn Tyr Leu Cys Ile Glu Glu Glu Lys Ile Ile Asp His Ser
        595                 600                 605

His Ser Asp Gly Leu His Thr Ile His Glu His Asp Leu His Ala Ala
    610                 615                 620

Ala His Asn His His Gly Glu Asn Lys Thr Val Leu Arg Lys His Asn
625                 630                 635                 640

His Gln Trp His His Lys His Ser His His Ser His Gly Pro Cys His
                645                 650                 655

Ser Gly Ser Asp Leu Lys Glu Thr Gly Ile Ala Asn Ile Ala Trp Met
            660                 665                 670

Val Ile Met Gly Asp Gly Ile His Asn Phe Ser Asp Gly Leu Ala Ile
        675                 680                 685

Gly Ala Ala Phe Ser Ala Gly Leu Thr Gly Gly Ile Ser Thr Ser Ile
    690                 695                 700

Ala Val Phe Cys His Glu Leu Pro His Glu Leu Gly Asp Phe Ala Val
705                 710                 715                 720

Leu Leu Lys Ala Gly Met Thr Val Lys Gln Ala Ile Val Tyr Asn Leu
                725                 730                 735

Leu Ser Ala Met Met Ala Tyr Ile Gly Met Leu Ile Gly Thr Ala Val
            740                 745                 750

Gly Gln Tyr Ala Asn Asn Ile Thr Leu Trp Ile Phe Ala Val Thr Ala
        755                 760                 765

Gly Met Phe Leu Tyr Val Ala Leu Val Asp Met Leu Pro Glu Met Leu
    770                 775                 780

His Gly Asp Gly Asp Asn Glu Glu His Gly Phe Cys Pro Val Gly Gln
785                 790                 795                 800

Phe Ile Leu Gln Asn Leu Gly Leu Leu Phe Gly Phe Ala Ile Met Leu
                805                 810                 815

Val Ile Ala Leu Tyr Glu Asp Lys Ile Val Phe Asp Ile Gln Phe
            820                 825                 830

<210>  29
<211>  2744
<212>  DNA
<213>  Homo sapiens

<220>
<221>  CDS
<222>  (138)..(2387)

<400>  29
ctcgtgccga attcggcacg agaccgcgtg ttcgcgcctg gtagagattt ctcgaagaca      60

ccagtgggcc cgtgtggaac caaacctgcg cgcgtggccg ggccgtggga caacgaggcc     120

gcggagacga aggcgca atg gcg agg aag tta tct gta atc ttg atc ctg        170
                   Met Ala Arg Lys Leu Ser Val Ile Leu Ile Leu
                   1               5                   10

acc ttt gcc ctc tct gtc aca aat ccc ctt cat gaa cta aaa gca gct       218
Thr Phe Ala Leu Ser Val Thr Asn Pro Leu His Glu Leu Lys Ala Ala
            15                  20                  25

gct ttc ccc cag acc act gag aaa att agt ccg aat tgg gaa tct ggc       266
Ala Phe Pro Gln Thr Thr Glu Lys Ile Ser Pro Asn Trp Glu Ser Gly
        30                  35                  40

att aat gtt gac ttg gca att tcc aca cgg caa tat cat cta caa cag       314
Ile Asn Val Asp Leu Ala Ile Ser Thr Arg Gln Tyr His Leu Gln Gln
    45                  50                  55

ctt ttc tac cgc tat gga gaa aat aat tct ttg tca gtt gaa ggg ttc       362
Leu Phe Tyr Arg Tyr Gly Glu Asn Asn Ser Leu Ser Val Glu Gly Phe
60                  65                  70                  75

aga aaa tta ctt caa aat ata ggc ata gat aag att aaa aga atc cat       410
Arg Lys Leu Leu Gln Asn Ile Gly Ile Asp Lys Ile Lys Arg Ile His
                80                  85                  90

ata cac cat gac cac gac cat cac tca gac cac gag cat cac tca gac       458
Ile His His Asp His Asp His His Ser Asp His Glu His His Ser Asp
            95                  100                 105

cat gag cgt cac tca gac cat gag cat cac tca gac cac gag cat cac 506
His Glu Arg His Ser Asp His Glu His His Ser Asp His Glu His His
        110                 115                 120

tct gac cat aat cat gct gct tct ggt aaa aat aag cga aaa gct ctt 554
Ser Asp His Asn His Ala Ala Ser Gly Lys Asn Lys Arg Lys Ala Leu
    125                 130                 135

tgc cca gac cat gac tca gat agt tca ggt aaa gat cct aga aac agc 602
Cys Pro Asp His Asp Ser Asp Ser Ser Gly Lys Asp Pro Arg Asn Ser
140                 145                 150                 155

cag ggg aaa gga gct cac cga cca gaa cat gcc agt ggt aga agg aat 650
Gln Gly Lys Gly Ala His Arg Pro Glu His Ala Ser Gly Arg Arg Asn
                160                 165                 170

gtc aag gac agt gtt agt gct agt gaa gtg acc tca act gtg tac aac 698
Val Lys Asp Ser Val Ser Ala Ser Glu Val Thr Ser Thr Val Tyr Asn
            175                 180                 185

act gtc tct gaa gga act cac ttt cta gag aca ata gag act cca aga 746
Thr Val Ser Glu Gly Thr His Phe Leu Glu Thr Ile Glu Thr Pro Arg
        190                 195                 200

cct gga aaa ctc ttc ccc aaa gat gta agc agc tcc act cca ccc agt 794
Pro Gly Lys Leu Phe Pro Lys Asp Val Ser Ser Ser Thr Pro Pro Ser
    205                 210                 215

gtc aca tca aag agc cgg gtg agc cgg ctg gct ggt agg aaa aca aat 842
Val Thr Ser Lys Ser Arg Val Ser Arg Leu Ala Gly Arg Lys Thr Asn
220                 225                 230                 235

gaa tct gtg agt gag ccc cga aaa ggc ttt atg tat tcc aga aac aca 890
Glu Ser Val Ser Glu Pro Arg Lys Gly Phe Met Tyr Ser Arg Asn Thr
                240                 245                 250

aat gaa aat cct cag gag tgt ttc aat gca tca aag cta ctg aca tct 938
Asn Glu Asn Pro Gln Glu Cys Phe Asn Ala Ser Lys Leu Leu Thr Ser
            255                 260                 265

cat ggc atg ggc atc cag gtt ccg ctg aat gca aca gag ttc aac tat 986
His Gly Met Gly Ile Gln Val Pro Leu Asn Ala Thr Glu Phe Asn Tyr
        270                 275                 280

ctc tgt cca gcc atc atc aac caa att gat gct aga tct tgt ctg att 1034
Leu Cys Pro Ala Ile Ile Asn Gln Ile Asp Ala Arg Ser Cys Leu Ile
    285                 290                 295

cat aca agt gaa aag aag gct gaa atc cct cca aag acc tat tca tta 1082
His Thr Ser Glu Lys Lys Ala Glu Ile Pro Pro Lys Thr Tyr Ser Leu
300                 305                 310                 315

caa ata gcc tgg gtt ggt ggt ttt ata gcc att tcc atc atc agt ttc 1130
Gln Ile Ala Trp Val Gly Gly Phe Ile Ala Ile Ser Ile Ile Ser Phe
                320                 325                 330

ctg tct ctg ctg ggg gtt atc tta gtg cct ctc atg aat cgg gtg ttt 1178
Leu Ser Leu Leu Gly Val Ile Leu Val Pro Leu Met Asn Arg Val Phe
            335                 340                 345

ttc aaa ttt ctc ctg agt ttc ctt gtg gca ctg gcc gtt ggg act ttg 1226
Phe Lys Phe Leu Leu Ser Phe Leu Val Ala Leu Ala Val Gly Thr Leu
        350                 355                 360

agt ggt gat gct ttt tta cac ctt ctt cca cat tct cat gca agt cac 1274
Ser Gly Asp Ala Phe Leu His Leu Leu Pro His Ser His Ala Ser His
    365                 370                 375

cac cat agt cat agc cat gaa gaa cca gca atg gaa atg aaa aga gga 1322

His His Ser His Ser His Glu Glu Pro Ala Met Glu Met Lys Arg Gly
380                 385                 390                 395

cca ctt ttc agt cat ctg tct tct caa aac ata gaa gaa agt gcc tat      1370
Pro Leu Phe Ser His Leu Ser Ser Gln Asn Ile Glu Glu Ser Ala Tyr
                400                 405                 410

ttt gat tcc acg tgg aag ggt cta aca gct cta gga ggc ctg tat ttc      1418
Phe Asp Ser Thr Trp Lys Gly Leu Thr Ala Leu Gly Gly Leu Tyr Phe
            415                 420                 425

atg ttt ctt gtt gaa cat gtc ctc aca ttg atc aaa caa ttt aaa gat      1466
Met Phe Leu Val Glu His Val Leu Thr Leu Ile Lys Gln Phe Lys Asp
        430                 435                 440

aag aag aaa aag aat cag aag aaa cct gaa aat gat gat gat gtg gag      1514
Lys Lys Lys Lys Asn Gln Lys Lys Pro Glu Asn Asp Asp Asp Val Glu
    445                 450                 455

att aag aag cag ttg tcc aag tat gaa tct caa ctt tca aca aat gag      1562
Ile Lys Lys Gln Leu Ser Lys Tyr Glu Ser Gln Leu Ser Thr Asn Glu
460                 465                 470                 475

gag aaa gta gat aca gat gat cga act gaa ggc tat tta cga gca gac      1610
Glu Lys Val Asp Thr Asp Asp Arg Thr Glu Gly Tyr Leu Arg Ala Asp
                480                 485                 490

tca caa gag ccc tcc cac ttt gat tct cag cag cct gca gtc ttg gaa      1658
Ser Gln Glu Pro Ser His Phe Asp Ser Gln Gln Pro Ala Val Leu Glu
            495                 500                 505

gaa gaa gag gtc atg ata gct cat gct cat cca cag gaa gtc tac aat      1706
Glu Glu Glu Val Met Ile Ala His Ala His Pro Gln Glu Val Tyr Asn
        510                 515                 520

gaa tat gta ccc aga ggg tgc aag aat aaa tgc cat tca cat ttc cac      1754
Glu Tyr Val Pro Arg Gly Cys Lys Asn Lys Cys His Ser His Phe His
    525                 530                 535

gat aca ctc ggc cag tca gac gat ctc att cac cac cat cat gac tac      1802
Asp Thr Leu Gly Gln Ser Asp Asp Leu Ile His His His His Asp Tyr
540                 545                 550                 555

cat cat att ctc cat cat cac cac cac caa aac cac cat cct cac agt      1850
His His Ile Leu His His His His His Gln Asn His His Pro His Ser
                560                 565                 570

cac agc cag cgc tac tct cgg gag gag ctg aaa gat gcc ggc gtc gcc      1898
His Ser Gln Arg Tyr Ser Arg Glu Glu Leu Lys Asp Ala Gly Val Ala
            575                 580                 585

act ttg gcc tgg atg gtg ata atg ggt gat ggc ctg cac aat ttc agc      1946
Thr Leu Ala Trp Met Val Ile Met Gly Asp Gly Leu His Asn Phe Ser
        590                 595                 600

gat ggc cta gca att ggt gct gct ttt act gaa ggc tta tca agt ggt      1994
Asp Gly Leu Ala Ile Gly Ala Ala Phe Thr Glu Gly Leu Ser Ser Gly
    605                 610                 615

tta agt act tct gtt gct gtg ttc tgt cat gag ttg cct cat gaa tta      2042
Leu Ser Thr Ser Val Ala Val Phe Cys His Glu Leu Pro His Glu Leu
620                 625                 630                 635

ggt gac ttt gct gtt cta cta aag gct ggc atg acc gtt aag cag gct      2090
Gly Asp Phe Ala Val Leu Leu Lys Ala Gly Met Thr Val Lys Gln Ala
                640                 645                 650

gtc ctt tat aat gca ttg tca gcc atg ctg gcg tat ctt gga atg gca      2138
Val Leu Tyr Asn Ala Leu Ser Ala Met Leu Ala Tyr Leu Gly Met Ala

            655                 660                 665

aca gga att ttc att ggt cat tat gct gaa aat gtt tct atg tgg ata 2186
Thr Gly Ile Phe Ile Gly His Tyr Ala Glu Asn Val Ser Met Trp Ile
        670                 675                 680

ttt gca ctt act gct ggc tta ttc atg tat gtt gct ctg gtt gat atg 2234
Phe Ala Leu Thr Ala Gly Leu Phe Met Tyr Val Ala Leu Val Asp Met
    685                 690                 695

gta cct gaa atg ctg cac aat gat gct agt gac cat gga tgt agc cgc 2282
Val Pro Glu Met Leu His Asn Asp Ala Ser Asp His Gly Cys Ser Arg
700                 705                 710                 715

tgg ggg tat ttc ttt tta cag aat gct ggg atg ctt ttg ggt ttt gga 2330
Trp Gly Tyr Phe Phe Leu Gln Asn Ala Gly Met Leu Leu Gly Phe Gly
                720                 725                 730

att atg tta ctt att tcc ata ttt gaa cat aaa atc gtg ttt cgt ata 2378
Ile Met Leu Leu Ile Ser Ile Phe Glu His Lys Ile Val Phe Arg Ile
            735                 740                 745

aat ttc tag ttaaggttta aatgctagag tagcttaaaa agttgtcata 2427
Asn Phe

gtttcagtag gtcataggga gatgagtttg tatgctgtac tatgcagcgt ttaaagttag 2487

tgggttttgt gatttttgta ttgaatattg ctgtctgtta caaagtcagt taaaggtacg 2547

ttttaatatt taagttattc tatcttggag ataaaatctg tatgtgcaat tcaccggtat 2607

taccagttta ttatgtaaac aagagatttg gcatgacatg ttctgtatgt ttcagggaaa 2667

aatgtcttta atgctttttc aagaactaac acagttattc ctatactgga ttttaggtct 2727

ctgaagaact gctggtg 2744

<210> 30
<211> 749
<212> PRT
<213> Homo sapiens

<400> 30

Met Ala Arg Lys Leu Ser Val Ile Leu Ile Leu Thr Phe Ala Leu Ser
1               5                   10                  15

Val Thr Asn Pro Leu His Glu Leu Lys Ala Ala Ala Phe Pro Gln Thr
            20                  25                  30

Thr Glu Lys Ile Ser Pro Asn Trp Glu Ser Gly Ile Asn Val Asp Leu
        35                  40                  45

Ala Ile Ser Thr Arg Gln Tyr His Leu Gln Gln Leu Phe Tyr Arg Tyr
    50                  55                  60

Gly Glu Asn Asn Ser Leu Ser Val Glu Gly Phe Arg Lys Leu Leu Gln
65                  70                  75                  80

Asn Ile Gly Ile Asp Lys Ile Lys Arg Ile His Ile His His Asp His
                85                  90                  95

Asp His His Ser Asp His Glu His His Ser Asp His Glu Arg His Ser
            100                 105                 110

Asp His Glu His His Ser Asp His Glu His His Ser Asp His Asn His
        115                 120                 125

Ala Ala Ser Gly Lys Asn Lys Arg Lys Ala Leu Cys Pro Asp His Asp
    130                 135                 140

Ser Asp Ser Ser Gly Lys Asp Pro Arg Asn Ser Gln Gly Lys Gly Ala
145                 150                 155                 160

His Arg Pro Glu His Ala Ser Gly Arg Arg Asn Val Lys Asp Ser Val
                165                 170                 175

Ser Ala Ser Glu Val Thr Ser Thr Val Tyr Asn Thr Val Ser Glu Gly
            180                 185                 190

Thr His Phe Leu Glu Thr Ile Glu Thr Pro Arg Pro Gly Lys Leu Phe
        195                 200                 205

Pro Lys Asp Val Ser Ser Ser Thr Pro Pro Ser Val Thr Ser Lys Ser
    210                 215                 220

Arg Val Ser Arg Leu Ala Gly Arg Lys Thr Asn Glu Ser Val Ser Glu
225                 230                 235                 240

Pro Arg Lys Gly Phe Met Tyr Ser Arg Asn Thr Asn Glu Asn Pro Gln
                245                 250                 255

Glu Cys Phe Asn Ala Ser Lys Leu Leu Thr Ser His Gly Met Gly Ile
            260                 265                 270

Gln Val Pro Leu Asn Ala Thr Glu Phe Asn Tyr Leu Cys Pro Ala Ile
        275                 280                 285

Ile Asn Gln Ile Asp Ala Arg Ser Cys Leu Ile His Thr Ser Glu Lys
    290                 295                 300

Lys Ala Glu Ile Pro Pro Lys Thr Tyr Ser Leu Gln Ile Ala Trp Val
305                 310                 315                 320

Gly Gly Phe Ile Ala Ile Ser Ile Ile Ser Phe Leu Ser Leu Leu Gly
                325                 330                 335

Val Ile Leu Val Pro Leu Met Asn Arg Val Phe Phe Lys Phe Leu Leu
            340                 345                 350

Ser Phe Leu Val Ala Leu Ala Val Gly Thr Leu Ser Gly Asp Ala Phe
        355                 360                 365

Leu His Leu Leu Pro His Ser His Ala Ser His His His Ser His Ser
    370                 375                 380

His Glu Glu Pro Ala Met Glu Met Lys Arg Gly Pro Leu Phe Ser His
385                 390                 395                 400

Leu Ser Ser Gln Asn Ile Glu Glu Ser Ala Tyr Phe Asp Ser Thr Trp
                405                 410                 415

Lys Gly Leu Thr Ala Leu Gly Gly Leu Tyr Phe Met Phe Leu Val Glu
            420                 425                 430

His Val Leu Thr Leu Ile Lys Gln Phe Lys Asp Lys Lys Lys Lys Asn
        435                 440                 445

Gln Lys Lys Pro Glu Asn Asp Asp Asp Val Glu Ile Lys Lys Gln Leu
    450                 455                 460

Ser Lys Tyr Glu Ser Gln Leu Ser Thr Asn Glu Glu Lys Val Asp Thr
465                 470                 475                 480

Asp Asp Arg Thr Glu Gly Tyr Leu Arg Ala Asp Ser Gln Glu Pro Ser
                485                 490                 495

His Phe Asp Ser Gln Gln Pro Ala Val Leu Glu Glu Glu Glu Val Met
            500                 505                 510

Ile Ala His Ala His Pro Gln Glu Val Tyr Asn Glu Tyr Val Pro Arg
        515                 520                 525

Gly Cys Lys Asn Lys Cys His Ser His Phe His Asp Thr Leu Gly Gln
    530                 535                 540

Ser Asp Asp Leu Ile His His His His Asp Tyr His His Ile Leu His
545                 550                 555                 560

His His His His Gln Asn His His Pro His Ser His Ser Gln Arg Tyr
                565                 570                 575

Ser Arg Glu Glu Leu Lys Asp Ala Gly Val Ala Thr Leu Ala Trp Met
            580                 585                 590

Val Ile Met Gly Asp Gly Leu His Asn Phe Ser Asp Gly Leu Ala Ile
        595                 600                 605

Gly Ala Ala Phe Thr Glu Gly Leu Ser Ser Gly Leu Ser Thr Ser Val
    610                 615                 620

Ala Val Phe Cys His Glu Leu Pro His Glu Leu Gly Asp Phe Ala Val
625                 630                 635                 640

Leu Leu Lys Ala Gly Met Thr Val Lys Gln Ala Val Leu Tyr Asn Ala

645                 650                 655

Leu Ser Ala Met Leu Ala Tyr Leu Gly Met Ala Thr Gly Ile Phe Ile
            660                 665                 670

Gly His Tyr Ala Glu Asn Val Ser Met Trp Ile Phe Ala Leu Thr Ala
        675                 680                 685

Gly Leu Phe Met Tyr Val Ala Leu Val Asp Met Val Pro Glu Met Leu
    690                 695                 700

His Asn Asp Ala Ser Asp His Gly Cys Ser Arg Trp Gly Tyr Phe Phe
705                 710                 715                 720

Leu Gln Asn Ala Gly Met Leu Leu Gly Phe Gly Ile Met Leu Leu Ile
                725                 730                 735

Ser Ile Phe Glu His Lys Ile Val Phe Arg Ile Asn Phe
            740                 745

<210>  31
<211>  4627
<212>  DNA
<213>  Homo sapiens

<220>
<221>  CDS
<222>  (147)..(1625)

<400>  31
ccccggacgc agtgaggggg gtcggcgcgc gtgtctacgc ggacgcaccg gctaagctgc      60

ttctgccgcc gccggccgcc tgggaccttg cggtgaggct gcgcggggcc gaggccgcct     120

ccgagcgcca ggtttattca gtcacc atg aag ctg ctg ctg ctg cac ccg gcc      173
                             Met Lys Leu Leu Leu Leu His Pro Ala
                             1               5

ttc cag agc tgc ctc ctg ctg acc ctg ctt ggc tta tgg aga acc acc      221
Phe Gln Ser Cys Leu Leu Leu Thr Leu Leu Gly Leu Trp Arg Thr Thr
10                  15                  20                  25

cct gag gct cac gct tca tcc ctg ggt gca cca gct atc agc gct gcc      269
Pro Glu Ala His Ala Ser Ser Leu Gly Ala Pro Ala Ile Ser Ala Ala
                30                  35                  40

tcc ttc ctg cag gat cta ata cat cgg tat ggc gag ggt gac agc ccc      317
Ser Phe Leu Gln Asp Leu Ile His Arg Tyr Gly Glu Gly Asp Ser Pro
            45                  50                  55

act ctg cag cag ctg aag gcc cta ctc aac cac ctg gat gtg gga gtg      365
Thr Leu Gln Gln Leu Lys Ala Leu Leu Asn His Leu Asp Val Gly Val
        60                  65                  70

ggc cgg ggt aat gtc acc cag cac gtg caa gga cac agg aac ctc tcc      413
Gly Arg Gly Asn Val Thr Gln His Val Gln Gly His Arg Asn Leu Ser
    75                  80                  85

acg tgc ttt agt tct gga gac ctc ttc act gcc cac aat ttc agc gag      461
Thr Cys Phe Ser Ser Gly Asp Leu Phe Thr Ala His Asn Phe Ser Glu
90                  95                  100                 105

cag tcg cgg att ggg agc agc gag ctc cag gag ttc tgc ccc acc atc 509
Gln Ser Arg Ile Gly Ser Ser Glu Leu Gln Glu Phe Cys Pro Thr Ile
                110                 115                 120

ctc cag cag ctg gat tcc cgg gcc tgc acc tcg gag aac cag gaa aac 557
Leu Gln Gln Leu Asp Ser Arg Ala Cys Thr Ser Glu Asn Gln Glu Asn
            125                 130                 135

gag gag aat gag cag acg gag gag ggg cgg cca agc gct gtt gaa gtg 605
Glu Glu Asn Glu Gln Thr Glu Glu Gly Arg Pro Ser Ala Val Glu Val
        140                 145                 150

tgg ggc ttt ggt ttt ctc agt gtc tca ctg att aac ctg gcc tct ctc 653
Trp Gly Phe Gly Phe Leu Ser Val Ser Leu Ile Asn Leu Ala Ser Leu
    155                 160                 165

ctg gga gtc ctc gtc ctg ccc tgc aca gag aaa gcg ttt ttc agc cgt 701
Leu Gly Val Leu Val Leu Pro Cys Thr Glu Lys Ala Phe Phe Ser Arg
170                 175                 180                 185

gtg ctc act tac ttc atc gcc ctg tcc att gga acg ctg ctg tct aac 749
Val Leu Thr Tyr Phe Ile Ala Leu Ser Ile Gly Thr Leu Leu Ser Asn
                190                 195                 200

gcg cta ttc cag ctc atc cca gag gca ttt ggt ttc aac cct ctg gaa 797
Ala Leu Phe Gln Leu Ile Pro Glu Ala Phe Gly Phe Asn Pro Leu Glu
            205                 210                 215

gat tat tat gtc tcc aag tct gca gtg gtg ttt ggg ggc ttt tat ctt 845
Asp Tyr Tyr Val Ser Lys Ser Ala Val Val Phe Gly Gly Phe Tyr Leu
        220                 225                 230

ttc ttt ttc aca gag aag atc ttg aag att ctt ctt aag cag aaa aat 893
Phe Phe Phe Thr Glu Lys Ile Leu Lys Ile Leu Leu Lys Gln Lys Asn
    235                 240                 245

gag cat cat cat gga cac agc cat tat gcc tct gag tcg ctt ccc tcc 941
Glu His His His Gly His Ser His Tyr Ala Ser Glu Ser Leu Pro Ser
250                 255                 260                 265

aag aag gac cag gag gag ggg gtg atg gag aag ctg cag aac ggg gac 989
Lys Lys Asp Gln Glu Glu Gly Val Met Glu Lys Leu Gln Asn Gly Asp
                270                 275                 280

ctg gac cac atg att cct cag cac tgc agc agt gag ctg gac ggc aag 1037
Leu Asp His Met Ile Pro Gln His Cys Ser Ser Glu Leu Asp Gly Lys
            285                 290                 295

gcg ccc atg gtg gac gag aag gtc att gtg ggc tcg ctc tct gtg cag 1085
Ala Pro Met Val Asp Glu Lys Val Ile Val Gly Ser Leu Ser Val Gln
        300                 305                 310

gac ctg cag gct tcc cag agt gct tgc tac tgg ctg aaa ggt gtc cgc 1133
Asp Leu Gln Ala Ser Gln Ser Ala Cys Tyr Trp Leu Lys Gly Val Arg
    315                 320                 325

tac tct gat atc ggc act ctg gcc tgg atg atc act ctg agc gac ggc 1181
Tyr Ser Asp Ile Gly Thr Leu Ala Trp Met Ile Thr Leu Ser Asp Gly
330                 335                 340                 345

ctc cat aat ttc atc gat ggc ctg gcc atc ggt gct tcc ttc act gtg 1229
Leu His Asn Phe Ile Asp Gly Leu Ala Ile Gly Ala Ser Phe Thr Val
                350                 355                 360

tca gtt ttc caa ggc atc agc acc tcg gtg gcc atc ctc tgt gag gag 1277
Ser Val Phe Gln Gly Ile Ser Thr Ser Val Ala Ile Leu Cys Glu Glu
            365                 370                 375

ttc cca cgt gag cta gga gac ttt gtc atc ctg ctc aac gct ggg atg     1325
Phe Pro Arg Glu Leu Gly Asp Phe Val Ile Leu Leu Asn Ala Gly Met
        380                 385                 390

agc atc caa caa gct ctc ttc ttc aac ttc ctt tct gcc tgc tgc tgc     1373
Ser Ile Gln Gln Ala Leu Phe Phe Asn Phe Leu Ser Ala Cys Cys Cys
    395                 400                 405

tac ctg ggt ctg gcc ttt ggc atc ctg gcc ggc agc cac ttc tct gcc     1421
Tyr Leu Gly Leu Ala Phe Gly Ile Leu Ala Gly Ser His Phe Ser Ala
410                 415                 420                 425

aac tgg att ttt gcg cta gct gga gga atg ttc ttg tat att tct ctg     1469
Asn Trp Ile Phe Ala Leu Ala Gly Gly Met Phe Leu Tyr Ile Ser Leu
                430                 435                 440

gct gat atg ttc cct gag atg aat gag gtc tgt caa gag gat gaa agg     1517
Ala Asp Met Phe Pro Glu Met Asn Glu Val Cys Gln Glu Asp Glu Arg
            445                 450                 455

aag ggc agc atc ttg att cca ttt atc atc cag aac ctg ggc ctc ctg     1565
Lys Gly Ser Ile Leu Ile Pro Phe Ile Ile Gln Asn Leu Gly Leu Leu
        460                 465                 470

act gga ttc acc atc atg gtg gtc ctc acc atg tat tca gga cag atc     1613
Thr Gly Phe Thr Ile Met Val Val Leu Thr Met Tyr Ser Gly Gln Ile
    475                 480                 485

cag att ggg tag ggctctgcca agagcctgtg ggactggaag tcgggccctg     1665
Gln Ile Gly
490

ggctgcccga tcgccagccc gaggacttac catccacaat gcaccacgga agaggccgtt     1725

ctatgaaaaa ctgacacaga ctgtattcct gcattcaaat gtcagccgtt tgtaaaatgc     1785

tgtatcctag gaataagctg ccctggtaac cagtctctag ctagtgcctc ttgccctctc     1845

ctcacctcct tttctctcag tgactctgga acctgaatgc agcttacaag acaagcctga     1905

cttttttctc tgattacctt ggcctcctct tggaaccagt gctgaaaggt tttgaatcct     1965

ttacccaaca atgcaaaaat agagccaatg gttataactt ggctagaaat atcaagagtt     2025

gaatccatag tgtggggccc atgactctag ctgggcacct tggacctcca gctggccaat     2085

agaagagaca ggagacagga agccttccca ttttttcaaa gtctgtttaa ttgcctatta     2145

cttctctcaa agagaacctg aagtcagaac acatgagcag ggtgagaggt gaggcaaggt     2205

tcatcctgaa tgggagagga agtcgaacca ctgctgtgtg tcttgtcagg atgctcactt     2265

gttcctactg agatgctgga tattgatttt gtaacagcac ctggtgtttc acggctgtcc     2325

gagtgagcta acgtggcggt gtggctgcct ggacctcctc tttcaggtta acgctgacag     2385

aatggaggct caggctgtct gcaagaaaac agttggtttg gctgtgattt tgacctcctc     2445

ttccccactg ccatcttcta agagactttg tagctgcctc ctagaagcac attctgagca     2505

catttgagac ctctgtgtta gaggggagac tgcacaaact atcctccccc aggttgagac     2565

gtctgcagag tggcaagctg acttgtagaa atggggtgcc atttatgctc tacttagaca     2625

agggtaatca gaaatggaat cagtgcaggc aaaatttagg atttgccgct tccataaatc     2685

aaagcatgac taataggggg tctctgaaat gtaagggcac aaacttcact tagggcatcg     2745

cagatgtttg cagaatggtt ggcctaatga ttatgctaca gatgggtttt aaatgacccg     2805

tctaggttac tgcttccttg caaaaaagtc gaatcctgca ttgaattgaa tatgaatttc 2865
tctaactctc tccagaaaat ggatggagat aacttgtctt taaaactgta ggccagcctt 2925
ggccactgtg gagcccttgc ctccgagctc tggcttcaag gggagctctt ctccaggttc 2985
actaggtgaa ttgatttatt attatcatat tgataatgtg agattcttta gccactttgg 3045
ggagcctgtc cctccagaag cctttcttag tggtgcccac agttggagcc caggggccat 3105
gtttgcaaac tgattcatgt gcatggctga caggagtact ggttcactac caatgcctga 3165
gcttttctct tacatagaaa aactgtccgc tctcagtaat cacaagcagc atccgttttg 3225
ttttctcttc ttgggagaca tctgtcaaac caggaatatt cttgaaaaga acgtgagcag 3285
gaaaaactgc tggtgatact ttttttaagt tttgttttta tcttgcctgt tggcttcaat 3345
acatttgaga atacgctgaa gagggaaaat ttcagtgatg gagattctag attaaatatc 3405
aggactgatt tcctggtggg attatggtcc agttttacca aagaaccaat tccttgaatg 3465
ttggaatcta actttttata ttgtcattat tattgttgtt tttaaacggt tctttgtctt 3525
ttctgtttta tttttctcaa gctgctttca ggagctagca gaaaataact caaagttgaa 3585
gactctggaa gattttgctt taacctaact cgcattgatg tattaaattt ataattttag 3645
cattcccaat agatcctatc attccttaaa cataataccc tttgtcttgg agtagaatac 3705
taagttagag ttagtggatt tctagtttag gagaggagct caaaactata atctttaaca 3765
aattgaaaaa tgaaataggg tgttttccct ttttgtgcac acctatatta ccttaagaaa 3825
tttccttcca tagacagctg cctcaaaggg aaatcctctt taaaccgtag ttggcgcaga 3885
ggtcagtcct agtcggagct taggaggggc ggagacgctc acatcgtctg acttgagtcg 3945
ccactgattg tggcaacagc tttgcctcat gagtcaaaaa ttggcaattt cttttgattt 4005
ttagttgttg aatttgctgt ttcaagcatt tgtacatatt agaagtctaa ggagtagcaa 4065
gtcagtggga ggactttttc acccctggca ttagcagctt cgacctcatt ttccagatgc 4125
accagctcct attaataagt tagcaaggaa agtgtatgtc acgtgcagga acagtgaggc 4185
agggacaggg gttctgctcc ttctcacttc accaccggca cacagcttgc ccctgtcttt 4245
gcccccaaag gtattttgtg tctagtgtca aattggagct attcttcact ggtccttaac 4305
cttgggtttt aaaaagaagg cttctctgtt tgggtagcgt aagagctgag tatagtaagt 4365
cctcttccaa agagatggca atatgctggg catctacttt aaaacaaagt tgtctgattt 4425
ttgcaagaga ggttaggatt ttattgttct tatttccctt tacagttctg cagttccatc 4485
acagtatttt tttaaataac tcaggtgtat gagaagaaat tagaaaagaa aattaactta 4545
tgtggactgt aaatgtttta tttgtaagat tctataaata aagctatatt ctgtaattgt 4605
tgagaaaaaa aaaaaaaaaa aa 4627

<210> 32
<211> 492
<212> PRT
<213> Homo sapiens

<400> 32

Met Lys Leu Leu Leu Leu His Pro Ala Phe Gln Ser Cys Leu Leu Leu
1               5                   10                  15

Thr Leu Leu Gly Leu Trp Arg Thr Thr Pro Glu Ala His Ala Ser Ser
            20                  25                  30

Leu Gly Ala Pro Ala Ile Ser Ala Ala Ser Phe Leu Gln Asp Leu Ile
        35                  40                  45

His Arg Tyr Gly Glu Gly Asp Ser Pro Thr Leu Gln Gln Leu Lys Ala
    50                  55                  60

Leu Leu Asn His Leu Asp Val Gly Val Gly Arg Gly Asn Val Thr Gln
65                  70                  75                  80

His Val Gln Gly His Arg Asn Leu Ser Thr Cys Phe Ser Ser Gly Asp
                85                  90                  95

Leu Phe Thr Ala His Asn Phe Ser Glu Gln Ser Arg Ile Gly Ser Ser
            100                 105                 110

Glu Leu Gln Glu Phe Cys Pro Thr Ile Leu Gln Gln Leu Asp Ser Arg
        115                 120                 125

Ala Cys Thr Ser Glu Asn Gln Glu Asn Glu Glu Asn Glu Gln Thr Glu
    130                 135                 140

Glu Gly Arg Pro Ser Ala Val Glu Val Trp Gly Phe Gly Phe Leu Ser
145                 150                 155                 160

Val Ser Leu Ile Asn Leu Ala Ser Leu Leu Gly Val Leu Val Leu Pro
                165                 170                 175

Cys Thr Glu Lys Ala Phe Phe Ser Arg Val Leu Thr Tyr Phe Ile Ala
            180                 185                 190

Leu Ser Ile Gly Thr Leu Leu Ser Asn Ala Leu Phe Gln Leu Ile Pro
        195                 200                 205

Glu Ala Phe Gly Phe Asn Pro Leu Glu Asp Tyr Tyr Val Ser Lys Ser
    210                 215                 220

Ala Val Val Phe Gly Gly Phe Tyr Leu Phe Phe Phe Thr Glu Lys Ile
225                 230                 235                 240

Leu Lys Ile Leu Leu Lys Gln Lys Asn Glu His His His Gly His Ser
                245                 250                 255

His Tyr Ala Ser Glu Ser Leu Pro Ser Lys Lys Asp Gln Glu Glu Gly
            260                 265                 270

Val Met Glu Lys Leu Gln Asn Gly Asp Leu Asp His Met Ile Pro Gln
        275                 280                 285

His Cys Ser Ser Glu Leu Asp Gly Lys Ala Pro Met Val Asp Glu Lys
    290                 295                 300

Val Ile Val Gly Ser Leu Ser Val Gln Asp Leu Gln Ala Ser Gln Ser
305                 310                 315                 320

Ala Cys Tyr Trp Leu Lys Gly Val Arg Tyr Ser Asp Ile Gly Thr Leu
                325                 330                 335

Ala Trp Met Ile Thr Leu Ser Asp Gly Leu His Asn Phe Ile Asp Gly
            340                 345                 350

Leu Ala Ile Gly Ala Ser Phe Thr Val Ser Val Phe Gln Gly Ile Ser
        355                 360                 365

Thr Ser Val Ala Ile Leu Cys Glu Glu Phe Pro Arg Glu Leu Gly Asp
    370                 375                 380

Phe Val Ile Leu Leu Asn Ala Gly Met Ser Ile Gln Gln Ala Leu Phe
385                 390                 395                 400

Phe Asn Phe Leu Ser Ala Cys Cys Cys Tyr Leu Gly Leu Ala Phe Gly
                405                 410                 415

Ile Leu Ala Gly Ser His Phe Ser Ala Asn Trp Ile Phe Ala Leu Ala
            420                 425                 430

Gly Gly Met Phe Leu Tyr Ile Ser Leu Ala Asp Met Phe Pro Glu Met
        435                 440                 445

Asn Glu Val Cys Gln Glu Asp Glu Arg Lys Gly Ser Ile Leu Ile Pro
    450                 455                 460

Phe Ile Ile Gln Asn Leu Gly Leu Leu Thr Gly Phe Thr Ile Met Val
465                 470                 475                 480

Val Leu Thr Met Tyr Ser Gly Gln Ile Gln Ile Gly
                485                 490

<210>  33
<211>  2178
<212>  DNA
<213>  Homo sapiens

<220>
<221>  CDS
<222>  (87)..(2030)

<400>  33
caaccccagc aaagccgccc tcagccagcc cagaagcact gggccttggc cacagcaaca      60
cccactgagc acgctgggag ctgagt atg gcg tcc ctg gtc tcg ctg gag ctg 113
ggg ctg ctt ctg gct gtg ctg gtg gtg acg gcg acg gcg tcc ccg cct 161
gct ggt ctg ctg agc ctg ctc acc tct ggc cag ggc gct ctg gat caa 209
gag gct ctg ggc ggc ctg tta aat acg ctg gcg gac cgt gtg cac tgc 257
acc aac ggg ccg tgt gga aag tgc ctg tct gtg gag gac gcc ctg ggc 305
ctg ggc gag cct gag ggg tca ggg ctg ccc ccg ggc ccg gtc ctg gag 353
gcc agg tac gtc gcc cgc ctc agt gcc gcc gcc gtc ctg tac ctc agc 401
aac ccc gag ggc acc tgt gag gac act cgg gct ggc ctc tgg gcc tct 449
cat gca gac cac ctc ctg gcc ctg ctc gag agc ccc aag gcc ctg acc 497
ccg ggc ctg agc tgg ctg ctg cag agg atg cag gcc cgg gct gcc ggc 545
cag acc ccc aag acg gcc tgc gta gat atc cct cag ctg ctg gag gag 593
gcg gtg ggg gcg ggg gct ccg ggc agt gct ggc ggc gtc ctg gct gcc 641
ctg ctg gac cat gtc agg agc ggg tct tgc ttc cac gcc ttg ccg agc 689
cct cag tac ttc gtg gac ttt gtg ttc cag cag cac agc agc gag gtc 737
cct atg acg ctg gcc gag ctg tca gcc ttg atg cag cgc ctg ggg gtg 785
ggc agg gag gcc cac agt gac cac agt cat cgg cac agg gga gcc agc 833
agc cgg gac cct gtg ccc ctc atc agc tcc agc aac agc tcc agt gtg 881
tgg gac acg gta tgc ctg agt gcc agg gac gtg atg gct gca tat gga 929
ctg tcg gaa cag gct ggg gtg acc ccg gag gcc tgg gcc caa ctg agc 977
cct gcc ctg ctc caa cag cag ctg agt gga gcc tgc acc tcc cag tcc 1025
agg ccc ccc gtc cag gac cag ctc agc cag tca gag agg tat ctg tac 1073
ggc tcc ctg gcc acg ctg ctc atc tgc ctc tgc gcg gtc ttt ggc ctc 1121
ctg ctg ctg acc tgc act ggc tgc agg ggg gtc acc cac tac atc ctg 1169
cag acc ttc ctg agc ctg gca gtg ggt gca ctc act ggg gac gct gtc 1217
ctg cat ctg acg ccc aag gtg ctg ggg ctg cat aca cac agc gaa gag 1265
ggc ctc agc cca cag ccc acc tgg cgc ctc ctg gct atg ctg gcc ggg 1313
ctc tac gcc ttc ttc ctg ttt gag aac ctc ttc aat ctc ctg ctg ccc 1361
agg gac ccg gag gac ctg gag gac ggg ccc tgc ggc cac agc agc cat 1409
agc cac ggg ggc cac agc cac ggt gtg tcc ctg cag ctg gca ccc agc 1457
gag ctc cgg cag ccc aag ccc ccc cac gag ggc tcc cgc gca gac ctg 1505
gtg gcg gag gag agc ccg gag ctg ctg aac cct gag ccc agg aga ctg 1553
agc cca gag ttg agg cta ctg ccc tat atg atc act ctg ggc gac gcc 1601
gtg cac aac ttc gcc gac ggg ctg gcc gtg ggc gcc gcc ttc gcg tcc 1649
tcc tgg aag acc ggg ctg gcc acc tcg ctg gcc gtg ttc tgc cac gag 1697
ttg cca cac gag ctg ggg gac ttc gcc gcc ttg ctg cac gcg ggg ctg 1745
tcc gtg cgc caa gca ctg ctg ctg aac ctg gcc tcc gcg ctc acg gcc      1793
ttc gct ggt ctc tac gtg gca ctc gcg gtt gga gtc agc gag gag agc      1841
gag gcc tgg atc ctg gca gtg gcc acc ggc ctg ttc ctc tac gta gca      1889
ctc tgc gac atg ctc ccg gcg atg ttg aaa gta cgg gac ccg cgg ccc      1937
tgg ctc ctc ttc ctg ctg cac aac gtg ggc ctg ctg ggc ggc tgg acc      1985
gtc ctg ctg ctg ctg tcc ctg tac gag gat gac atc acc ttc tga          2030
taccctgccc tagtccccca cctttgactt aagatcccac acctcacaaa cctacagccc    2090
agaaaccaga agcccctata gaggccccag tcccaactcc agtaaagaca ctcttgtcct    2150
tggaaaaaaa aaaaaaaaaa aaaaaaaa                                        2178

<210>  34
<211>  647
<212>  PRT
<213>  Homo sapiens

<400>  34

<210>  35
<211>  3246

<212>  DNA
<213>  Homo sapiens

<220>
<221>  CDS
<222>  (478)..(1860)

<220>
<221>  misc_feature
<222>  (2530)..(2530)
<223>  n is a, c, g, or t

<400>  35
agtgtggttt tagtttttcc taagaagtgg cgtggtttgg ggctttatat ccgggaggag      60
catatgtacg caaatcctgg ggcgtttgca aacccggatc cggggcgtct ggccccatgc     120
ccggccgggc gtttgagggc tactgccacg cagcgtttct ggagcctgcc ggctggtgcc     180
ctggtggcct ttatctctgt ccccctttgt cctctttatc tcaggctctc caggaggccg     240
gggggcccac tccgcctatc gctcccctcg gctacgctgc cactccaatg ccccgcaggt     300
cgcgagctgc tgttctttcg aaggcgccgg agaaccaggg gcgtcccgcg ccacctctga     360
ctcggagcag cgccgagcac tgacgctccc gcccttgggc aaggacgcca gtgcgcccgc     420
gcgcgtccct ctgcgcggca gcccgtcgcg ggccctcaag gggaagccca ggccagg        477
atg gcc ccg ggt cgc gcg gtg gcc ggg ctc ctg ttg ctg gcg gcc gcc       525
ggc ctc gga gga gtg gcg gag ggg cca ggg cta gcc ttc agc gag gat       573
gtg ctg agc gtg ttc ggc gcg aat ctg agc ctg tcg gcg gcg cag ctc       621
cag cac ttg ctg gag cag atg gga gcc gcc tcc cgc gtg ggc gtc ccg       669
gag cct ggc cag ctg cac ttc aac cag tgt tta act gct gaa gag atc       717
ttt tcc ctt cat ggc ttt tca aat gct acc caa ata acc agc tcc aaa       765
ttc tct gtc atc tgt cca gca gtc tta cag caa ttg aac ttt cac cca       813
tgt gag gat cgg ccc aag cac aaa aca aga cca agt cat tca gaa gtt       861
tgg gga tat gga ttc ctg tca gtg acg att att aat ctg gca tct ctc       909
ctc gga ttg att ttg act cca ctg ata aag aaa tct tat ttc cca aag       957
att ttg acc ttt ttt gtg ggg ctg gct att ggg act ctt ttt tca aat 1005
gca att ttc caa ctt att cca gag gca ttt gga ttt gat ccc aaa gtc 1053
gac agt tat gtt gag aag gca gtt gct gtg ttt ggt gga ttt tac cta 1101
ctt ttc ttt ttt gaa aga atg cta aag atg tta tta aag aca tat ggt 1149
cag aat ggt cat acc cac ttt gga aat gat aac ttt ggt cct caa gaa 1197
aaa act cat caa cct aaa gca tta cct gcc atc aat ggt gtg aca tgc 1245
tat gca aat cct gct gtc aca gaa gct aat gga cat atc cat ttt gat 1293
aat gtc agt gtg gta tct cta cag gat gga aaa aaa gag cca agt tca 1341
tgt acc tgt ttg aag ggg ccc aaa ctg tca gaa ata ggg acg att gcc 1389
tgg atg ata acg ctc tgc gat gcc ctc cac aat ttc atc gat ggc ctg 1437
gcg att ggg gct tcc tgc acc ttg tct ctc ctt cag gga ctc agt act 1485
tcc ata gca atc cta tgt gag gag ttt ccc cac gag tta gga gac ttt 1533
gtg atc cta ctc aat gca ggg atg agc act cga caa gcc ttg cta ttc 1581
aac ttc ctt tct gca tgt tcc tgc tat gtt ggg cta gct ttt ggc att 1629
ttg gtg ggc aac aat ttc gct cca aat att ata ttt gca ctt gct gga 1677
ggc atg ttc ctc tat att tct ctg gca gat atg ttt cca gag atg aat 1725
gat atg ctg aga gaa aag gta act gga aga aaa acc gat ttc acc ttc 1773
ttc atg att cag aat gct gga atg tta act gga ttc aca gcc att cta     1821
ctc att acc ttg tat gca gga gaa atc gaa ttg gag taa tagaaaatgg     1870
aagatggtgt tgttaataaa ggcatttaat agataaaaac atctccaaaa aggattttga     1930
agctgatcct atttagttaa aaagataatt ttgctttcaa ctgtaggtcc agaaaactaa     1990
ttattggcat cagtctgtga aatagtccat tatttgttgt taaaaatgct tcaaaaggtt     2050
ttcagtgtca gtctgagatg cctggtatat aggagccttt gggaaatact tatttttcag     2110
tattccatgc atattagata tcaccatgaa gcaagagaca tgcattctat aatcatgtag     2170
acactcagac tcaggggaaa atacaagtta tatcctgaaa gcctttaaaa ctctatggta     2230
ggatcaaaga ttcaaatggt ttcagagagg ttttatttca attaatttgt tctagtgctt     2290
tcaagagcaa gtacatcaaa atgtagaagg taaaatgtat gcaacactaa tataaattat     2350
tccaagtctt taaggagcca aagaaaaaaa agatttctca cagctttttg ttctgttttg     2410
tatttcaatt aggaacttgc agtattattt tgaaaaccat tctaaaataa taggagttag     2470
gaaataaata aagttttgct agccctgcta agttcaggct tagaggctta tcgctaagtn     2530
taaacttcac cagattccac gaaaagctgg atagcttttt ttctgactta tgttgtggtt     2590
gcacccctca caaatggcag aacagtatgt aaagctggta acacctcggt ttcagtgcac     2650
catgtgtttg ctttgtgaag gtgaagaata tgttggttta gagaaagaaa ttggatgtaa     2710
ttttatgcaa tttactttta aagacaaaca taactattta gcagagaata ttttaataaa     2770
tgcaaaacaa cagctggact gctgtacatc aaggacagat taactggaaa acatatgttc     2830
cttatgtgtg attgagagcc attcagaaaa gacttccttt gtgttcagcc tatacttttc     2890
catatggtat accttgaaaa aaattagcac accatggtta tttttctacc ttttataaaa     2950
gacagagcct gtttactcat ttagaagata gagaaaattg gtctaaaatt gaacatccta     3010
gattcacact cccaagtcac ttaaggtgat ttgatggtga ggaaaatgat tgacaaagcc     3070
caacaatgat ctcaggaatt acattttcca acagaccaaa aaatgttttc atgtagcagc     3130
aatgcagatt tggtgaatat ttaatatata ttttagtatg tatttcactt tatgactgac     3190
aattaaaaaa tattgtttgg ccaaatagta aacacccttt tgaaaccatg aaaaaa     3246

<210>  36
<211>  460
<212>  PRT
<213>  Homo sapiens

<400>  36

<210>  37
<211>  1895
<212>  DNA
<213>  Homo sapiens

<220>
<221>  CDS
<222>  (106)..(1725)

<400>  37
gtctacagca aggcctaata ggggacctga gggcacagtc ctcaggatgt ttcggggaga      60

ataggagcca gaacctgagc ccctaagcca ttcccctcac caatg atg ggg tcc cca     117
                                                  Met Gly Ser Pro
                                                  1

gtg agt cat ctg ctg gcc ggc ttc tgt gtg tgg gtc gtc ttg ggc tgg       165
Val Ser His Leu Leu Ala Gly Phe Cys Val Trp Val Val Leu Gly Trp
5                   10                  15                  20

gta ggg ggc tca gtc ccc aac ctg ggc cct gct gag cag gag cag aac       213
Val Gly Gly Ser Val Pro Asn Leu Gly Pro Ala Glu Gln Glu Gln Asn
                25                  30                  35

cat tac ctg gcc cag ctg ttt ggc ctg tac ggc gag aat ggg acg ctg       261
His Tyr Leu Ala Gln Leu Phe Gly Leu Tyr Gly Glu Asn Gly Thr Leu
            40                  45                  50

act gca ggg ggc ttg gcg cgg ctt ctc cac agc ctg ggg cta ggc cga 309
Thr Ala Gly Gly Leu Ala Arg Leu Leu His Ser Leu Gly Leu Gly Arg
        55                  60                  65

gtt cag ggg ctt cgc ctg gga cag cat ggg cct ctg act gga cgg gct 357
Val Gln Gly Leu Arg Leu Gly Gln His Gly Pro Leu Thr Gly Arg Ala
    70                  75                  80

gca tcc cca gct gca gac aat tcc aca cac agg cca cag aac cct gag 405
Ala Ser Pro Ala Ala Asp Asn Ser Thr His Arg Pro Gln Asn Pro Glu
85                  90                  95                  100

ctg agt gtg gat gtc tgg gca ggg atg cct ctg ggt ccc tca ggg tgg 453
Leu Ser Val Asp Val Trp Ala Gly Met Pro Leu Gly Pro Ser Gly Trp
                105                 110                 115

ggt gac ctg gaa gag tca aag gcc cct cac cta ccc cgt ggg cca gcc 501
Gly Asp Leu Glu Glu Ser Lys Ala Pro His Leu Pro Arg Gly Pro Ala
            120                 125                 130

ccc tcg ggc ctg gac ctc ctt cac agg ctt ctg ttg ctg gac cac tca 549
Pro Ser Gly Leu Asp Leu Leu His Arg Leu Leu Leu Leu Asp His Ser
        135                 140                 145

ttg gct gac cac ctg aat gag gat tgt ctg aac ggc tcc cag ctg ctg 597
Leu Ala Asp His Leu Asn Glu Asp Cys Leu Asn Gly Ser Gln Leu Leu
    150                 155                 160

gtc aat ttt ggc ttg agc ccc gct gct cct ctg acc cct cgt cag ttt 645
Val Asn Phe Gly Leu Ser Pro Ala Ala Pro Leu Thr Pro Arg Gln Phe
165                 170                 175                 180

gct ctg ctg tgc cca gcc ctg ctt tat cag atc gac agc cgc gtc tgc 693
Ala Leu Leu Cys Pro Ala Leu Leu Tyr Gln Ile Asp Ser Arg Val Cys
                185                 190                 195

atc ggc gct ccg gcc cct gca ccc cca ggg gat cta cta tct gcc ctg 741
Ile Gly Ala Pro Ala Pro Ala Pro Pro Gly Asp Leu Leu Ser Ala Leu
            200                 205                 210

ctt cag agt gcc ctg gca gtc ctg ttg ctc agc ctc cct tct ccc cta 789
Leu Gln Ser Ala Leu Ala Val Leu Leu Leu Ser Leu Pro Ser Pro Leu
        215                 220                 225

tcc ctg ctg ctg ctg cgg ctc ctg gga cct cgt cta cta cgg ccc ttg 837
Ser Leu Leu Leu Leu Arg Leu Leu Gly Pro Arg Leu Leu Arg Pro Leu
    230                 235                 240

ctg ggc ttc ctg ggg gcc ctg gcg gtg ggc act ctt tgt ggg gat gca 885
Leu Gly Phe Leu Gly Ala Leu Ala Val Gly Thr Leu Cys Gly Asp Ala
245                 250                 255                 260

ctg cta cat ctg cta ccg cat gca caa gaa ggg cgg cac gca gga cct 933
Leu Leu His Leu Leu Pro His Ala Gln Glu Gly Arg His Ala Gly Pro
                265                 270                 275

ggc gga cta cca gag aag gac ctg ggc ccg ggg ctg tca gtg ctc gga 981
Gly Gly Leu Pro Glu Lys Asp Leu Gly Pro Gly Leu Ser Val Leu Gly
            280                 285                 290

ggc ctc ttc ctg ctc ttt gtg ctg gag aac atg ctg ggg ctt ttg cgg 1029
Gly Leu Phe Leu Leu Phe Val Leu Glu Asn Met Leu Gly Leu Leu Arg
        295                 300                 305

cac cga ggg ctc agg cca aga tgc tgc agg cga aaa cga agg aat ctc 1077
His Arg Gly Leu Arg Pro Arg Cys Cys Arg Arg Lys Arg Arg Asn Leu
    310                 315                 320

gaa aca cgc aac ttg gat ccg gag aat ggc agt ggg atg gcc ctt cag 1125

Glu Thr Arg Asn Leu Asp Pro Glu Asn Gly Ser Gly Met Ala Leu Gln
325                 330                 335                 340

ccc cta cag gca gct cca gag cca ggg gct cag ggc cag agg gag aag      1173
Pro Leu Gln Ala Ala Pro Glu Pro Gly Ala Gln Gly Gln Arg Glu Lys
                345                 350                 355

aac agc cag cac cca cca gct ctg gcc cct cct ggg cac caa ggc cac      1221
Asn Ser Gln His Pro Pro Ala Leu Ala Pro Pro Gly His Gln Gly His
            360                 365                 370

agt cat ggg cac cag ggt ggc act gat atc acg tgg atg gtc ctc ctg      1269
Ser His Gly His Gln Gly Gly Thr Asp Ile Thr Trp Met Val Leu Leu
        375                 380                 385

gga gat ggt cta cac aac ctc act gat ggg ctg gcc ata ggt gct gcc      1317
Gly Asp Gly Leu His Asn Leu Thr Asp Gly Leu Ala Ile Gly Ala Ala
    390                 395                 400

ttc tct gat ggc ttc tcc agc ggc ctc agt acc acc tta gcg gtc ttc      1365
Phe Ser Asp Gly Phe Ser Ser Gly Leu Ser Thr Thr Leu Ala Val Phe
405                 410                 415                 420

tgc cat gag ctg ccc cac gaa ctg ggt gac ttt gcc atg ctg ctc cag      1413
Cys His Glu Leu Pro His Glu Leu Gly Asp Phe Ala Met Leu Leu Gln
                425                 430                 435

tca ggg ctg tcc ttt cgg cgg ctg ctg ctg ctg agc ctc gtg tct gga      1461
Ser Gly Leu Ser Phe Arg Arg Leu Leu Leu Leu Ser Leu Val Ser Gly
            440                 445                 450

gcc ctg gga ttg ggg ggt gca gtc ctg ggg gtg ggg ctc agc ctg ggc      1509
Ala Leu Gly Leu Gly Gly Ala Val Leu Gly Val Gly Leu Ser Leu Gly
        455                 460                 465

cct gtc ccc ctc act ccc tgg gtg ttt ggg gtc act gct ggg gtc ttc      1557
Pro Val Pro Leu Thr Pro Trp Val Phe Gly Val Thr Ala Gly Val Phe
    470                 475                 480

ctc tat gtg gcc ctt gtg gac atg cta cca gcc ctg ctt cgt cct ccg      1605
Leu Tyr Val Ala Leu Val Asp Met Leu Pro Ala Leu Leu Arg Pro Pro
485                 490                 495                 500

gag ccc ctg cct acg ccc cat gtg ctc ctg cag ggg ctg ggg ctg ctg      1653
Glu Pro Leu Pro Thr Pro His Val Leu Leu Gln Gly Leu Gly Leu Leu
                505                 510                 515

ctg ggg ggc ggc ctc atg ctt gcc ata acc ctg ctg gag gag cgg cta      1701
Leu Gly Gly Gly Leu Met Leu Ala Ile Thr Leu Leu Glu Glu Arg Leu
            520                 525                 530

ctg ccc gtg acc act gag ggc tga tggggccagt ggaaaggggt cgggttgccc      1755
Leu Pro Val Thr Thr Glu Gly
        535

ttccttcccc ccaaccacag gaatggaggc gggacacagg gccagtagga gcaataggat      1815

tttaataaac agaacccatc ccaaaaaaaa aaaaaaaaaa aaaaaaaaaa aaaaaaaaaa      1875

aaaaaaaaaa aaaaaaaaaa                                                  1895

<210>  38
<211>  539
<212>  PRT
<213>  Homo sapiens

<400>  38
   Met Gly Ser Pro Val Ser His Leu Leu Ala Gly Phe Cys Val Trp Val
   1               5                   10                  15
   Val Leu Gly Trp Val Gly Gly Ser Val Pro Asn Leu Gly Pro Ala Glu
               20                  25                  30
   Gln Glu Gln Asn His Tyr Leu Ala Gln Leu Phe Gly Leu Tyr Gly Glu
           35                  40                  45
   Asn Gly Thr Leu Thr Ala Gly Gly Leu Ala Arg Leu Leu His Ser Leu
       50                  55                  60
   Gly Leu Gly Arg Val Gln Gly Leu Arg Leu Gly Gln His Gly Pro Leu
   65                  70                  75                  80
   Thr Gly Arg Ala Ala Ser Pro Ala Ala Asp Asn Ser Thr His Arg Pro
                   85                  90                  95
   Gln Asn Pro Glu Leu Ser Val Asp Val Trp Ala Gly Met Pro Leu Gly
               100                 105                 110
   Pro Ser Gly Trp Gly Asp Leu Glu Glu Ser Lys Ala Pro His Leu Pro
           115                 120                 125
   Arg Gly Pro Ala Pro Ser Gly Leu Asp Leu Leu His Arg Leu Leu Leu
       130                 135                 140
   Leu Asp His Ser Leu Ala Asp His Leu Asn Glu Asp Cys Leu Asn Gly
   145                 150                 155                 160
   Ser Gln Leu Leu Val Asn Phe Gly Leu Ser Pro Ala Ala Pro Leu Thr
                   165                 170                 175
   Pro Arg Gln Phe Ala Leu Leu Cys Pro Ala Leu Leu Tyr Gln Ile Asp
               180                 185                 190
   Ser Arg Val Cys Ile Gly Ala Pro Ala Pro Ala Pro Pro Gly Asp Leu
           195                 200                 205
   Leu Ser Ala Leu Leu Gln Ser Ala Leu Ala Val Leu Leu Leu Ser Leu
       210                 215                 220
   Pro Ser Pro Leu Ser Leu Leu Leu Leu Arg Leu Leu Gly Pro Arg Leu
   225                 230                 235                 240
   Leu Arg Pro Leu Leu Gly Phe Leu Gly Ala Leu Ala Val Gly Thr Leu
                   245                 250                 255
   Cys Gly Asp Ala Leu Leu His Leu Leu Pro His Ala Gln Glu Gly Arg
               260                 265                 270
   His Ala Gly Pro Gly Gly Leu Pro Glu Lys Asp Leu Gly Pro Gly Leu
   275 280 285
   Ser Val Leu Gly Gly Leu Phe Leu Leu Phe Val Leu Glu Asn Met Leu
       290                 295                 300
   Gly Leu Leu Arg His Arg Gly Leu Arg Pro Arg Cys Cys Arg Arg Lys
   305                 310                 315                 320
   Arg Arg Asn Leu Glu Thr Arg Asn Leu Asp Pro Glu Asn Gly Ser Gly
                   325                 330                 335
   Met Ala Leu Gln Pro Leu Gln Ala Ala Pro Glu Pro Gly Ala Gln Gly
               340                 345                 350
   Gln Arg Glu Lys Asn Ser Gln His Pro Pro Ala Leu Ala Pro Pro Gly
           355                 360                 365
   His Gln Gly His Ser His Gly His Gln Gly Gly Thr Asp Ile Thr Trp
       370                 375                 380
   Met Val Leu Leu Gly Asp Gly Leu His Asn Leu Thr Asp Gly Leu Ala
   385                 390                 395                 400
   Ile Gly Ala Ala Phe Ser Asp Gly Phe Ser Ser Gly Leu Ser Thr Thr
                   405                 410                 415
   Leu Ala Val Phe Cys His Glu Leu Pro His Glu Leu Gly Asp Phe Ala
               420                 425                 430
   Met Leu Leu Gln Ser Gly Leu Ser Phe Arg Arg Leu Leu Leu Leu Ser
           435                 440                 445
   Leu Val Ser Gly Ala Leu Gly Leu Gly Gly Ala Val Leu Gly Val Gly
       450                 455                 460
   Leu Ser Leu Gly Pro Val Pro Leu Thr Pro Trp Val Phe Gly Val Thr
   465                 470                 475                 480
   Ala Gly Val Phe Leu Tyr Val Ala Leu Val Asp Met Leu Pro Ala Leu
                   485                 490                 495
   Leu Arg Pro Pro Glu Pro Leu Pro Thr Pro His Val Leu Leu Gln Gly
               500                 505                 510
   Leu Gly Leu Leu Leu Gly Gly Gly Leu Met Leu Ala Ile Thr Leu Leu
           515                 520                 525
   Glu Glu Arg Leu Leu Pro Val Thr Thr Glu Gly
       530                 535

<210> 39
<211> 2636

<212>  DNA
<213>  Homo sapiens

<220>
<221>  CDS
<222>  (222)..(2186)

<400>  39
aaaataatcc tctctagctc ccagtgcaca gtcaaagaat tttaaaaaca gggaactttg      60

taactgtgaa atactctcca ggatttaaaa ggctgtggag ctccagataa agaatcgttt     120

atctttcttc tgaagaaatt cctttggtta caagtttacc ccataaacgg caacacactc     180

acctccatcc aagacagact caaggtggag gaagcgtgga a atg tgc ttc cgg aca     236
                                              Met Cys Phe Arg Thr
                                              1               5

aag ctc tca gta tcc tgg gtg cca ttg ttt ctt cta ctc agc cgt gtt       284
Lys Leu Ser Val Ser Trp Val Pro Leu Phe Leu Leu Leu Ser Arg Val
                10                  15                  20

ttt tct act gag aca gac aaa ccc tca gcc cag gac agc aga agc cgt       332
Phe Ser Thr Glu Thr Asp Lys Pro Ser Ala Gln Asp Ser Arg Ser Arg
            25                  30                  35

ggg agt tca ggc caa ccg gca gac ctg cta cag gtt ctc tct gct ggt       380
Gly Ser Ser Gly Gln Pro Ala Asp Leu Leu Gln Val Leu Ser Ala Gly
        40                  45                  50

gac cac cca ccc cac aac cac tca aga agc ctc atc aaa aca ttg ttg       428
Asp His Pro Pro His Asn His Ser Arg Ser Leu Ile Lys Thr Leu Leu
    55                  60                  65

gag aaa act ggg tgc cca cgg agg aga aac gga atg caa gga gat tgc       476
Glu Lys Thr Gly Cys Pro Arg Arg Arg Asn Gly Met Gln Gly Asp Cys
70                  75                  80                  85

aat ctg tgc ttt gaa cca gat gca cta tta cta ata gct gga gga aat       524
Asn Leu Cys Phe Glu Pro Asp Ala Leu Leu Leu Ile Ala Gly Gly Asn
                90                  95                  100

ttt gaa gat cag ctt aga gaa gaa gtg gtc cag aga gtt tct ctt ctc       572
Phe Glu Asp Gln Leu Arg Glu Glu Val Val Gln Arg Val Ser Leu Leu
            105                 110                 115

ctt ctc tat tac att att cat cag gaa gag atc tgt tct tca aag ctc       620
Leu Leu Tyr Tyr Ile Ile His Gln Glu Glu Ile Cys Ser Ser Lys Leu
        120                 125                 130

aac atg agt aat aaa gag tat aaa ttt tac cta cac agc cta ctg agc       668
Asn Met Ser Asn Lys Glu Tyr Lys Phe Tyr Leu His Ser Leu Leu Ser
    135                 140                 145

ctc agg cag gat gaa gat tcc tct ttc ctt tca cag aat gag aca gaa       716
Leu Arg Gln Asp Glu Asp Ser Ser Phe Leu Ser Gln Asn Glu Thr Glu
150                 155                 160                 165

gat atc ttg gct ttc acc agg cag tac ttt gac act tct caa agc cag       764
Asp Ile Leu Ala Phe Thr Arg Gln Tyr Phe Asp Thr Ser Gln Ser Gln
                170                 175                 180

tgt atg gaa acc aaa acg ctg cag aaa aaa tct gga ata gtg agc agt       812
Cys Met Glu Thr Lys Thr Leu Gln Lys Lys Ser Gly Ile Val Ser Ser
            185                 190                 195

gaa ggt gct aat gaa agt acg ctt cct cag ttg gca gcc atg atc att       860
Glu Gly Ala Asn Glu Ser Thr Leu Pro Gln Leu Ala Ala Met Ile Ile

200                 205                 210

act ttg tcc ctc cag ggt gtt tgt ctg gga caa gga aac ttg cct tcc     908
Thr Leu Ser Leu Gln Gly Val Cys Leu Gly Gln Gly Asn Leu Pro Ser
    215                 220                 225

cca gac tac ttt aca gaa tat att ttc agt tcc ttg aat cgt acg aat     956
Pro Asp Tyr Phe Thr Glu Tyr Ile Phe Ser Ser Leu Asn Arg Thr Asn
230                 235                 240                 245

acc ctc cgc cta tca gaa cta gac caa ctc ctc aac act ctc tgg acc    1004
Thr Leu Arg Leu Ser Glu Leu Asp Gln Leu Leu Asn Thr Leu Trp Thr
                250                 255                 260

aga agt act tgt atc aaa aat gag aaa atc cat caa ttt caa agg aaa    1052
Arg Ser Thr Cys Ile Lys Asn Glu Lys Ile His Gln Phe Gln Arg Lys
            265                 270                 275

caa aac aac ata ata acc cat gat cag ggc tat tct aat ttc tct tca    1100
Gln Asn Asn Ile Ile Thr His Asp Gln Gly Tyr Ser Asn Phe Ser Ser
        280                 285                 290

tcc atg gaa aaa gag tct gag gat ggt cca gtt tcc tgg gat cag acc    1148
Ser Met Glu Lys Glu Ser Glu Asp Gly Pro Val Ser Trp Asp Gln Thr
    295                 300                 305

tgc ttc tct gct agg cag ctg gtg gag ata ttt cta cag aag ggc ctc    1196
Cys Phe Ser Ala Arg Gln Leu Val Glu Ile Phe Leu Gln Lys Gly Leu
310                 315                 320                 325

tca ctc att tct aag gag gac ttt aag caa atg agt cca ggg atc atc    1244
Ser Leu Ile Ser Lys Glu Asp Phe Lys Gln Met Ser Pro Gly Ile Ile
                330                 335                 340

cag cag ctc ctc agc tgc tcc tgc cac tta ccc aag gac caa caa gca    1292
Gln Gln Leu Leu Ser Cys Ser Cys His Leu Pro Lys Asp Gln Gln Ala
            345                 350                 355

aag ctg cca cct acc act ctg gag aaa tac ggc tac agc acg gtg gct    1340
Lys Leu Pro Pro Thr Thr Leu Glu Lys Tyr Gly Tyr Ser Thr Val Ala
        360                 365                 370

gtc acc ctt ctc aca ctg ggc tcc atg ctg ggg aca gcg ctg gtc ctt    1388
Val Thr Leu Leu Thr Leu Gly Ser Met Leu Gly Thr Ala Leu Val Leu
    375                 380                 385

ttc cat agc tgt gag gag aac tac agg ctt atc tta cag ctg ttt gtg    1436
Phe His Ser Cys Glu Glu Asn Tyr Arg Leu Ile Leu Gln Leu Phe Val
390                 395                 400                 405

ggc ttg gcc gtc ggg aca ctg tct ggg gac gct ctg ctc cac ctt atc    1484
Gly Leu Ala Val Gly Thr Leu Ser Gly Asp Ala Leu Leu His Leu Ile
                410                 415                 420

cct cag gtt ctt ggt tta cat aag cag gaa gcc cca gaa ttt ggg cat    1532
Pro Gln Val Leu Gly Leu His Lys Gln Glu Ala Pro Glu Phe Gly His
            425                 430                 435

ttc cat gaa agc aaa ggt cat att tgg aaa ctg atg gga tta att gga    1580
Phe His Glu Ser Lys Gly His Ile Trp Lys Leu Met Gly Leu Ile Gly
        440                 445                 450

ggc atc cat gga ttt ttc ttg ata gaa aaa tgt ttt att ctt ctt gta    1628
Gly Ile His Gly Phe Phe Leu Ile Glu Lys Cys Phe Ile Leu Leu Val
    455                 460                 465

tca cca aat gac aag aaa agc cca gaa gat tca cag gca gct gaa atg    1676
Ser Pro Asn Asp Lys Lys Ser Pro Glu Asp Ser Gln Ala Ala Glu Met
470                 475                 480                 485

cct ata ggc agt atg aca gcc tcc aac aga aaa tgt aaa gcc att agc     1724
Pro Ile Gly Ser Met Thr Ala Ser Asn Arg Lys Cys Lys Ala Ile Ser
                490                 495                 500

ttg tta gca atc atg att ctg gtt ggg gac agc ctg cat aat ttt gca     1772
Leu Leu Ala Ile Met Ile Leu Val Gly Asp Ser Leu His Asn Phe Ala
            505                 510                 515

gat ggc cta gcc ata gga gca gcc ttc tca tca tca tcc gag tca gga     1820
Asp Gly Leu Ala Ile Gly Ala Ala Phe Ser Ser Ser Ser Glu Ser Gly
        520                 525                 530

gtg acc act acg att gct atc ttg tgt cat gaa atc cca cat gaa atg     1868
Val Thr Thr Thr Ile Ala Ile Leu Cys His Glu Ile Pro His Glu Met
    535                 540                 545

gga gac ttt gcc gtg ctc tta agc tct gga ctt tct atg aag act gcc     1916
Gly Asp Phe Ala Val Leu Leu Ser Ser Gly Leu Ser Met Lys Thr Ala
550                 555                 560                 565

atc ctg atg aat ttt ata agc tcc cta act gcc ttc atg gga tta tac     1964
Ile Leu Met Asn Phe Ile Ser Ser Leu Thr Ala Phe Met Gly Leu Tyr
                570                 575                 580

att ggc ctt tcc gtg tca gct gat cca tgt gtt caa gac tgg atc ttc     2012
Ile Gly Leu Ser Val Ser Ala Asp Pro Cys Val Gln Asp Trp Ile Phe
            585                 590                 595

aca gtc act gct ggg atg ttc tta tat tta tcc ttg gtt gaa atg ctt     2060
Thr Val Thr Ala Gly Met Phe Leu Tyr Leu Ser Leu Val Glu Met Leu
        600                 605                 610

cct gaa atg act cat gtt caa aca caa cga ccc tgg atg atg ttt ctc     2108
Pro Glu Met Thr His Val Gln Thr Gln Arg Pro Trp Met Met Phe Leu
    615                 620                 625

ctg caa aac ttt gga ttg atc cta ggt tgg ctt tct ctc ctg ctc ttg     2156
Leu Gln Asn Phe Gly Leu Ile Leu Gly Trp Leu Ser Leu Leu Leu Leu
630                 635                 640                 645

gct ata tat gag caa aat att aaa ata taa gtgaggatct tcaacatctt     2206
Ala Ile Tyr Glu Gln Asn Ile Lys Ile
                650

tcaaaaatgc atttatatag tcttactttg tttctttcat tgcactctat aatgattttt     2266

aaattaagaa ttttttatct taggcaaagt gtgtctcttt caattcatta acttattaat     2326

tttataatgc agttttattt ttggaaacat ataaatatca gactgtcctt aattgaaatt     2386

ttgtctttgg tttccaacac catgatgaag ctcttgcttt ttaaaaagta gttagtaaat     2446

tctgcatgaa ttttagtaaa ctttaaaaaa tagatttttt ccctaagaaa gaatgtttgt     2506

agaatttaaa gtggacagat gcctgttggg gtaaaatcaa ctgcaacttt ttgatgttaa     2566

tttttttccc tgtgcaatta taaactataa gcaagttaag tgacaagcaa atgtaataaa     2626

gactagtttt                                                          2636

<210>  40
<211>  654
<212>  PRT
<213>  Homo sapiens

<400>  40

Met Cys Phe Arg Thr Lys Leu Ser Val Ser Trp Val Pro Leu Phe Leu
1               5                   10                  15

Leu Leu Ser Arg Val Phe Ser Thr Glu Thr Asp Lys Pro Ser Ala Gln
            20                  25                  30

Asp Ser Arg Ser Arg Gly Ser Ser Gly Gln Pro Ala Asp Leu Leu Gln
        35                  40                  45

Val Leu Ser Ala Gly Asp His Pro Pro His Asn His Ser Arg Ser Leu
    50                  55                  60

Ile Lys Thr Leu Leu Glu Lys Thr Gly Cys Pro Arg Arg Arg Asn Gly
65                  70                  75                  80

Met Gln Gly Asp Cys Asn Leu Cys Phe Glu Pro Asp Ala Leu Leu Leu
                85                  90                  95

Ile Ala Gly Gly Asn Phe Glu Asp Gln Leu Arg Glu Glu Val Val Gln
            100                 105                 110

Arg Val Ser Leu Leu Leu Leu Tyr Tyr Ile Ile His Gln Glu Glu Ile
        115                 120                 125

Cys Ser Ser Lys Leu Asn Met Ser Asn Lys Glu Tyr Lys Phe Tyr Leu
    130                 135                 140

His Ser Leu Leu Ser Leu Arg Gln Asp Glu Asp Ser Ser Phe Leu Ser
145                 150                 155                 160

Gln Asn Glu Thr Glu Asp Ile Leu Ala Phe Thr Arg Gln Tyr Phe Asp
                165                 170                 175

Thr Ser Gln Ser Gln Cys Met Glu Thr Lys Thr Leu Gln Lys Lys Ser
            180                 185                 190

Gly Ile Val Ser Ser Glu Gly Ala Asn Glu Ser Thr Leu Pro Gln Leu
        195                 200                 205

Ala Ala Met Ile Ile Thr Leu Ser Leu Gln Gly Val Cys Leu Gly Gln
    210                 215                 220

Gly Asn Leu Pro Ser Pro Asp Tyr Phe Thr Glu Tyr Ile Phe Ser Ser
225                 230                 235                 240

Leu Asn Arg Thr Asn Thr Leu Arg Leu Ser Glu Leu Asp Gln Leu Leu
                245                 250                 255

Asn Thr Leu Trp Thr Arg Ser Thr Cys Ile Lys Asn Glu Lys Ile His
            260                 265                 270

Gln Phe Gln Arg Lys Gln Asn Asn Ile Ile Thr His Asp Gln Gly Tyr

        275                 280                 285

Ser Asn Phe Ser Ser Ser Met Glu Lys Glu Ser Glu Asp Gly Pro Val
    290                 295                 300

Ser Trp Asp Gln Thr Cys Phe Ser Ala Arg Gln Leu Val Glu Ile Phe
305                 310                 315                 320

Leu Gln Lys Gly Leu Ser Leu Ile Ser Lys Glu Asp Phe Lys Gln Met
                325                 330                 335

Ser Pro Gly Ile Ile Gln Gln Leu Leu Ser Cys Ser Cys His Leu Pro
            340                 345                 350

Lys Asp Gln Gln Ala Lys Leu Pro Pro Thr Thr Leu Glu Lys Tyr Gly
        355                 360                 365

Tyr Ser Thr Val Ala Val Thr Leu Leu Thr Leu Gly Ser Met Leu Gly
    370                 375                 380

Thr Ala Leu Val Leu Phe His Ser Cys Glu Glu Asn Tyr Arg Leu Ile
385                 390                 395                 400

Leu Gln Leu Phe Val Gly Leu Ala Val Gly Thr Leu Ser Gly Asp Ala
                405                 410                 415

Leu Leu His Leu Ile Pro Gln Val Leu Gly Leu His Lys Gln Glu Ala
            420                 425                 430

Pro Glu Phe Gly His Phe His Glu Ser Lys Gly His Ile Trp Lys Leu
        435                 440                 445

Met Gly Leu Ile Gly Gly Ile His Gly Phe Phe Leu Ile Glu Lys Cys
    450                 455                 460

Phe Ile Leu Leu Val Ser Pro Asn Asp Lys Lys Ser Pro Glu Asp Ser
465                 470                 475                 480

Gln Ala Ala Glu Met Pro Ile Gly Ser Met Thr Ala Ser Asn Arg Lys
                485                 490                 495

Cys Lys Ala Ile Ser Leu Leu Ala Ile Met Ile Leu Val Gly Asp Ser
            500                 505                 510

Leu His Asn Phe Ala Asp Gly Leu Ala Ile Gly Ala Ala Phe Ser Ser
        515                 520                 525

Ser Ser Glu Ser Gly Val Thr Thr Thr Ile Ala Ile Leu Cys His Glu
    530                 535                 540

Ile Pro His Glu Met Gly Asp Phe Ala Val Leu Leu Ser Ser Gly Leu
545                 550                 555                 560

Ser Met Lys Thr Ala Ile Leu Met Asn Phe Ile Ser Ser Leu Thr Ala
                565                 570                 575

Phe Met Gly Leu Tyr Ile Gly Leu Ser Val Ser Ala Asp Pro Cys Val
            580                 585                 590

Gln Asp Trp Ile Phe Thr Val Thr Ala Gly Met Phe Leu Tyr Leu Ser
        595                 600                 605

Leu Val Glu Met Leu Pro Glu Met Thr His Val Gln Thr Gln Arg Pro
    610                 615                 620

Trp Met Met Phe Leu Leu Gln Asn Phe Gly Leu Ile Leu Gly Trp Leu
625                 630                 635                 640

Ser Leu Leu Leu Leu Ala Ile Tyr Glu Gln Asn Ile Lys Ile
                645                 650

<210>  41
<211>  2309
<212>  DNA
<213>  Homo sapiens

<220>
<221>  CDS
<222>  (43)..(1158)

<400>  41
gggccgcccg gatcgccgcc gccgccgccg cacgtacgtg gc atg cct gga tgt        54
                                               Met Pro Gly Cys
                                               1

ccc tgc cct ggc tgt ggc atg gcg ggc cca agg ctc ctc ttc ctc act      102
Pro Cys Pro Gly Cys Gly Met Ala Gly Pro Arg Leu Leu Phe Leu Thr
5                   10                  15                  20

gcc ctt gcc ctg gag ctc ttg gga agg gct ggg ggt tcc cag ccg gcc      150
Ala Leu Ala Leu Glu Leu Leu Gly Arg Ala Gly Gly Ser Gln Pro Ala
                25                  30                  35

ctc cgg agc cgg ggg act gcg acg gcc tgt cgc ctg gac aac aag gaa      198
Leu Arg Ser Arg Gly Thr Ala Thr Ala Cys Arg Leu Asp Asn Lys Glu
            40                  45                  50

agc gag tcc tgg ggg gct ctg ctg agc gga gag cgg ctg gac acc tgg      246
Ser Glu Ser Trp Gly Ala Leu Leu Ser Gly Glu Arg Leu Asp Thr Trp
        55                  60                  65

atc tgc tcc ctc ctg ggt tcc ctc atg gtg ggg ctc agt ggg gtc ttc      294
Ile Cys Ser Leu Leu Gly Ser Leu Met Val Gly Leu Ser Gly Val Phe
    70                  75                  80

ccg ttg ctt gtc att ccc cta gag atg ggg acc atg ctg cgc tca gaa      342
Pro Leu Leu Val Ile Pro Leu Glu Met Gly Thr Met Leu Arg Ser Glu
85                  90                  95                  100

gct ggg gcc tgg cgc ctg aag cag ctg ctc agc ttc gcc ctg ggg gga      390
Ala Gly Ala Trp Arg Leu Lys Gln Leu Leu Ser Phe Ala Leu Gly Gly
                105                 110                 115

ctc ttg ggc aat gtg ttt ctg cat ctg ctg ccc gaa gcc tgg gcc tac 438
Leu Leu Gly Asn Val Phe Leu His Leu Leu Pro Glu Ala Trp Ala Tyr
            120                 125                 130

acg tgc agc gcc agc cct ggt ggt gag ggg cag agc ctg cag cag cag 486
Thr Cys Ser Ala Ser Pro Gly Gly Glu Gly Gln Ser Leu Gln Gln Gln
        135                 140                 145

caa cag ctg ggg ctg tgg gtc att gct ggc atc ctg acc ttc ctg gcg 534
Gln Gln Leu Gly Leu Trp Val Ile Ala Gly Ile Leu Thr Phe Leu Ala
    150                 155                 160

ttg gag aag atg ttc ctg gac agc aag gag gag ggg acc agc cag gcc 582
Leu Glu Lys Met Phe Leu Asp Ser Lys Glu Glu Gly Thr Ser Gln Ala
165                 170                 175                 180

ccc aac aaa gac ccc act gct gct gcc gcc gca ctc aat gga ggc cac 630
Pro Asn Lys Asp Pro Thr Ala Ala Ala Ala Ala Leu Asn Gly Gly His
                185                 190                 195

tgt ctg gcc cag ccg gct gca gag ccc ggc ctc ggt gcc gtg gtc cgg 678
Cys Leu Ala Gln Pro Ala Ala Glu Pro Gly Leu Gly Ala Val Val Arg
            200                 205                 210

agc atc aaa gtc agc ggc tac ctc aac ctg ctg gcc aac acc atc gat 726
Ser Ile Lys Val Ser Gly Tyr Leu Asn Leu Leu Ala Asn Thr Ile Asp
        215                 220                 225

aac ttc acc cac ggg ctg gct gtg gct gcc agc ttc ctt gtg agc aag 774
Asn Phe Thr His Gly Leu Ala Val Ala Ala Ser Phe Leu Val Ser Lys
    230                 235                 240

aag atc ggg ctc ctg aca acc atg gcc atc ctc ctg cat gag atc ccc 822
Lys Ile Gly Leu Leu Thr Thr Met Ala Ile Leu Leu His Glu Ile Pro
245                 250                 255                 260

cat gag gtg ggc gac ttt gcc atc ctg ctc cgg gcc ggc ttt gac cga 870
His Glu Val Gly Asp Phe Ala Ile Leu Leu Arg Ala Gly Phe Asp Arg
                265                 270                 275

tgg agc gca gcc aag ctg caa ctc tca aca gcg ctg ggg ggc cta ctg 918
Trp Ser Ala Ala Lys Leu Gln Leu Ser Thr Ala Leu Gly Gly Leu Leu
            280                 285                 290

ggc gct ggc ttc gcc atc tgt acc cag tcc ccc aag gga gta gtt ggg 966
Gly Ala Gly Phe Ala Ile Cys Thr Gln Ser Pro Lys Gly Val Val Gly
        295                 300                 305

tgt tct ccc gct gca gag gag acg gca gcc tgg gtc ctg ccc ttc acc 1014
Cys Ser Pro Ala Ala Glu Glu Thr Ala Ala Trp Val Leu Pro Phe Thr
    310                 315                 320

tct ggc ggc ttt ctc tac atc gcc ttg gtg aac gtg ctc cct gac ctc 1062
Ser Gly Gly Phe Leu Tyr Ile Ala Leu Val Asn Val Leu Pro Asp Leu
325                 330                 335                 340

ttg gaa gaa gag gac ccg tgg cgc tcc ctg cag cag ctg ctt ctg ctc 1110
Leu Glu Glu Glu Asp Pro Trp Arg Ser Leu Gln Gln Leu Leu Leu Leu
                345                 350                 355

tgt gcg ggc atc gtg gta atg gtg ctg ttc tcg ctc ttc gtg gat taa 1158
Cys Ala Gly Ile Val Val Met Val Leu Phe Ser Leu Phe Val Asp
            360                 365                 370

ctttccctga tgccgacgcc cctgccccct gcagcaataa gatgctcgga ttcactctgt 1218

gaccgcatat gtgagaggca gagagggcga gtggctgcga gagagaatga gcctcccgcc 1278

agacaggagg gaggtgcgtg tggatgtatg tggtgtgcac atgtggccag aggtgtgtgc 1338

gcgagaccga cactgtgatc cctgtgctgg gtccggggcc cagtgtagcg cctgtcccca 1398

gccatgctgt ggttacctct ccttgccgcc ctgtcacctt cacctcctgg agtaagcagc 1458

gaggaagagc agcactggtc ccaagcagag gccttgccct gctgggaccc cgggagtgag 1518

agcagcccaa ggatcccagg gtgcagggaa ctccagagct gcccacctcc cactgccccc 1578

tcagcacaca cacagtcccc aggcggccta ggggccaagg ctggggcggc tttggtccct 1638

tttcctggcc cttccttccc cacttctaag ccaaagaaag gagaggcagg tgctcctgta 1698

ccccagcccc actcagcact gacagtcccc agctcctagt agtgagctgg gaggcgcttc 1758

ctaagaccct ttcctcaggg ctgccctggg agctcattcc tggccaacac gccctggcag 1818

caccagcagc tcttgccacc tccagctgcc aaacagcagc ctgccgggca gggagcagcc 1878

ccaggccaga gaggcctccc ggtccagctc agggatgctc ctgccagcac aggggccagg 1938

gactcctgga gcaggcacat agtgagcccg ggcagccctg cccagctcag gcccctttcc 1998

ttccccattg aggttggggt aggtgggggc ggtgagggct ccacgttgtc agcgctcagg 2058

aatgtgctcc ggcagagtgc tgaagccata atccccaacc atttcccttg gctgacgccc 2118

aggtactcag ctggcccact ccacagccag gcctggccct gcccttcacc gtggatgttt 2178

tcagaagtgg ccatcgagag gtctggatgg ttttatagca actttgctgt gattccgttt 2238

gtatctgtaa atatttgttc tatagataag atacaaataa atattatcca cataaaaaaa 2298

aaaaaaaaaa a 2309

<210> 42
<211> 371
<212> PRT
<213> Homo sapiens

<400> 42

Met Pro Gly Cys Pro Cys Pro Gly Cys Gly Met Ala Gly Pro Arg Leu
1               5                   10                  15

Leu Phe Leu Thr Ala Leu Ala Leu Glu Leu Leu Gly Arg Ala Gly Gly
            20                  25                  30

Ser Gln Pro Ala Leu Arg Ser Arg Gly Thr Ala Thr Ala Cys Arg Leu
        35                  40                  45

Asp Asn Lys Glu Ser Glu Ser Trp Gly Ala Leu Leu Ser Gly Glu Arg
    50                  55                  60

Leu Asp Thr Trp Ile Cys Ser Leu Leu Gly Ser Leu Met Val Gly Leu
65                  70                  75                  80

Ser Gly Val Phe Pro Leu Leu Val Ile Pro Leu Glu Met Gly Thr Met
                85                  90                  95

Leu Arg Ser Glu Ala Gly Ala Trp Arg Leu Lys Gln Leu Leu Ser Phe
            100                 105                 110

Ala Leu Gly Gly Leu Leu Gly Asn Val Phe Leu His Leu Leu Pro Glu
        115                 120                 125

Ala Trp Ala Tyr Thr Cys Ser Ala Ser Pro Gly Gly Glu Gly Gln Ser
    130                 135                 140

Leu Gln Gln Gln Gln Gln Leu Gly Leu Trp Val Ile Ala Gly Ile Leu
145                 150                 155                 160

Thr Phe Leu Ala Leu Glu Lys Met Phe Leu Asp Ser Lys Glu Glu Gly
                165                 170                 175

Thr Ser Gln Ala Pro Asn Lys Asp Pro Thr Ala Ala Ala Ala Ala Leu
            180                 185                 190

Asn Gly Gly His Cys Leu Ala Gln Pro Ala Ala Glu Pro Gly Leu Gly
        195                 200                 205

Ala Val Val Arg Ser Ile Lys Val Ser Gly Tyr Leu Asn Leu Leu Ala
    210                 215                 220

Asn Thr Ile Asp Asn Phe Thr His Gly Leu Ala Val Ala Ala Ser Phe
225                 230                 235                 240

Leu Val Ser Lys Lys Ile Gly Leu Leu Thr Thr Met Ala Ile Leu Leu
                245                 250                 255

His Glu Ile Pro His Glu Val Gly Asp Phe Ala Ile Leu Leu Arg Ala
            260                 265                 270

Gly Phe Asp Arg Trp Ser Ala Ala Lys Leu Gln Leu Ser Thr Ala Leu
        275                 280                 285

Gly Gly Leu Leu Gly Ala Gly Phe Ala Ile Cys Thr Gln Ser Pro Lys
    290                 295                 300

Gly Val Val Gly Cys Ser Pro Ala Ala Glu Glu Thr Ala Ala Trp Val
305                 310                 315                 320

Leu Pro Phe Thr Ser Gly Gly Phe Leu Tyr Ile Ala Leu Val Asn Val
                325                 330                 335

Leu Pro Asp Leu Leu Glu Glu Glu Asp Pro Trp Arg Ser Leu Gln Gln
            340                 345                 350

Leu Leu Leu Leu Cys Ala Gly Ile Val Val Met Val Leu Phe Ser Leu
        355                 360                 365

Phe Val Asp
    370

<210>  43
<211>  19
<212>  DNA
<213>  Artificial

<220>
<223>  A target sequence for siRNA

<400>  43
aggagaaagt agatacaga                                                    19

1/116